(19) Europäisches Patentamt / European Patent Office / Office européen des brevets

(11) **EP 4 130 014 A1**

(12) **EUROPEAN PATENT APPLICATION**
published in accordance with Art. 153(4) EPC

(43) Date of publication:
**08.02.2023 Bulletin 2023/06**

(21) Application number: **21780885.6**

(22) Date of filing: **31.03.2021**

(51) International Patent Classification (IPC):
***C07F 9/74*** *(1968.09)* ***A61K 31/00*** *(1974.07)*
***A61P 1/16*** *(2000.01)* ***A61P 25/00*** *(2000.01)*
***A61P 29/00*** *(2000.01)* ***A61P 35/00*** *(2000.01)*

(52) Cooperative Patent Classification (CPC):
**A61K 31/00; A61K 39/395; A61P 1/16; A61P 25/00; A61P 29/00; A61P 35/00; C07F 9/74; C07K 14/81; C07K 16/40; C12N 9/12**

(86) International application number:
**PCT/CN2021/084773**

(87) International publication number:
**WO 2021/197396 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2020 CN 202010246586**

(71) Applicant: **Nuo-Beta Pharmaceutical Technology (Shanghai) Co., Ltd.**
**Shanghai 201210 (CN)**

(72) Inventors:
- **HUANG, Fude**
  **Shanghai 201210 (CN)**
- **WANG, Wenan**
  **Shanghai 201210 (CN)**
- **HONG, Feng**
  **Shanghai 201210 (CN)**
- **WEI, Wanguo**
  **Shanghai 201210 (CN)**
- **ZHANG, Jiangang**
  **Shanghai 201210 (CN)**
- **JIAO, Changping**
  **Shanghai 201210 (CN)**
- **CAO, Luxiang**
  **Shanghai 201210 (CN)**

(74) Representative: **Engelhard, Markus**
**Boehmert & Boehmert**
**Anwaltspartnerschaft mbB**
**Pettenkoferstrasse 22**
**80336 München (DE)**

# (54) DEUTERATED OXOPHENYLARSINE COMPOUND AND USE THEREOF

(57) Disclosed are a deuterated oxophenylarsine, or a pharmaceutically acceptable salt thereof, and a pharmaceutical composition containing a pharmaceutically acceptable carrier and the deuterated oxophenylarsine. The deuterated oxophenylarsine can be used for treating and preventing cancers and related diseases.

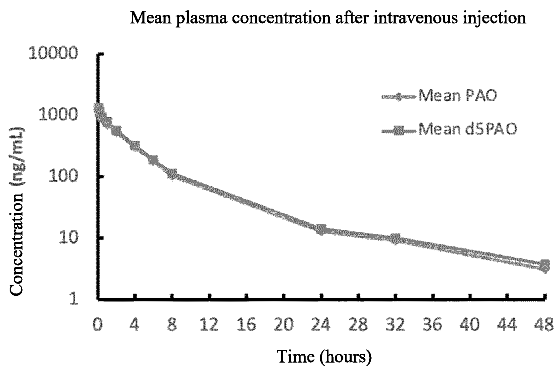


*FIG. 1*

EP 4 130 014 A1

## Description

### Technical Field

The present invention belongs to the field of chemical synthesis, and in particular relates to a novel deuterated oxophenylarsine compound and a preparation method therefor and use thereof.

### Background Art

Oxophenylarsine (Phenylarsine oxide, PAO) is a known biology inhibitor. Arsenic atoms in oxophenylarsine have high affinity to sulfur atoms of sulfhydryl in a biomolecule. Recent studies have found that oxophenylarsine is a PI4KIIIα inhibitor that can be used to treat Alzheimer's disease.

Deuterium is a stable isotope of hydrogen. Compared with hydrogen, deuterium can form a more stable chemical bond, which makes a drug molecule more stable. Human subject research has found that the substitution with deuterium can alter the half-life period of a drug and reduce the frequency of administration while maintaining the original activity and selectivity. Deuterated drugs have become a new direction and mode for new drug research and development. In 2017, the United States Food and Drug Administration approved the world's first deuterated drug, namely deuterated tetrabenazine (AUSTEDOTM, for treating Huntington's disease and its related dyskinesia). At present, a plurality of deuterated drugs have entered clinical research.

### Summary of the Invention

In one aspect, the present invention provides a compound of formula I or a pharmaceutically acceptable salt thereof,

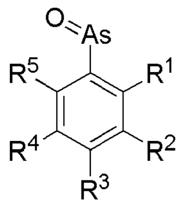

formula (I)

wherein $R^1$, $R^2$, $R^3$, $R^4$ and $R^5$ are independently selected from hydrogen, deuterium, halogen, methyl, mono-deuterated methyl, di-deuterated methyl or tri-deuterated methyl, and at least one of $R^1$, $R^2$, $R^3$, $R^4$ and $R^5$ is deuterium or deuterated.

In one embodiment, $R^1$, $R^2$, $R^3$, $R^4$ and $R^5$ are independently selected from hydrogen or deuterium, and at least one, at least two, and preferably at least three, four or five of $R^1$, $R^2$, $R^3$, $R^4$ and $R^5$ are deuterium.

In a specific embodiment, the compound is selected from the group consisting of:

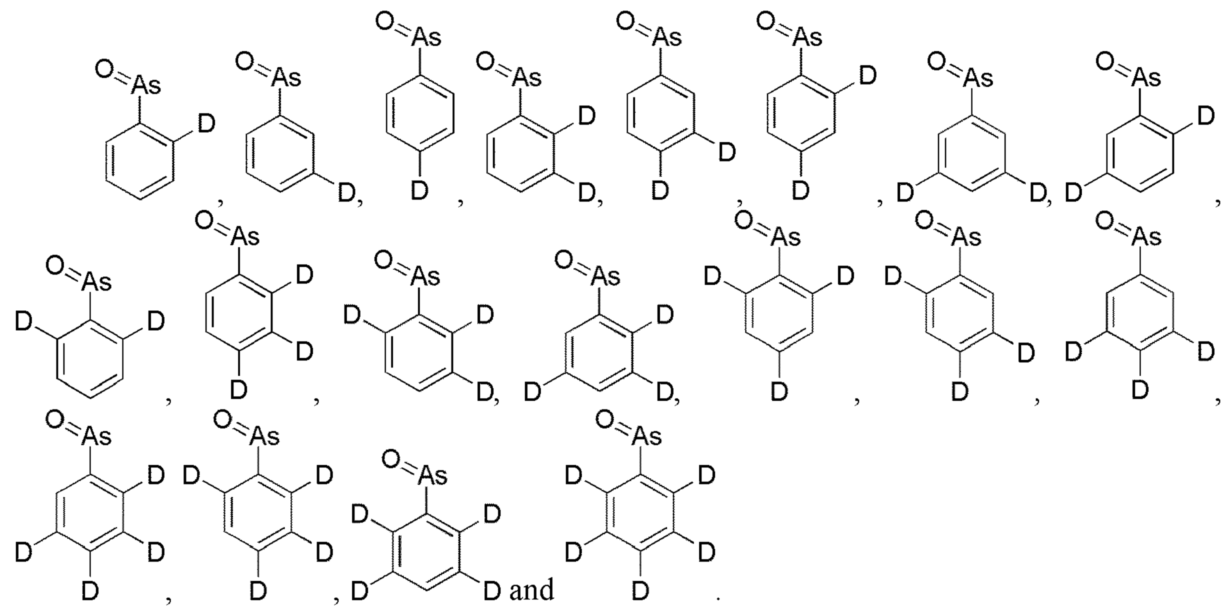

, , , , , , , , , , , , , , , , , , , and .

In another aspect, the present invention discloses the use of the above-mentioned compound or the pharmaceutically acceptable salt thereof in the preparation of a drug for preventing or treating a disease or pathological reaction in a subject.

In one embodiment, the disease is selected from a tumor, cachexia such as a malignancy or cachexia caused by a chemotherapeutic drug for treating tumor, Alzheimer's disease, a disease related to intracellular protein misfolding, a lysosomal storage disease, an inflammatory reaction, tissue and organ fibrosis, an infectious disease caused by a virus and neurosis.

In one embodiment, the subject is a human or a non-human mammal.

In a specific embodiment, the tumor is selected from lymphoma, cervical cancer, liver cancer, breast cancer such as triple negative breast cancer, lung cancer such as non-small cell lung cancer or small cell lung cancer, colorectal cancer, gastric cancer, skin cancer such as melanoma, osteocarcinoma, osteosarcoma, myeloma, leukemia or ovarian cancer.

In a specific embodiment, the disease related to intracellular protein misfolding is Parkinson's disease, Lewy body dementia, multiple system atrophy, inclusion body myositis, frontotemporal dementia, Huntington's disease, a polyglutamine disease, amyotrophic lateral sclerosis or a prion disease.

In a specific embodiment, the lysosomal storage disease is a sphingolipid metabolism disorder such as Gaucher disease, Niemann-Pick disease type C, mucopolysaccharidosis, a glycogen storage disease, a glycoprotein storage disease, a lipid storage disease, post-translational modification deficiency, an integral membrane protein deficiency disorder, neuronal ceroid lipofuscinosis or a disorder of lysosome-related organelles.

In a specific embodiment, the inflammatory reaction is manifested by an increase in inflammatory factors such as TNF-$\alpha$ or IL-6 in local tissue or systemic blood.

In a specific embodiment, the tissue and organ fibrosis is selected from pulmonary fibrosis or hepatic fibrosis.

In a specific embodiment, the virus comprises a coronavirus and a non-coronavirus, and preferably, the coronavirus is selected from avian infectious bronchitis virus, porcine epidemic diarrhea virus, porcine transmissible gastroenteritis virus, porcine hemagglutinating encephalomyelitis virus, porcine delta coronavirus, canine respiratory coronavirus, mouse hepatitis virus, feline coronavirus, human coronavirus, severe acute respiratory syndrome virus, middle East respiratory syndrome virus or novel coronavirus, and the non-coronavirus is selected from a hepatitis C virus or an HIV.

In a specific embodiment, the neurosis is selected from neurasthenia, anxiety, depression or mania.

In another aspect, the present invention discloses use of the above-mentioned compound or the pharmaceutically acceptable salt thereof in the preparation of a drug for preventing or treating a disease in a subject, the use further comprising administering a second agent to a subject in need thereof. The present invention discloses use of the above-mentioned compound or the pharmaceutically acceptable salt thereof and the second agent in the preparation of a drug in a combined administration for preventing or treating a disease in a subject.

In one embodiment, the disease is selected from a tumor, and the second agent is an agent for treating tumor.

In a specific embodiment, the disease is selected from pulmonary fibrosis, and the second agent is an agent for treating pulmonary fibrosis, such as a vascular endothelial growth factor receptor tyrosine kinase inhibitor, preferably

nintedanib.

In a specific embodiment, the second agent is an agent for treating tumor, and the agent for treating tumor is selected from at least one of paclitaxel, gemcitabine, cyclophosphamide and temozolomide.

In one embodiment, the above-mentioned compound or the pharmaceutically acceptable salt thereof is administered prior to, subsequent to or concurrently with administration of the second agent.

In another aspect, the present invention discloses a pharmaceutical composition comprising the above-mentioned compound or the pharmaceutically acceptable salt thereof and a pharmaceutically acceptable carrier.

In one embodiment, the pharmaceutical composition further comprises a drug for treating tumor.

In a specific embodiment, the drug for treating tumor is selected from at least one of paclitaxel, gemcitabine, cyclophosphamide and temozolomide.

In another aspect, the present invention discloses a method for preparing the above-mentioned compound or the pharmaceutically acceptable salt thereof, the method comprising the following steps:

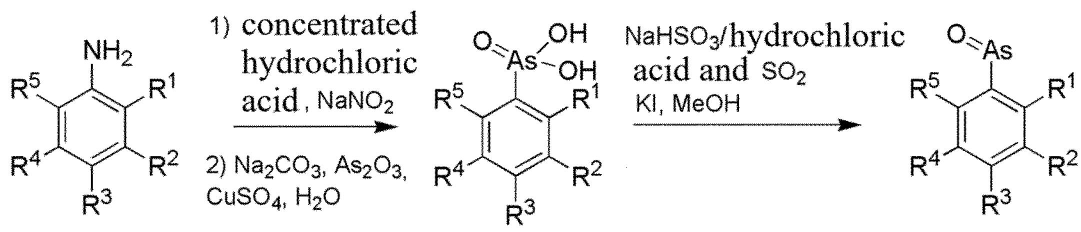


1) adding concentrated hydrochloric acid and an aqueous solution of sodium nitrite to an aqueous solution of aniline or a salt thereof that has a structure corresponding to formula (I) at 0-10°C, and maintaining the temperature below 5°C;

2) heating an aqueous solution of sodium carbonate, arsenic trioxide and copper sulfate to 90°C-100°C and then cooling the aqueous solution, adding the solution prepared in step 1) to the cooled aqueous solution, stirring and filtering the resulting mixture, adjusting a pH value of a filtrate by adding an acid, and separating the precipitated solid; and

3) stirring the precipitated solid, potassium iodide, sodium hydrogen sulfite or hydrochloric acid and sulfur dioxide in methanol until the reaction is complete, and then performing post-treatment to obtain the compound.

In one embodiment, the post-treatment in step 3) comprises adjusting a pH value to an appropriate value with an acid or a base, extracting with ethyl acetate, combining organic phases, and then evaporating to dryness.

In yet another aspect, the present invention discloses use of oxophenylarsine and a derivative thereof in the preparation of a drug for preventing or treating tissue and organ fibrosis such as pulmonary fibrosis or hepatic fibrosis.

The present invention discloses use of oxophenylarsine and a derivative thereof in the preparation of a drug for preventing or treating an inflammatory reaction, wherein the inflammatory reaction is manifested by an increase in inflammatory factors such as TNF-$\alpha$ or IL-6 in local tissue or systemic blood.

The present invention discloses use of oxophenylarsine and a derivative thereof in the preparation of a drug for preventing or treating cachexia such as malignancy or cachexia caused by a chemotherapeutic drug for treating tumor.

The present invention discloses use of oxophenylarsine and a derivative thereof in the preparation of a drug for preventing or treating tumor.

In one embodiment, the oxophenylarsine and the derivative thereof have a structure of formula (II) or a pharmaceutically acceptable salt thereof,

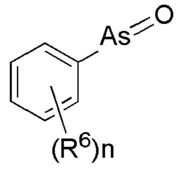

formula (II)

wherein (a) $R^6$ is each independently selected from H, halogen, nitro, cyano, hydroxyl, amino, carbamoyl, C1-6 alkylsulfuryl, C1-6 alkyl, C1-6 cycloalkyl, C2-6 alkynyl, C2-6 alkenyl, C1-6 alkoxy, C1-6 haloalkyl, C1-6 alkylene-NH2, C1-6 alkylene-NH-C(O)H, -As(O), -N=NH, N-(C1-6 alkyl)amino, N,N-(C1-6 alkyl)2amino, -NH-C(O)H, -NH-S(O)2H, -C(O)OH,

- OC(O)H, -SH, -S(O)2H, -S(O)2-NH2 or heterocyclyl and is optionally substituted with $R^7$ or $R^8$, wherein the $R^7$ and $R^8$ are each independently selected from amino, C1-6 alkyl, C1-6 alkoxy, C1-6 haloalkyl, N-(C1-6 alkyl)amino, N-(6 to 12-membered aryl)amino, N,N-(C1-6 alkyl)2amino, C3-6 cycloalkyl, 6-12 membered aryl or 3-12 membered heterocyclyl and are optionally substituted with one or more of halogen, nitro, cyano, hydroxyl, amino, carbamoyl, -NH-C(O)-$R^{10}$, -C(O)O$R^9$, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-, wherein the $R^9$ is C1-6 alkyl and is optionally substituted with one or more of halogen, nitro, cyano, hydroxyl, amino, carbamoyl, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-, wherein the $R^{10}$ is selected from H, C1-6 alkyl, C2-6 alkynyl, C2-6 alkenyl, C1-6 alkoxy or C1-6 haloalkyl; and/or (b) $R^6$ on two adjacent carbon atoms forms a 5-12 membered cycloalkyl, aryl or heterocyclyl, which is optionally substituted with one or more of halogen, nitro, cyano, hydroxyl, amino, carbamoyl, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-,
wherein n is an integer from 0 to 5.

In one embodiment, n is an integer from 0 to 2, and the $R^6$ is each independently selected from H, halogen, nitro, cyano, hydroxyl, amino, carbamoyl, C1-6 alkylsulfuryl, C1-6 alkyl, C1-6 cycloalkyl, C1-6 alkoxy, C1-6 haloalkyl, -As(O), N-(C1-6 alkyl)amino, N,N-(C1-6 alkyl)2amino, -NH-C(O)H or -NH-S(O)2H and is optionally substituted with the $R^7$ or $R^8$.

In one embodiment, n is an integer from 0 to 2, and the $R^6$ is each independently selected from H, halogen, nitro, cyano, hydroxyl, amino, C1-6 alkylsulfuryl, C1-6 alkyl, C1-6 cycloalkyl, C1-6 alkoxy, C1-6 haloalkyl, -As(O), -NH-C(O)H or -NH-S(O)2H and is optionally substituted with the $R^7$ or $R^8$.

In one embodiment, n is 1 or 2, and the $R^6$ is each independently selected from H, halogen, amino, C1-6 alkylsulfuryl, C1-6 cycloalkyl, C1-6 alkoxy, C1-6 haloalkyl, -NH-C(O)$R^7$ or -NH-S(O)2$R^8$, wherein the $R^7$ is C1-6 alkyl which is optionally substituted with 6-12 membered aryl, and the $R^8$ is 6-12 membered aryl which is optionally substituted with one of halogen, C1-6 alkoxy or C1-6 haloalkyl.

In one embodiment, the $R^6$ is located at an ortho position and/or a para position to a - As(O) group.

In one embodiment, n is 0.

In one embodiment, the compound is selected from the group consisting of:

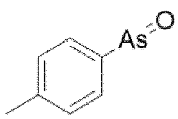 ,
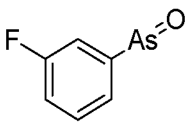 ,
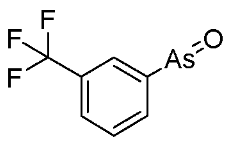 ,
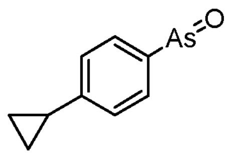 ,
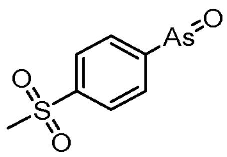 ,
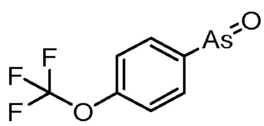 ,
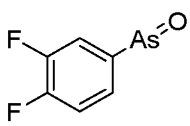 ,
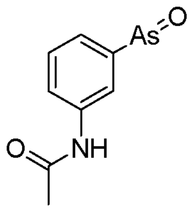 ,
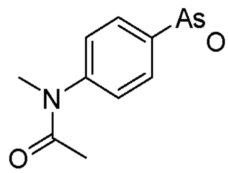 ,
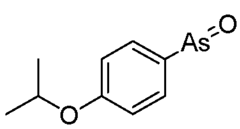 ,
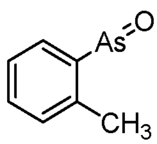 ,
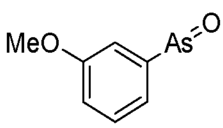 ,
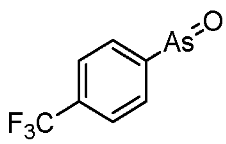 ,
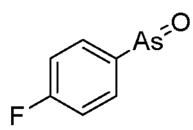 ,
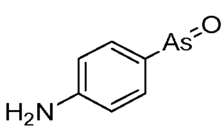 ,
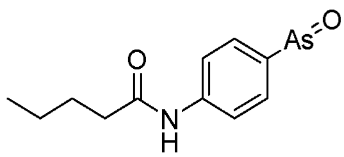 ,
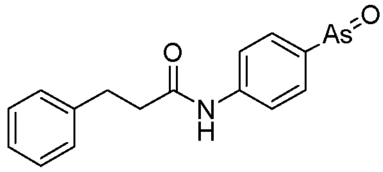 ,

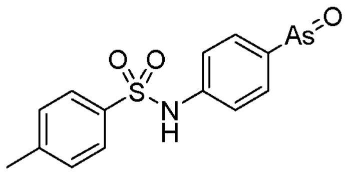 , 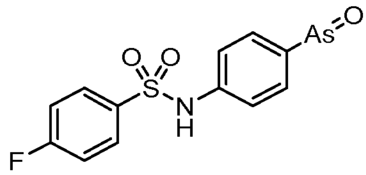 , 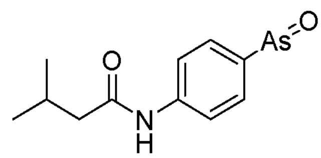 ,

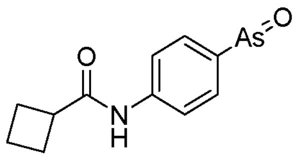 , 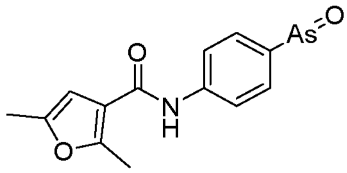 , 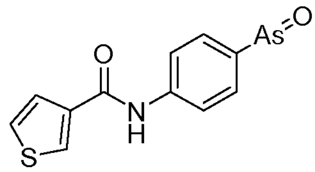 ,

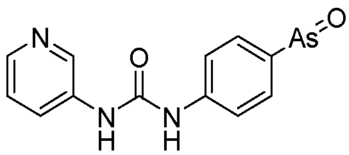 , 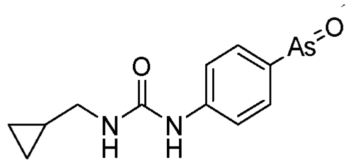 , 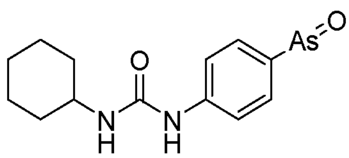 ,

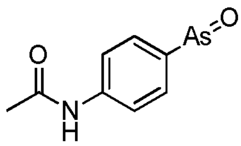 , 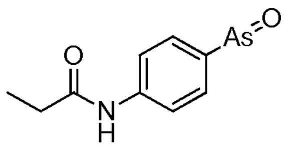 , 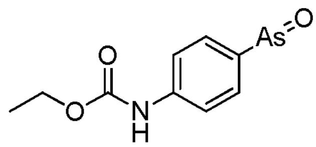 ,

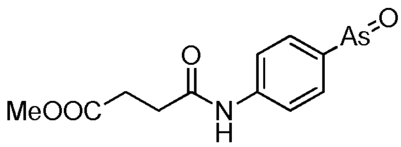 , 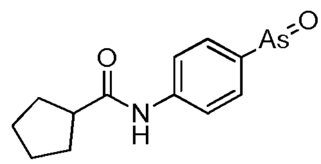 , 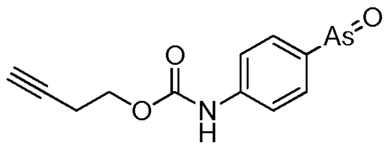 ,

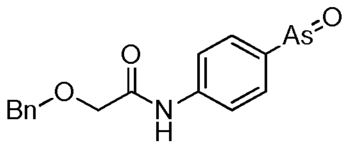 , 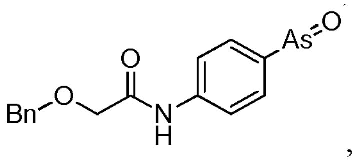 , 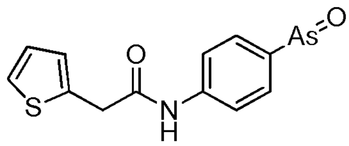 ,

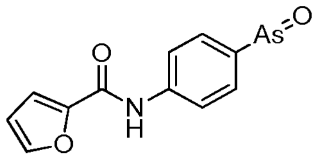 , 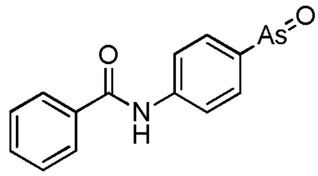 , 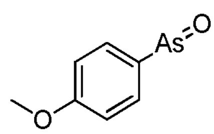 , 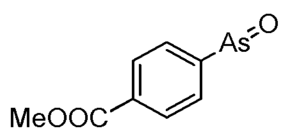 ,

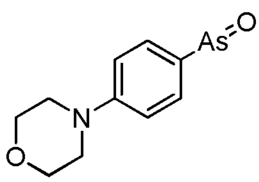 , 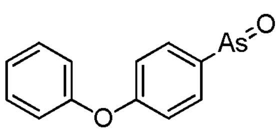 , 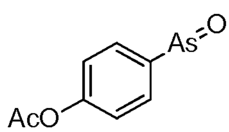 , 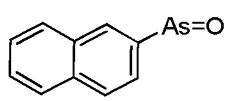 ,

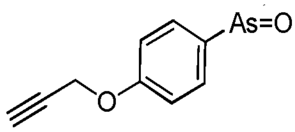,
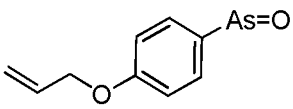,
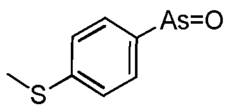,
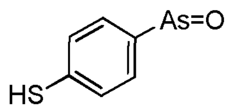,

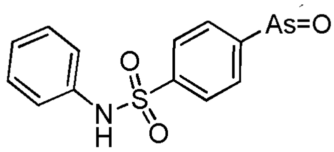,
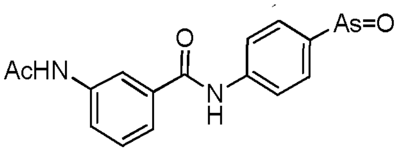,
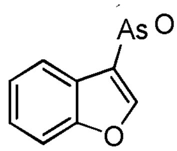,
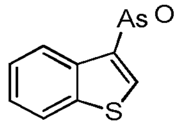,

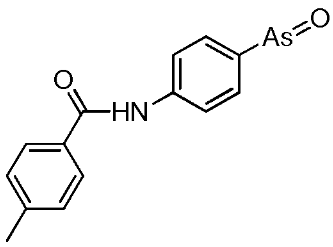,
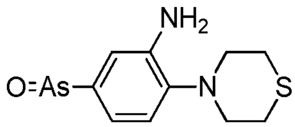,
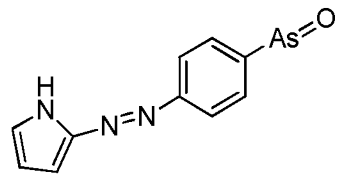,

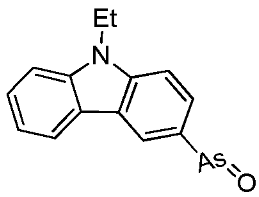,
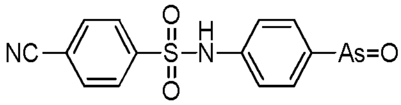,
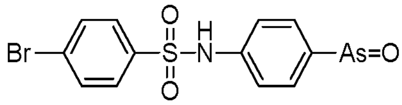,

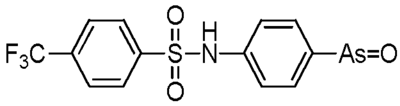,
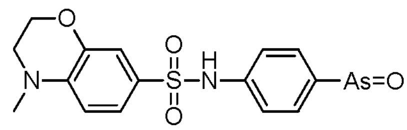,
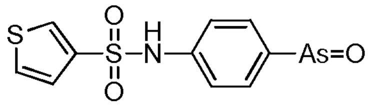,

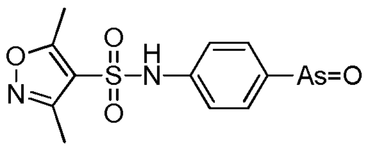,
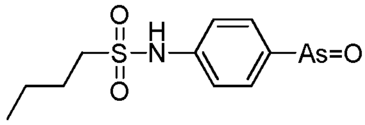,
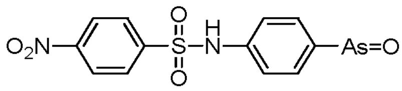,

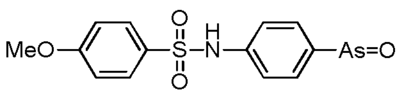,
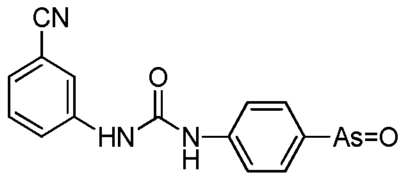,

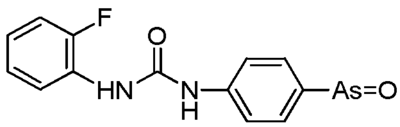,
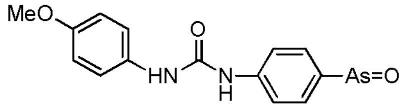,

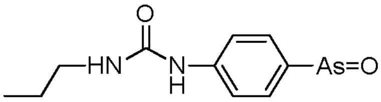, 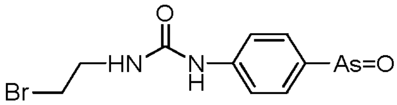, 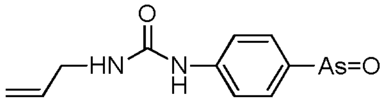,

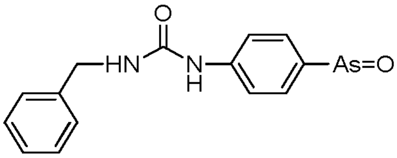, 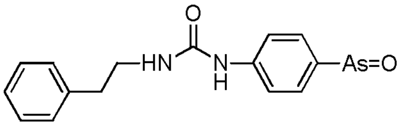, 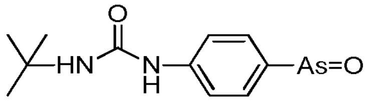,

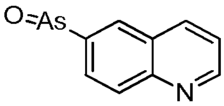, 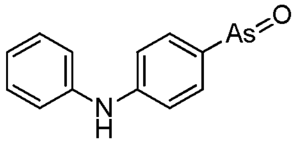, 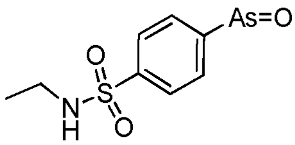,

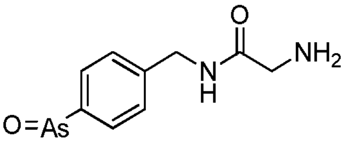, 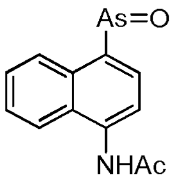, 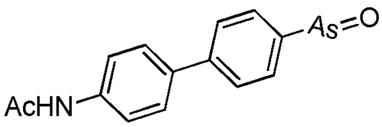,

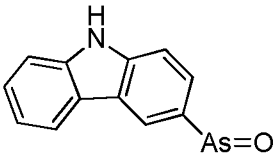, 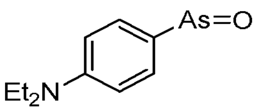, 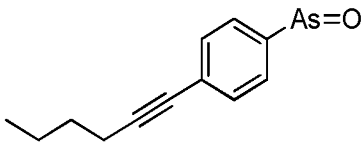,

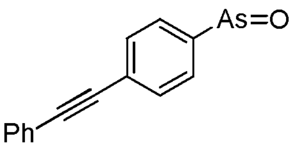, 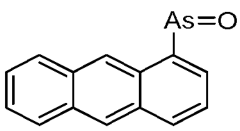, 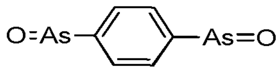, 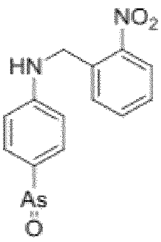,

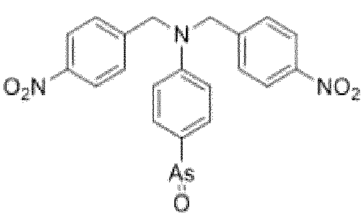, 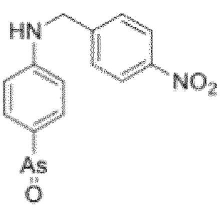, 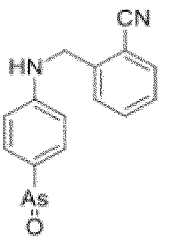, 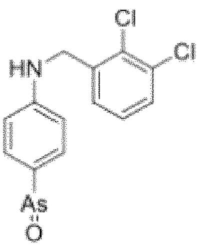,

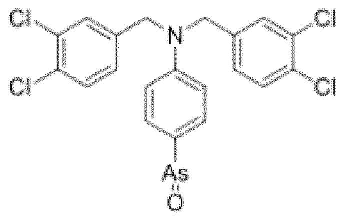 and 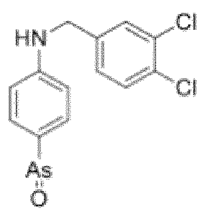 .

In one embodiment, the object is a human or a mammal.

In one embodiment, the tumor is selected from lymphoma, cervical cancer, liver cancer, breast cancer such as triple negative breast cancer, lung cancer such as non-small cell lung cancer or small cell lung cancer, colorectal cancer, gastric cancer, skin cancer such as melanoma, osteocarcinoma, osteosarcoma, myeloma, leukemia or ovarian cancer.

In one embodiment, the use further comprises administering a second agent to an object in need thereof, wherein the second agent is preferably an agent for treating tumor.

In one embodiment, the second agent is an agent for treating tumor.

In one embodiment, the compound is administered prior to, subsequent to or concurrently with administration of the second agent.

In one embodiment, the agent for treating tumor is selected from at least one of paclitaxel, gemcitabine, cyclophosphamide and temozolomide.

The present invention further discloses a method for screening a drug for preventing or treating a disease, the method comprising contacting a candidate drug with PI4KIIIα proteins or nucleic acids or PI4KIIIα and detecting whether the candidate drug can inhibit the formation or activity of PI4KIIIα, wherein the disease is selected from tissue or organ fibrosis, an inflammatory reaction, cachexia and a tumor.

In one embodiment, the tissue and organ fibrosis is selected from pulmonary fibrosis or hepatic fibrosis.

In one embodiment, the inflammatory reaction is manifested by an increase in inflammatory factors such as TNF-α or IL-6 in local tissue or systemic blood.

In one embodiment, the tumor is selected from lymphoma, cervical cancer, liver cancer, breast cancer such as triple negative breast cancer, lung cancer such as non-small cell lung cancer or small cell lung cancer, colorectal cancer, gastric cancer, skin cancer such as melanoma, osteocarcinoma, osteosarcoma, myeloma, leukemia or ovarian cancer.

Brief Description of the Drawings

FIG. 1 shows a plasma drug concentration-time curve for pharmacokinetics after single-dose intravenous injection of 0.1 mg/kg PAO or d5PAO in male SD rats.

FIG. 2 shows a plasma drug concentration-time curve for pharmacokinetics after single-dose oral lavage of 0.2 mg/kg PAO or d5PAO in male SD rats.

FIG. 3 shows a vector map of an α-synuclein overexpression plasmid.

FIG. 4 shows a standard preparation diagram of α-synuclein ELISA.

FIG. 5 shows inhibitory effects of d5PAO and PAO on apoptosis of SH-sy5y cells. FIG. 5A shows effects of certain concentrations of d5PAO and PAO on viability of SH-sy5y cells as detected by MTT (n = 5; mean ± SEM; One-way ANOVA; ***p < 0.0001 *vs.* ctrl; and ### p < 0.0001 *vs.* ctrl). FIG. 5B shows immunofluorescent staining images, wherein propidium iodide (PI) is added in culture solution, and the mixture was co-incubated for 15 min, followed by immunofluorescent staining of Ki67.

FIG. 6 shows effects of d5PAO and PAO on improving viability of stably-transformed APP (SW) HEK293 cells and promoting Aβ release. FIG. 6A shows effects of certain concentrations of d5PAO and PAO on viability of stably-transformed APP (SW) HEK293 cells as detected by MTT (n = 5; mean ± SEM; One-way ANOVA; and **p < 0.001, ***p < 0.0001 *vs.* ctrl). FIG. 6B shows Aβ contents in supernatants as detected by an ELISA kit, wherein Aβ values of each group are calculated according to a standard curve. FIG. 6C shows fold changes of Aβ contents in each group (data is normalized with ctrl as 1; n = 3; mean ± SEM; One-way ANOVA; and *p < 0.03, **p < 0.001, ***p < 0.0001 vs. ctrl).

FIG. 7 shows comparisons of effects of deuterated compound PAO with a structural formula on promoting Aβ release. FIG. 7A shows Aβ contents in supernatants as detected by an ELISA kit, wherein Aβ values of each group are calculated according to a standard curve. FIG. 7B shows fold changes of Aβ contents in each group (data is normalized

with ctrl as 1; n = 3; mean ± SEM; One-way ANOVA; *p < 0.03, **p < 0.001, ***p < 0.0001 *vs.* ctrl; and ##p < 0.001, ###p < 0.0001 vs. 50 nM d5PAO).

FIG. 8 shows effects of d5PAO and PAO on reducing damage of SH-sy5y cells caused by α-synuclein overexpression and on promoting α-synuclein release. FIG. 8A shows effects of certain concentrations of d5PAO and PAO on viability of transiently-transformed α-synuclein cells as detected by MTT (n = 5; mean ± SEM; One-way ANOVA; and *p < 0.03, **p < 0.001, ***p < 0.0001 vs. ctrl). FIG. 8B shows α-synuclein contents in supernatants as detected by an ELISA kit, wherein α-synuclein values of each group are calculated according to a standard curve (n = 3; mean ± SEM; One-way ANOVA; and *p < 0.03 vs. Ctrl). FIG. 8C shows fold changes of α-synuclein contents in each group (data is normalized with α-syn OE group as 1; n = 3; mean ± SEM; One-way ANOVA; and *p < 0.03 vs. ctrl).

FIG. 9 shows protective effects of d5PAO and PAO in an SH-SY5Y cell model constructed by CBE. FIG. 9A shows viability of SH-SY5Y cells treated with CBE for 48 h, as detected by MTT. FIG. 9B shows viability of SH-SY5Y cells that are firstly treated with 100 μM of CBE for 24 h, then starved (high-glucose DMEM free of FBS) while being treated with 100 μM of CBE for another 24 h, and finally treated with different concentrations of PAO for 24 h, as detected by MTT. FIG. 9C shows viability of SH-SY5Y cells that are firstly treated with 100 μM of CBE for 24 h, then starved (high-glucose DMEM free of FBS) while being treated with 100 μM of CBE for another 24 h, and finally treated with different concentrations of d5PAO and PAO for 24 h, as detected by MTT (n = 5; data is expressed as mean ± SEM; ###p < 0.0001 vs. ctrl; and **p < 0.001 vs. 100 μM CBE, ***p < 0.0001 vs. 100 μM CBE).

FIG. 10 shows effects of PAO on inhibiting CBE-induced lysosome and GlcCer storage and promoting GlcCer efflux. FIG. 10A shows immunofluorescent staining images of Lyso-tracker, wherein SH-SY5Y cells are co-incubated with lysosome trackers for 30 min, then the supernatant is removed, different concentrations of PAO are added, the mixture was incubated for 10 min, and Lyso-tracker is observed by immunofluorescent staining. FIG. 10B shows statistical analysis of fluorescent intensity of Lyso-tracker in each group (scale bar: 50 μm; n = 5; One-way ANOVA; ##p < 0.001 vs. ctrl; and **p < 0.001, ***p < 0.0001 vs. 100 μM CBE). FIG. 10C shows statistical analysis of GlcCer concentrations in cell lysates of each group according to LC/MS measurement values. FIG. 10D shows statistical analysis of GlcCer concentrations in supernatants of cell culture mediums of each group according to LC/MS measurement values.

FIG. 11 shows effects of *PI4Ka* knockdown on promoting a reduction in lysosomal storage. FIG. 11A shows PI4KIIIα protein levels as detected by Western blot, wherein SH-SY5Y cells are treated with different shRNA interfering lentiviral vectors (sh-ctrl, sh1-*PI4Ka,* sh2-*PI4Ka* and sh3-*PI4Ka*) for 48 h. FIG. 11B shows statistical analysis results of Western blot. FIG. 11C shows fluorescent intensity as detected by immunofluorescent staining of Lyso-tracker after treatment with shRNA interfering lentiviral vectors. FIG. 11D shows the statistical analysis (scale bar: 50 μm; n = 5; data is expressed as mean ± SEM; One-way ANOVA; and ***p < 0.0001).

FIG. 12 shows a pGMLV-SC5RNAi vector map.

FIG. 13 shows bright-field images of MRC-5 cells treated for 24 h, wherein the MRC-5 cells are cultured in MEM (FBS-free, containing 5 ng/mL TGF-β1 and different concentrations of PAO or d5PAO according to groups) for 24 h (scale bar: 50 μm).

FIG. 14 shows inhibitory effects of d5PAO and PAO on expression of α-SMA and Calponin1 in MRC-5 cell models. FIG. 14A shows expression levels of α-SMA and Calponin1 as detected by Western blot. FIG. 14B shows statistical analysis results of expression levels of α-SMA in each group. FIG. 14C shows statistical analysis results of expression levels of Calponin1 in each group (the protein signal intensity is analyzed by Image J software, with the signal intensity of ctrl as 1; n = 3; One-way ANOVA; data is expressed as mean ± SEM; *p < 0.03, **p < 0.001, ***p < 0.0001 vs. 5 ng/mL TGF-β1; and #p < 0.03 vs. ctrl).

FIG. 15 shows inhibitory effects of d5PAO and PAO on expression of α-SMA and Calponin1 in MRC-5 cell models. FIG. 15A shows immunofluorescent staining images of α-SMA in each group (red: α-SMA, blue: DAPI (nuclei), and scale bar: 50 μm). FIG. 15B shows immunofluorescent staining images of Calponin1 in each group (red: Calponin1, blue: DAPI (nuclei), and scale bar: 50 μm). FIGs. 15C and 15D show statistical analysis results of immunofluorescent intensity of α-SMA and Calponin1, respectively (with the mean value of ctrl as 1; n = 5; One-way ANOVA; data is expressed as mean ± SEM; *p < 0.03, **p < 0.001, ***p < 0.0001 vs. 5 ng/mL TGF-β1; and ###p < 0.0001 vs. ctrl).

FIG. 16 shows regulatory effects of d5PAO and PAO on expression of Calponin1 in MSC. FIG. 16A shows immunofluorescent staining images of Calponin1 in each group (red: Calponin1, blue: DAPI (cell nuclei), and scale bar: 50 μm). FIG. 16B shows statistical analysis results of immunofluorescent intensity of Calponin1 (n = 4; One-way ANOVA; and data is expressed as mean ± SEM).

FIG. 17 shows inhibitory effects of d5PAO and PAO on secretion of COL1 in MRC-5 cells during fibrosis. FIG. 17A shows COL1 concentrations in supernatants of each group as detected by ELISA (n = 6, and data is expressed as mean ± SEM). FIG. 17B shows statistical analysis of COL1 concentrations in supernatants of each group (with the mean value of ctrl as 1; data is expressed as mean ± SEM; *p < 0.03, **p < 0.001, ***p < 0.0001 *vs.* 5 ng/mL TGF-β1; and #p < 0.0001 vs. ctrl).

FIG. 18 shows effects of shRNA interfering lentiviral vectors on reducing expression of PI4KIIIα, wherein the shRNA

interfering lentiviral vectors are co-incubated with MRC-5 cells for 48 h, and proteins are collected for Western blot. FIG. 18A shows detection of PI4KIIIα proteins. FIG. 18B shows data analysis results of Image J software (data is normalized with sh-ctrl as 1 and expressed as mean ± SEM; n = 3; and ***p < 0.0001 vs. sh-ctrl).

FIG. 19 shows inhibitory effects of *PI4Ka* knockdown on expression of Calponin1 and α-SMA in MRC-5 cells treated with TGF-β1, wherein after the MRC-5 cells are adhered to the wall, lentiviral vectors with different sequences are added, and the mixture is cultured for 24 h and treated with or without 5 ng/mL TGF- β1 according to groups for 24 h, and immunofluorescent staining is performed prior to observation. FIG. 19A shows immunofluorescent staining images of α-SMA in each group (red: α-SMA, blue: DAPI (nuclei), green: green fluorescent protein (GFP), and scale bar: 50 μm). FIG. 19B shows immunofluorescent staining images of calponin1 in each group (red: Calponin1, blue: DAPI (cell nuclei), green: green fluorescent protein (GFP), and scale bar: 50 μm). Statistical analysis results of immunofluorescent intensity of α-SMA (FIG. 19C) and Calponin1 are showed (data is normalized with the mean value of sh-ctrl as 1 and expressed as mean ± SEM; n = 5; *p < 0.03, ***p < 0.0001 vs. sh-ctrl+5 ng/mL TGF-β1; and ##p < 0.001, ###p < 0.0001 vs. sh-ctrl).

FIG. 20 shows inhibitory effects of d5PAO and PAO on secretion of IL-6 and TNF-α in BV2 cell inflammatory models. FIG. 20A shows TNF-α concentrations in supernatants of BV2 cells as detected by ELISA, wherein TNF-α contents (pg/mL) are calculated according to a standard curve. FIG. 20B shows relative concentration changes of TNF-α in each group (with the mean concentration of ctrl as 1). FIG. 20C shows IL-6 concentrations in supernatants of BV2 cells as detected by ELISA, wherein IL-6 contents (pg/mL) are calculated according to a standard curve. FIG. 20D shows relative concentration changes of IL-6 in each group (with the mean concentration of ctrl as 1; n = 3; One-way ANOVA; data is expressed as mean ± SEM; *p < 0.03, **p < 0.001, ***p < 0.0001 vs. 1 μg/mL LPS; and #p < 0.03, ##p < 0.001 vs. ctrl).

FIG. 21 shows inhibitory effects of PAO on breast cancer.

FIG. 22 shows inhibitory effects of PAO on lymphoma.

FIG. 23 shows inhibitory effects of d5PAO on melanoma.

FIG. 24 shows inhibitory effects of d5PAO on melanoma on day 28 of administration.

FIG. 25 shows comparison of effects of high-dose PAO and d5PAO via lavage on weights and survival rates of mice.

FIG. 26 shows effects of PAO on weights of breast cancer mouse models.

FIG. 27 shows effects of PAO on weights of pancreatic cancer models.

FIG. 28 shows effects of PAO on weights of lymphoma animal models.

FIG. 29 shows effects of d5PAO in a combined administration on weights of mice with melanoma.

FIG. 30 shows inhibitory effects of PAO and d5PAO on HCoV229E (influenza coronavirus).

FIG. 31 shows anti-anxiety effects of PAO and d5PAO, wherein d5PAO has a more significant anti-anxiety effect than PAO.

FIG. 32 shows anti-depression effects of PAO and d5PAO, wherein d5PAO has a more significant and stable anti-depression effect than PAO.

FIG. 33 shows inhibitory effects of d5PAO and PAO on cholesterol storage caused by U18666A (scale bar: 50 μm).

FIG. 34 shows effects of PAO on promoting expression of LC3B and p62 and effects of Baf-A1 on blocking protection of PAO in a cell model. FIG. 34A shows LC3B and p62 proteins as detected by Western blot. FIGs. 34B and 34C show statistical analysis of signal intensity of LC3B and p62 proteins as analyzed by Image J software. FIG. 34D shows immunofluorescent staining images of LC3B and p62 (red: p62, green: LC3B, and scale bar: 50 μm). FIG. 34E shows cell viability in each group as detected by MTT and the statistical analysis (n = 5; data is expressed as mean ± SEM; One-way ANOVA; ###p < 0.0001 *vs.* ctrl; and **p < 0.001 *vs.* 100 μM CBE).

FIG. 35 shows effects of *PI4Ka* knockdown on activation of ALP. FIGs. 35A and 35B show LC3B protein levels as detected by Western blot, wherein SH-SY5Y cells are treated with different shRNA interfering lentiviral vectors (sh-ctrl, h1-PI4Ka, sh2-PI4Ka and sh3-PI4Ka) for 48 h, and the statistical analysis. FIGs. 35C and 35D show LC3B protein levels, wherein the cells are transfected by shRNA interfering lentiviral vectors while receiving CBE treatment for 48 h of co-incubation, and the statistical analysis (n = 3; data is expressed as mean ± SEM; One-way ANOVA; and *p < 0.03 vs. sh-ctrl).

FIG. 36 shows percentages of acetylcholine-induced enhanced pause (Penh) values relative to a baseline.

FIG. 37 shows total counts of eosinophils, macrophages, neutrophils and lymphocytes in bronchoalveolar lavage fluid (BALF) (T.Test; one-tailed; * < 0.5, ** < 0.1 *vs.* the total cell content of the model group).

FIG. 38 shows respective counts of eosinophils, macrophages, neutrophils and lymphocytes in bronchoalveolar lavage fluid (BALF) (T.Test; one-tailed; * < 0.5, ** < 0.1 vs. the BALF of the model group).

FIG. 39 shows collagen type I contents in plasma (taking a normal group as ctrl).

FIG. 40 shows comparison of effects of PAO and dPAO with nintedanib (a positive control drug) on the down-regulation of hyaluronic acid in plasma of mice with pulmonary fibrosis (taking a normal group as ctrl).

Detailed Description of The Invention

Hereinafter, the present invention will be described in detail according to the embodiments and in conjunction with the accompanying drawings. The above aspects of the present invention and other aspects of the present invention will be apparent from the following detailed description. The scope of the present invention is not limited to the following embodiments.

The term "compound" as used herein is intended to include all stereoisomers (e.g., enantiomers and diastereomers), geometric isomers, tautomers and isotopes of the indicated structure.

In another aspect, the present invention relates to deuterated oxophenylarsine, preferably wherein all hydrogen atoms on a benzene ring are substituted with deuterated isotopes.

The compound described herein can be asymmetric (e.g., having one or more stereocenters). Unless otherwise indicated, all stereoisomers, such as enantiomers and diastereomers, are intended to be included. The compound described herein may have various geometric isomers involving, for example, olefins and carbon-carbon double bonds, and all the stable isomers have been considered herein. Cis- and trans-geometric isomers of the compound are described herein and may be isolated in the form of an isomer mixture or an individual isomer.

The compound described herein also includes a tautomeric form. The tautomeric form results from the exchange of a single bond with an adjacent double bond accompanied by the migration of protons. The tautomeric form includes proton tautomers in isomeric protonation states that have the same chemical formula and total charge. Examples of the proton tautomers include keto-enol, amide-imidic acid, lactam-lactim, enamine-imine and cyclic forms, wherein protons may occupy two or more positions (such as 1H- and 3H-imidazole, 1H-, 2H- and 4H-1,2,4-triazole, 1H- and 2H-isoindole, and 1H- and 2H-pyrazole) of a heterocyclic system. The tautomeric form can be equilibrated or sterically locked into one form by means of suitable substitution.

In certain embodiments, the small-molecule compound described herein can be obtained by means of organic synthesis. The compound described herein, including a salt, ester, hydrate or solvate thereof, can be prepared by means of any well-known organic synthesis technique and can be synthesized according to a variety of possible synthetic routes.

The term "oxophenylarsine" (PAO) as used herein refers to a small-molecule compound with the following specific chemical structure:

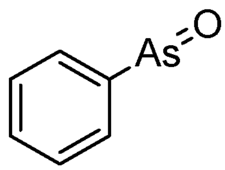

.

Disease related to intracellular protein misfolding

The term "disease related to intracellular protein misfolding" as used herein refers to a disease characterized by aggregation of abnormally folded proteins in the cytoplasm and is also diagnosed as protein aggregation (accumulation) or protein misfolding disease. In addition, the term "disease related to intracellular protein misfolding" also includes some intracellular inclusion body diseases, such as a disease with protein aggregation and inclusion body formation, wherein such inclusion body is mainly formed by aggregation of a core protein due to misfolding, with attachment of various stress proteins involved in responding to unfolded proteins.

The disease related to intracellular protein misfolding includes, but is not limited to, Parkinson’s disease (PD), Lewy body dementia (LBD), multiple system atrophy (MSA), inclusion body myositis (IBM), frontotemporal dementia (FTD), Huntington’s disease (HD), a polyamine disease (PolyQ), amyotrophic lateral sclerosis (ALS) and a prion disease.

Lysosomal storage disease

The term "lysosomal storage disease" as used herein refers to a disease caused by the accumulation of some endogenous or exogenous substances in lysosomes for various reasons, including but not limited to, lysosomal function deficiency caused by insufficient enzyme activity in lysosomes and the lack of processing and correction enzymes for activator proteins, transport proteins or lysosomal proteins, and due to the lysosomal function deficiency, the corresponding substrate is unable to be digested in secondary lysosomes and thus is accumulated, and a metabolic disorder occurs to lead to a storage disease, etc.

The lysosomal storage disease includes, but is not limited to, a sphingolipid metabolism disorder, mucopolysaccharidosis, a glycogen storage disease, a glycoprotein storage disease, a lipid storage disease, post-translational modification deficiency, an integral membrane protein deficiency disorder, neuronal ceroid lipofuscinosis or a disorder

of lysosome-related organelles. Among them, the sphingolipid metabolism disorder includes, but is not limited to, Fabry disease, dermotosis of metabolism disturbance (Farbe disease), Gaucher disease types I, II and III and perinatal lethal Gaucher disease, GM1 gangliosidosis types I, II and III, GM2 gangliosidosis (amaurotic familial idiocy), GM2 gangliosidosis, globoid cell leukodystrophy (Krabbe disease), metachromatic leukodystrophy and Niemann-Pick types A and B; the mucopolysaccharidosis includes, but is not limited to, Hurler-Scheie syndrome and Scheie syndrome (ML I), Hunter syndrome (MPS II), Sanfilippo syndrome A (MPS IIIA), Sanfilippo syndrome B (MPS IIIB), Sanfilippo syndrome C (MPS IIIC), Sanfilippo syndrome D (MPS IIID), eccentro-osteochondrodysplasia syndrome (MPS IVA), eccentro-osteochondrodysplasia syndrome (MPS IVB), mucopolysaccharidosis type VI (Maroteaux-lamy syndrome, MPS VI), Sly disease (MPS VII) and MPS IX; the glycogen storage disease includes, but is not limited to, rare Pompe disease (GSD II); the glycoprotein storage disease includes, but is not limited to, alpha-mannosidosis, beta-mannosidosis, fucosidosis, aspartylglucosaminuria, Schindler disease type I (infantile neuroaxonal dystrophy), Schindler disease type II (Kanzaki disease), Schindler disease type III (intermediate severe), sialidosis type I (cherry-red spot-myoclonus syndrome), sialidosis type II (mucopolysaccharidosis I) and galactosialidosis; the lipid storage disease includes, but is not limited to, acid lipase deficiency such as Wolman's disease and cholesteryl ester storage disease; the post-translational modification deficiency includes, but is not limited to, multiple sulfatase deficiency, mucolipidosis type II$\alpha/\beta$ (I-cell disease), mucolipidosis type II$\alpha/\beta$ (pseudo-Hurler syndrome), and mucolipidosis type III$\gamma$ (pseudo-Hurler syndrome variant); the integral membrane protein deletion disorder includes, but is not limited to, homocystinuria, Danon disease, a myoclonus-renal failure syndrome, sialidosis (such as ISSD, Salla disease and intermediate severe Salla disease), Niemann-Pick disease types C1 and C2 and mucolipidosis type IV; the neuronal ceroid lipofuscinosis includes, but is not limited to, ceroid lipofuscinosis type 1 (Haltia-Santavuori disease and INCL), neuronal ceroid lipofuscinosis type 2 (Jansky-Bielschowsky disease), ceroid lipofuscinosis type 3 (Batten-Spielmeyer-Sjogren disease), ceroid lipofuscinosis type 4 (Parry disease and Kufs disease types A and B), ceroid lipofuscinosis type 5 (finnish variant late infantile), ceroid lipofuscinosis type 6 (Lake-Cavanagh or Indian variant), ceroid lipofuscinosis type 7 (Turkish variant), ceroid lipofuscinosis type 8 (Northern epilepsy and epilepsy-intellectual disability), ceroid lipofuscinosis type 9, ceroid lipofuscinosis type 10, ceroid lipofuscinosis type 11, ceroid lipofuscinosis type 12, ceroid lipofuscinosis type 13 and ceroid lipofuscinosis type 14; and the disorder of lysosome-related organelles includes, but is not limited to, Hermansky-Pudlak syndrome type 1, Hermansky-Pudlak syndrome type 2, Hermansky-Pudlak syndrome type 3, Hermansky-Pudlak syndrome type 4, Hermansky-Pudlak syndrome type 5, Hermansky-Pudlak syndrome type 6, Hermansky-Pudlak syndrome type 7, Hermansky-Pudlak syndrome type 8, Hermansky-Pudlak syndrome type 9, Griscelli syndrome type 1 (Elejalde syndrome), Griscelli syndrome type 2 and Chédiak-Higashi disease.

Drug administration and medical use

The term "pharmaceutically acceptable" as used herein refers to those compounds, materials, compositions and/or dosage forms that are, within the scope of reasonable medical judgment, suitable for use in contact with human and animal tissues without undue toxicity, irritation, allergic response or other problems or complications, and are commensurate with a reasonable benefit/risk ratio. In certain embodiments, pharmaceutically acceptable compounds, materials, compositions and/or dosage forms are those approved by a regulatory agency (e.g., the United States Food and Drug Administration, the China Food and Drug Administration, or the European Medicines Agency) or listed on the generally accepted pharmacopoeia (such as the United States Pharmacopoeia, the Chinese Pharmacopoeia, or the European Pharmacopoeia) and used for animals (more particularly, humans).

The term "object" as used herein may include a human and a non-human animal. The non-human animal includes all vertebrates, such as a mammal and a non-mammal. The "object" also may be livestock (e.g., a cow, a pig, a sheep, a chicken, a rabbit or a horse), a rodent (e.g., rat or mouse), a primate (e.g., a gorilla or a monkey) or a domestic animal (e.g., a dog or a cat). The "object" may be a male or female object, and also may be of different ages. The human "object" may be Caucasian, African, Asian, Sumerian or other races, or a hybrid of different races. The human "object" may be an old person, an adult, an adolescent, a child or an infant.

In some embodiments, the object described herein is a human or non-human primate.

The deuterated oxophenylarsine disclosed herein can be administered by means of administration routes known in the art, such as an injection (e.g., subcutaneous injection, intraperitoneal injection, intravenous injection (including intravenous drip or infusion), intramuscular injection or intradermal injection) administration or a non-injection administration (e.g., oral administration, nasal administration, sublingual administration, rectal administration or topical administration). In some embodiments, the deuterated oxophenylarsine described herein is administered orally, subcutaneously, intramuscularly or intravenously. In some embodiments, the deuterated oxophenylarsine described herein is administered orally.

As used herein, the term "therapeutically effective amount" refers to an amount of a drug that can alleviate or eliminate a disease or symptom in an object or prophylactically inhibit or prevent the occurrence of a disease or symptom. The therapeutically effective amount may be an amount of a drug that alleviates one or more diseases or symptoms in

an object to a certain extent; an amount of a drug that can partially or fully restore one or more physiological or biochemical parameters associated with the cause of the disease or symptom to normal; and/or an amount of a drug that can reduce the possibility of the onset of a disease or symptom. In some embodiments, the term "therapeutically effective amount" as used herein refers to an amount of a drug that can alleviate or eliminate a disease related to intracellular protein misfolding or a lysosomal storage disease in an object.

The therapeutically effective amount of deuterated oxophenylarsine provided herein depends on a variety of factors known in the art, such as weight, age, medical history, current treatment received, object's health condition, the strength of drug-drug interaction, allergies, hypersensitivity and side effects, as well as administration routes and the extent of disease progression. A person skilled in the art (such as a doctor or a veterinarian) may reduce or increase the dose according to these or other conditions or requirements.

In some embodiments, the treatment further comprises administering a second agent to an object in need thereof.

In some embodiments, the second agent is an agent for treating a disease related to intracellular protein misfolding, and includes, but is not limited to, levodopa and riluzole.

In some embodiments, the deuterated oxophenylarsine is administered prior to, subsequent to, or concurrently with the second agent.

The present application also relates to a method for preventing or treating a disease related to intracellular protein misfolding, the method comprising administering an effective amount of deuterated oxophenylarsine to an object in need thereof.

The present application also relates to a method for preventing or treating a lysosomal storage disease, the method comprising administering an effective amount of deuterated oxophenylarsine to a subject in need thereof.

**Example 1. Synthesis and physical properties of compounds**

**1. Synthesis and physical properties of d5PAO (penta-deuterated oxophenylarsine)**

**1.1 Synthesis of d5PAO**

The synthetic route of d5PAO is as follows:

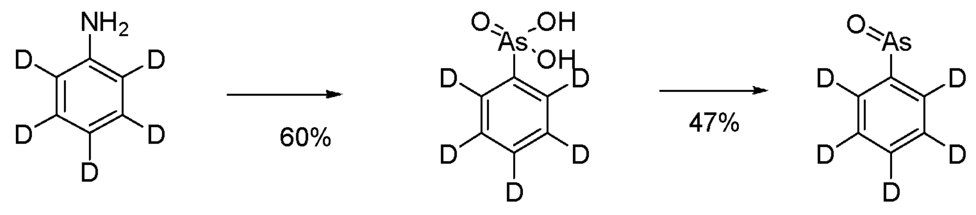


d5-Aniline d5-PA d5PAO

Step 1: synthesis of d5-PA:

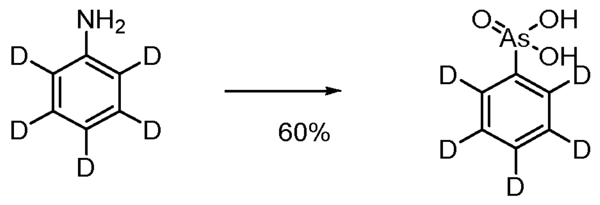


d5-Aniline d5-PA

91.35 mL of water and 18.27 g of penta-deuterated aniline (d5-Aniline) were added to a three-necked flask, stirred and cooled to 0-10°C. 37.45 mL of concentrated hydrochloric acid was added dropwise. After that, an aqueous solution of sodium nitrite (13.43 g of solid sodium nitrite dissolved in 36.5 mL of water) was added dropwise, and the temperature was maintained below or at 5°C for about 2-3 h to complete the preparation of a diazonium salt.

274 mL of purified water, 69.06 g of sodium carbonate, 36.82 g of arsenic trioxide and 2.83 g of copper sulfate

pentahydrate ($CuSO4 \cdot 5H_2O$) were added to another container, heated to 90°C-100°C, stirred thermostatically for 30 min, and then cooled to 5°C-15°C. The diazonium salt prepared above was slowly added in batches, and the temperature was controlled to be lower than 15°C. The resulting mixture was stirred for 2-3 h, then naturally heated to room temperature, stirred overnight and filtered. The filter cake was rinsed with water. The filtrate was combined. The pH value of the system was adjusted to 3.0 by slowly adding concentrated hydrochloric acid, with a small amount of brown floccules precipitated. Suction filtration was performed. The filtrate was washed three times with 100 mL of ethyl acetate, and the aqueous phase was concentrated under reduced pressure at 50°C-60°C to about 170 mL, with a large amount of white solids precipitated. Suction filtration was performed. The filter cake was rinsed with cold water and dehumidified, and the solid was directly dried at 50°C in a blast oven for 18 h to obtain 35 g of d5-PA as an off-white solid (yield: 90.83%, MS ES+(m/z): 208.0 [$(M+H)^+$]).

Step 2: Synthesis of d5-PAO:

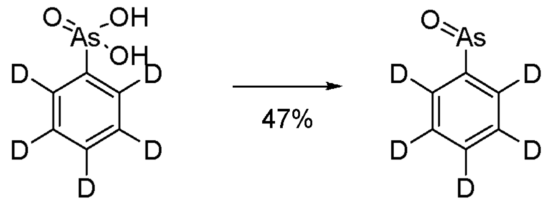


d5-PA d5PAO

400 mL of purified water, 25 g of d5-PA, 75.4 g of sodium bisulfite, 0.32 g of potassium iodide, and 125 mL of methanol were added to a three-necked flask and stirred overnight at 30°C-40°C. The reaction was terminated until most of the raw materials were reacted completely. The temperature of the resulting solution was controlled below 30°C, and the pH value was adjusted to 7.0 by adding concentrated hydrochloric acid. The mixture was extracted with ethyl acetate for 4 times. Then, the organic phases were combined and concentrated at 35°C-45°C to dryness to obtain 20 g of wet white solid. The solid was heated to reflux with 240 mL of tert-butyl methyl ether for 1 h and then cooled. Suction filtration was performed. The filter cake was washed with cold MTBE and dried at 50°C in a blast oven overnight to obtain 8.7 g of d5PAO as a white solid ($^{13}$C-NMR ($\delta$, DMSO-d6): 149.9, 129.6, 129.4, 128.2, MS ES+(m/z): 173.99 [$(M+H)^+$]).

**1.2 Physicochemical properties of d5PAO**

1.2.1 Instruments

Agilent1260Prime high performance liquid chromatograph, Mettler Toledo XS105 balance (0.01 mg), KQ5200B ultrasonic instrument (Kunshan Ultrasonic Instruments Co., Ltd.), BR2000-GM variable speed oscillator (VWR International) and 0.45-$\mu$m filter membrane (Shanghai Qingyang Biotechnology Co., Ltd.).

1.2.2 Experimental drugs

d5PAO (purity: 97.9%), acetonitrile (chromatographically pure, Sinopharm Chemical Reagent Co., Ltd.), and hydrochloric acid and DMSO (analytically pure, Sinopharm Chemical Reagent Co., Ltd.).

1.2.3 Solution preparation

2 mL of 0.1 M hydrochloric acid solution was taken and diluted with deionized water to 20 mL to obtain a 0.01 M hydrochloric acid solution, and the pH was determined as 2 with a precision pH test paper.

2 mL of 0.01 M hydrochloric acid solution was taken and diluted with deionized water to 20 mL to obtain a 0.001 M hydrochloric acid solution.

2 mL of 0.001 M hydrochloric acid solution was taken and diluted with deionized water to 20 mL to obtain a 0.0001 M hydrochloric acid solution, and the pH of the solution was determined as 4 by a precision pH test paper.

20 mL of deionized water was taken, and the pH was determined as 6.

7.5 mg of d5PAO was taken into a centrifuge tube, followed by adding 1 mL of DMSO. The resulting mixture

was ultrasonically oscillated for dissolution and diluted to 10 mL with an acetonitrile/water (1/1) mixed liquid to obtain a 0.75 mg/mL d5PAO stock solution. The d5PAO stock solution was diluted into different d5PAO working solutions (0.3 mg/mL, 0.15 mg/mL, 0.075 mg/mL, 0.03 mg/mL and 0.015 mg/mL).

1.2.4 Chromatographic conditions

Liquid chromatograph: Agilent 1260 Infinity II Prime ultra-high pressure liquid chromatography system.

Chromatographic column: ACQUITY UPLC® Peptide C18130Å 2.1 * 100 mm ID., 1.7 μm (Waters).

Mobile phase A: water : ACN (v : v, 95 : 5) solution containing 0.01% AA and 2 mmol/L NH4OAc.

Mobile phase B: water : ACN (v : v, 5 : 95) solution containing 0.01% AA and 2 mmol/L NH4OAc.

Elution gradient:

| Time (min) | flow rate (mL/min) | A (%) | B (%) |
|---|---|---|---|
| Initial | 0.400 | 95 | 5 |
| 2.00 | 0.400 | 2 | 98 |
| 3.00 | 0.400 | 2 | 98 |
| 3.20 | 0.400 | 95 | 5 |
| 4.00 | 0.400 | 95 | 5 |

Column temperature: 40°C

Feeding volume: 4 μL

Detection wavelength: 254 nm

1.2.5 Methodological investigation

Investigation of linear relationship

Different d5PAO solutions (0.75 mg/mL, 0.3 mg/mL, 0.15 mg/mL, 0.075 mg/mL, 0.03 mg/mL and 0.015 mg/mL) were taken and measured according to the above chromatographic conditions, and the chromatograms were recorded. A linear regression of peak area against sample concentration was performed, and a regression equation was obtained:

$$y = 1647.7x + 0.9623, R^2 = 0.9999$$

The results showed that the d5PAO sample concentration was in the range of 0.015-0.75 mg mL-1 and had a good linear relationship with the peak area.

1.2.6 Determination of equilibrium solubility

Different pH buffer solutions were pipetted (2 mL each) into 3-mL centrifuge tubes. Excess d5PAO powder was added. Until a large amount of white insoluble precipitates appeared in the solution, the resulting mixture was ultrasonically treated for 30 min, put in a thermostatic oscillator, shaken at 25°C for 24 h, then ultrasonically treated for another 30 min and filtered with a 0.45-μm filter membrane. The subsequent filtrate was pipetted, 10-fold diluted with water and measured according to the above chromatographic conditions, and the chromatograms were recorded. The solubility of d5PAO in different pH buffers was calculated as 5.36 mg/mL, 5.39 mg/mL and 5.98 mg/mL, respectively.

Table 1. Solubility of d5PAO

| Drug | pH | Peak area | Corresponding solubility (mg/mL) |
|---|---|---|---|
| d5PAO | 6 | 884.20 | 5.36 |
| d5PAO | 4 | 889.51 | 5.39 |
| d5PAO | 2 | 986.70 | 5.98 |

## 2. Synthesis and physical properties of PAO

### 2.1 Synthesis of PAO

Synthesis route:

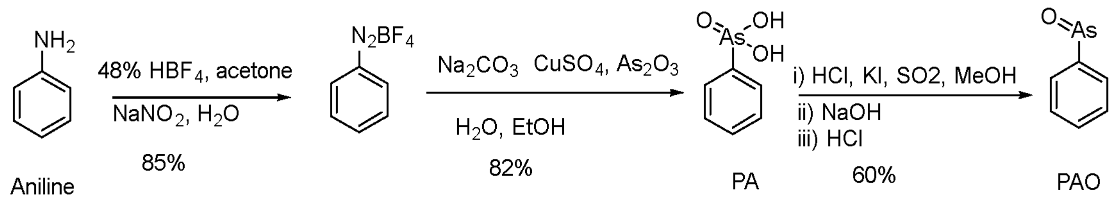


Aniline PA PAO

Step 1:

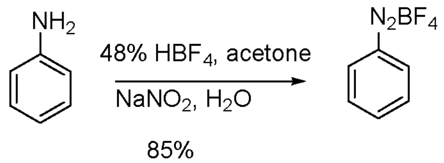


Aniline (10.0 g, 107 mmol, 1.0 eq.) and acetone (20 mL) were added to a 250-mL single-neck flask and cooled to about -15°C (high temperature causes a product to be dark), followed by slowly adding 48% HBF4 (30 mL, 29.5 g, 161 mmol, 1.5 eq.). $NaNO_2$ (11.0 g, 161 mmol, 1.5 eq.) was dissolved in $H_2O$ (20 mL), and the mixture was slowly added dropwise to the above solution. After the addition, the temperature was maintained at -15°C for 2 h, followed by stirring the reaction at 0°C for about 1 h. The solid (white) was filtered and washed with isopropyl ether (50 mL × 2). The product was dried under vacuum at 30°C (weight: 17.5 g, yield: 85%, pink solid) and directly used in the next step.

Step 2:

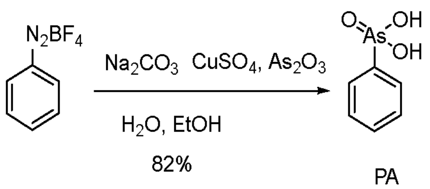


PA

$Na_2CO_3$ (21.2 g, 200.6 mmol, 3.85 eq.), $As_2O_3$ (11.3 g, 57.3 mmol, 1.1 eq.), $CuSO_4$-$5H_2O$ (800 mg, 3.13 mol, 0.06 eq.) and $H_2O$ (60 mL) were added to a 250-mL flask. The suspension was heated to about 90°C for about 20 min so that most of the solids can be dissolved. The solution was cooled to about 0-15°C. A suspension of azobenzene fluoroborate (10.0 g, 52.1 mmol, 1.0 eq.) and $H_2O$ (60 mL) was slowly added in batches, and a small amount of acetone was added to reduce the foam aggregation. After the addition, the mixture was stirred at room temperature for about 12 h, filtered with diatomite and washed with $H_2O$ (20 mL × 3), wherein if the filtrate is too dark, activated carbon can be added for decolorization prior to filtering. Concentrated hydrochloric acid (12 N, 40 mL) was added to the filtrate for neutralization, and the reaction liquid was further adjusted to be acidic. The filtrate was concentrated to about 50 mL. The solid was filtered and washed once with cold water. The filtrate was concentrated to produce solids. The solids were filtered and washed once with cold water. All the solids were combined and dried (white solid, weight: 8.6 g, yield: 82%, MS ES+(m/z): 202.9 [$(M+H)^+$]).

Step 3:

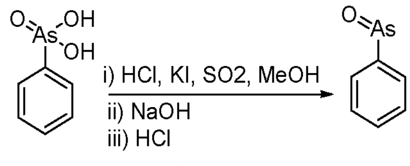


PA PAO

$SO_2$ was passed to a mixture of phenylarsenic acid (8.0 g, 40 mmol, 1.0 eq.), methanol (40 mL), concentrated hydrochloric acid (15 mL) and KI (catalyst amount, 50 mg) until saturation, and the resulting mixture was stirred at room temperature for 2 h. A NaOH solution (2N) was added to the mixture until the solution was transparent. Then, concentrated hydrochloric acid was added for neutralization. A white solid was precipitated out, filtered, recrystallized with water/ethanol (1/5) and dried (weight: 4.0 g, yield: 60%, white powdery solid, $^1$H NMR ($\delta$, $CDCl_3$): 7.41-7.62 (m, 3H), 7.75-7.78 (m, 2H). MS $ES^+$ (m/z): 168.8 [$(M+H)^+$]).

**2.2 Physicochemical properties of PAO**

PAO has a molecular formula of $C_6H_5AsO$ and a molecular weight of 168.03.

2.2.1 Instruments

Agilent1260Prime high performance liquid chromatograph, Mettler Toledo XS105 balance (0.01 mg), KQ5200B ultrasonic instrument (Kunshan Ultrasonic Instruments Co., Ltd.), BR2000-GM variable speed oscillator (VWR International) and 0.45-$\mu$m filter membrane (Shanghai Qingyang Biotechnology Co., Ltd.).

2.2.2 Experimental drugs

PAO (purity: 98%), acetonitrile (chromatographically pure, Sinopharm Chemical Reagent Co., Ltd.), and hydrochloric acid and DMSO (analytically pure, Sinopharm Chemical Reagent Co., Ltd.).

2.2.3 Solution preparation

2 mL of 0.1 M hydrochloric acid solution was taken and diluted with deionized water to 20 mL to obtain a 0.01 M hydrochloric acid solution, and the pH was determined as 2 with a precision pH test paper.

2 mL of 0.01 M hydrochloric acid solution was taken and diluted with deionized water to 20 mL to obtain a 0.001 M hydrochloric acid solution.

2 mL of 0.001 M hydrochloric acid solution was taken and diluted with deionized water to 20 mL to obtain a 0.0001 M hydrochloric acid solution, and the pH of the solution was determined as 4 by a precision pH test paper.

20 mL of deionized water was taken, and the pH was determined as 6.

7.5 mg of PAO was taken into a centrifuge tube, followed by adding 1 mL of DMSO. The resulting mixture was ultrasonically oscillated for dissolution and diluted to 10 mL with an acetonitrile/water (1/1) mixed liquid to obtain a 0.75 mg/mL PAO stock solution. The PAO stock solution was diluted into different PAO working solutions (0.3 mg/mL, 0.15 mg/mL, 0.075 mg/mL, 0.03 mg/mL and 0.015 mg/mL).

2.2.4 Chromatographic condition

Liquid chromatograph: Agilent 1260 Infinity II Prime ultra-high pressure liquid chromatography system.

Chromatographic column: ACQUITY UPLC® Peptide C18130Å 2.1 * 100 mm ID., 1.7 $\mu$m (Waters).

Mobile phase A: water : ACN (v : v, 95 : 5) solution containing 0.01% AA and 2 mmol/L NH4OAc.

Mobile phase B: water : ACN (v : v, 5 : 95) solution containing 0.01% AA and 2 mmol/L NH4OAc.

Elution gradient:

| Time (min) | flow rate (mL/min) | A (%) | B (%) |
|---|---|---|---|
| Initial | 0.400 | 95 | 5 |
| 2.00 | 0.400 | 2 | 98 |
| 3.00 | 0.400 | 2 | 98 |
| 3.20 | 0.400 | 95 | 5 |
| 4.00 | 0.400 | 95 | 5 |

Column temperature: 40°C
Feeding volume: 4 μL
Detection wavelength: 254 nm

2.2.5 Methodological investigation

Investigation of linear relationship

Different PAO solutions (0.75 mg/mL, 0.3 mg/mL, 0.15 mg/mL, 0.075 mg/mL, 0.03 mg/mL and 0.015 mg/mL) were taken and measured according to the above chromatographic conditions, and the chromatograms were recorded. A linear regression of peak area against sample concentration was performed, and a regression equation was obtained: $y = 1834.8x - 4.2355$ $R^2 = 1$
The results showed that the PAO sample concentration was in the range of 0.015-0.75 mg mL-1 and had a good linear relationship with the peak area.

2.2.6 Determination of equilibrium solubility

Different pH buffer solutions were pipetted (2 mL each) into 3-mL centrifuge tubes. Excess PAO powder was added. Until a large amount of white insoluble precipitates appeared in the solution, the resulting mixture was ultrasonically treated for 30 min, put in a thermostatic oscillator, shaken at 25°C for 24 h, then ultrasonically treated for another 30 min and filtered with a 0.45-μm filter membrane. The subsequent filtrate was pipetted, 10-fold diluted with water and measured according to the above chromatographic conditions, and the chromatograms were recorded. The solubility of PAO in different pH buffers was calculated as 1.15 mg/mL, 3.38 mg/mL and 4.52 mg/mL, respectively.

Table 2. Solubility of PAO

| Drug | pH | Peak area | Corresponding solubility (mg/mL) |
|---|---|---|---|
| PAO | 6 | 207.25 | 1.15 |
| PAO | 4 | 616.40 | 3.38 |
| PAO | 2 | 824.35 | 4.52 |

**3. Synthesis and physicochemical properties of deuterated compounds d1PAO, d2PAO and d3PAO**

**3.1 Preparation of d1PAO**

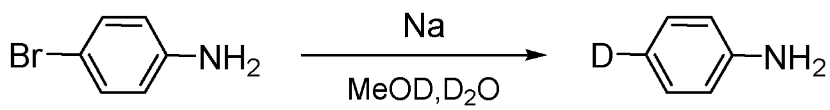


Under nitrogen, parabromoaniline (3.44 g) was dissolved in deuterated methanol (5 mL), and the reaction mixture was refluxed for 30 min, spin-dried 3 times and then dehumidified for use. 3 g of metallic sodium was added to deuterated methanol (10 mL) in batches, and after the reaction was completed, heavy water (30 mL) was slowly added to obtain a 10% solution of sodium deuteroxide in heavy water. Under nitrogen, the product in step 1 was dissolved in deuterated methanol (5 mL). Zinc powder (6.5 g) and the prepared 10% solution of sodium deuteroxide in heavy water

were added. The resulting mixture was heated to reflux for 3 h, cooled to room temperature after parabromoaniline disappeared as detected by TLC, extracted with ether and spin-dried at low temperature to obtain a product, p-deuterated aniline.

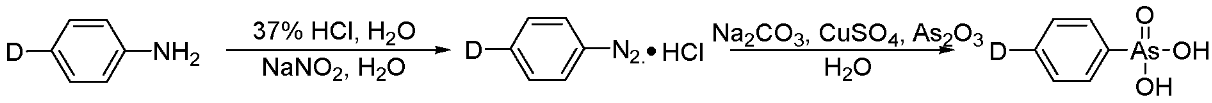


The p-deuterated aniline (0.94 g) obtained in the previous step and water (5 mL) were added to a round-bottomed flask with a magnetic stirring bar and cooled to 0-5°C. At this temperature, a 37% aqueous solution of hydrochloric acid (2 mL) was slowly added, and the mixture was diluted with 5 mL of water. The resulting solution was stirred and reacted for another 30 min. Then, an aqueous solution (724.5 mg, 10.5 mmol, 3 mL of $H_2O$) of $NaNO_2$ was slowly added to the reaction liquid within 30-40 min at 0-5°C to obtain a brownish yellow solution. The brownish yellow solution was stirred and reacted at low temperature for another 2 h. $Na_2CO_3$ (4.0 g, 38.0 mmol, 3.8 eq.), $As_2O_3$ (1.0 g, 5.0 mmol, 0.5 eq.), $CuSO_4 \cdot 5H_2O$ (150 mg, 0.6 mmol, 0.06 eq.) and $H_2O$ (13 mL) were added dropwise to another round-bottomed reaction flask. The mixture was reacted at 95°C for 45 min to obtain a green solution. The green solution was cooled to 0-5°C. The azo hydrochloride prepared in step 1 was slowly added dropwise to the reaction liquid in step 2, and the temperature of the reaction system was controlled to be lower than 5°C. When foam was generated during the dropwise addition, a small amount of acetone was added to remove the foam. When the foam disappeared, the dropwise addition was continued. The dropwise addition was completed within 1 h, and then the temperature was naturally raised. The resulting product was stirred overnight and filtered with diatomite, and the filter cake was rinsed with ice water (2 mL × 2). The aqueous phase was concentrated to 10 mL under reduced pressure at 50°C. The pH was adjusted to 7-8 by dropwise adding 4 mL of 6N HCl in an ice-water bath, with a small amount of yellowish-brown solid appeared. Suction filtration was performed, and then the solid was washed with 2 mL of ice water and discarded. The pH was adjusted to 2-3 by dropwise adding 2.5 mL of 6N HCl to the filtrate, with a lot of bubbles appeared. The filtrate was concentrated under reduced pressure. When a large amount of solids appeared in the system, the temperature was lowered, suction filtration was performed, and then the solids were collected. The pH was adjusted to 1 by adding 6N HCl to the resulting filtrate, and then rotary evaporation was performed. When a large amount of solids appeared in the system, the temperature was lowered, and suction filtration were performed. The mother liquor was washed twice, and the solid was collected. 900 mg of pure solid p-deuterated phenylarsonic acid was obtained (yield: 44%, 1H NMR (δ, DMSO-d6): 7.60 (d, 2H), 7.53 (d, 2H)).

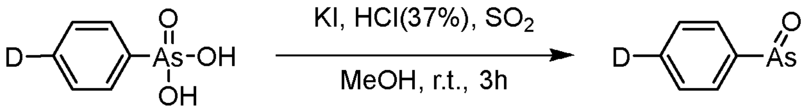


**d1PAO**

P-deuterated phenylarsonic acid (880 mg, 4.3 mmol, 1.0 eq.), KI (16.6 mg, 0.1 mmol, 0.023 eq.), 37% HCl (1.7 mL, 20.0 mmol, 4.6 eq.) and methanol (5.8 mL) were sequentially added to a 25-mL three-necked flask and stirred at room temperature for 5-10 min. $SO_2$ was continuously passed, and the reaction was carried out for 3 h. TLC showed the reaction was completed. The reaction liquid was subjected to suction filtration and washed with methanol (1.5 mL × 2). The pH was adjusted to 14 by dropwise adding 15% NaOH solution to the resulting liquid in an ice-water bath, and the system appeared as an orange cloudy liquid. The mixture was extracted with ethyl acetate (50 mL × 2). The organic phases were combined, dried over anhydrous $Na_2SO_4$, filtered and spin-dried at low temperature (20°C-28°C) to obtain a crude product. About 4 mL of diethyl ether was added to the crude product, and the mixture was pulped for about 30 min, filtered and dehumidified to obtain about 430 mg of DIPAO as an off-white solid ($^1H$ NMR (δ, DMSO-d6): 7.70 (d, 2H), 7.46 (d, 2H), MS $ES^+$ (m/z): 169.9 [$(M+H)^+$], yield: about 59%).

**3.2 Preparation of d2PAO**

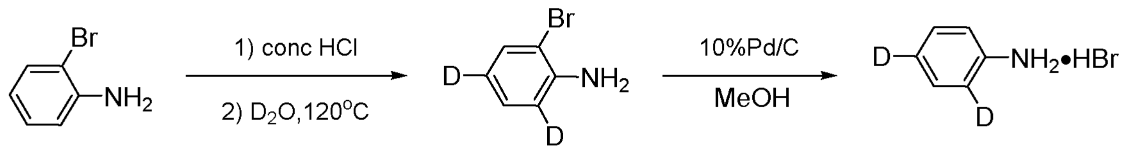


Concentrated hydrochloric acid (5 mL) was added dropwise to a solution of o-bromoaniline (3.44 g) in diethyl ether to obtain a solid. Suction filtration was performed with a sand core funnel, and the mixture was washed with diethyl ether and dehumidified to obtain aniline hydrochloride. Under nitrogen, the solid was dissolved in heavy water (7 mL) in a sealed tube and heated to 120°C, and the reaction was carried out for 24 h. The heavy water was spin-dried, and the nitrogen was replaced. Heavy water (7 mL) was added again, and the reaction was carried out for 48 h. The reactant was cooled to room temperature, and the pH was adjusted to 7 by using 30% sodium hydroxide. The mixture was extracted with diethyl ether, dried over anhydrous sodium sulfate and spin-dried to obtain 2-bromo-4,6-dideuterium-aniline. Under nitrogen, 22 mL of methanol was added to a mixture of 2-bromo-4,6-dideuterium-aniline and 10% Pd/C (300 mg). The nitrogen was replaced by hydrogen, and the reaction was carried out for 3 h. When TLC showed 2-bromo-4,6-dideuterium-aniline disappeared, the resulting reaction liquid was filtered with diatomite, washed with a small amount of methanol and directly spin-dried to obtain 2,4-dideuterium-aniline hydrobromide.

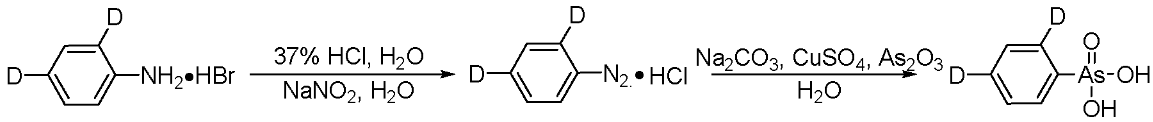


The 2,4-dideuterium-aniline hydrobromide obtained in the previous step and water (5 mL) were added to a 25-mL round-bottomed flask with a magnetic stirring bar and cooled to 0-5°C. At this temperature, a 37% aqueous solution (1.1 mL) of hydrochloric acid was slowly added, and the mixture was diluted with 5 mL of water. The resulting solution was stirred and reacted for another 30 min. Then, an aqueous solution (724.5 mg, 10.5 mmol, 3 mL of $H_2O$) of $NaNO_2$ was slowly added to the reaction liquid within 30-40 min at 0-5°C to obtain a brownish yellow solution. The brownish yellow solution was stirred and reacted at low temperature for another 2 h. $Na_2CO_3$ (4.0 g, 38.0 mmol, 3.8 eq.), $As_2O_3$ (1.0 g, 5.0 mmol, 0.5 eq.), $CuSO_4{\cdot}5H_2O$ (150 mg, 0.6 mmol, 0.06 eq.) and $H_2O$ (13 mL) were added dropwise to a 50-mL round-bottomed reaction flask. The mixture was reacted at 95°C for 45 min to obtain a green solution. The green solution was cooled to 0-5°C. The azo hydrochloride prepared in step 1 was slowly added dropwise to the reaction liquid in step 2, and the temperature of the reaction system was controlled to be lower than 5°C. When foam was generated during the dropwise addition, a small amount of acetone was added to remove the foam. When the foam disappeared, the dropwise addition was continued. The dropwise addition was completed within 1 h, and then the temperature was naturally raised. The resulting product was stirred overnight and filtered with diatomite, and the filter cake was rinsed with ice water (2 mL × 2). The aqueous phase was concentrated to 10 mL under reduced pressure at 50°C. The pH was adjusted to 7-8 by dropwise adding 4 mL of 6N HCl in an ice-water bath, with a small amount of yellowish-brown solid appeared. Suction filtration was performed, and then the solid was washed with 2 mL of ice water and discarded. The pH was adjusted to 3-4 by dropwise adding 2 mL of 6N HCl to the filtrate, with a viscous solid appeared. Suction filtration was performed, and the solid (NMR showed that this solid did not contain the product) was discarded. The resulting filtrate was concentrated to 8 mL, and the pH was adjusted to 2-3 by adding 6N HCl (0.5 mL), with a large amount of solids appeared. Suction filtration was performed to obtain 1.15 g of 2,4-dideuterium-phenylarsonic acid as a solid (yield: 56.4%, $^1H$ NMR (δ, DMSO-d6): 7.72 (d, 1H), 7.56-7.59 (m, 2H)).

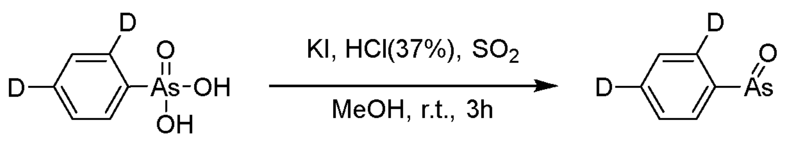


**d2PAO**

2,4-dideuterium-phenylarsonic acid (1.1 g, 5.4 mmol, 1.0 eq.), KI (20.6 mg, 0.124 mmol, 0.023 eq.), 37% HCl (2.1 mL, 25.0 mmol, 4.6 eq.) and MeOH (7.3 mL) were sequentially added to a 25-mL three-necked flask and stirred at room temperature for 5-10 min. $SO_2$ was continuously passed, and the reaction was carried out for 3 h. TLC showed the reaction was completed. The pH was adjusted to 7 by dropwise adding 15% NaOH solution in an ice-water bath, with a large amount of insoluble matter appeared in the system. The mixture was extracted with ethyl acetate (50 mL × 2). The organic phases were combined, dried over anhydrous $Na_2SO_4$, filtered and spin-dried at low temperature

(20°C-28°C) to obtain a crude product. About 3 mL of ethyl acetate was added to the crude product, and the mixture was pulped for about 30 min, filtered and dehumidified to obtain about 400 mg of d2PAO as an off-white solid ($^1H$ NMR (δ, DMSO-d6): 7.58 (d, 1H), 7.36-7.39 (m, 2H). MS $ES^+$ (m/z): 170.7 [(M+H)+], yield: about 48%).

### 3.3 Preparation of d3PAO

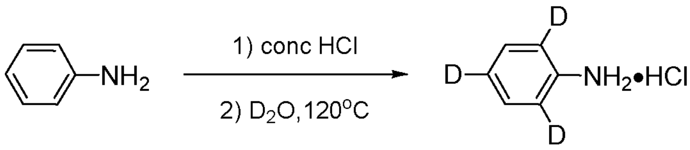


Concentrated hydrochloric acid (5 mL) was added dropwise to an aniline solution (2.65 g of aniline, 7 mL of heavy water) to obtain a solid. Suction filtration was performed with a sand core funnel, and the mixture was washed with diethyl ether and dehumidified to obtain aniline hydrochloride, which was directly used in the next step. Under nitrogen, the solid was dissolved in heavy water (7 mL) in a sealed tube and heated to 120°C, and the reaction was carried out for 24 h. The heavy water was spin-dried, and the nitrogen was replaced. Heavy water (7 mL) was added again, and the reaction was carried out for 48 h to obtain a solution of 2,4,6-trideuterium-aniline in heavy water, which was directly used in the next step.

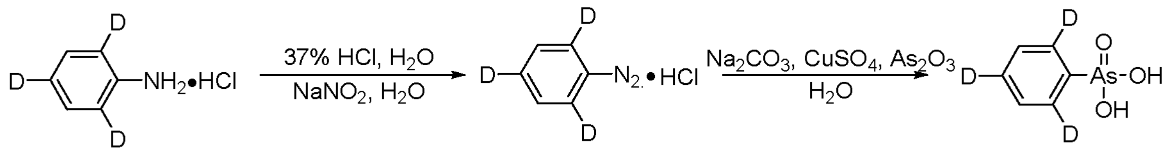


The solution (4.33 mL) of 2,4,6-trideuterium-aniline in heavy water and water (2.5 mL) were added to a 25-mL round-bottomed flask with a magnetic stirring bar and cooled to 0-5°C. At this temperature, a 37% aqueous solution (1.1 mL) of hydrochloric acid was slowly added, and the mixture was diluted with 5 mL of water. The resulting solution was stirred and reacted for another 30 min. Then, an aqueous solution (724.5 mg, 10.5 mmol, 3 mL of $H_2O$) of $NaNO_2$ was slowly added to the reaction liquid within 30-40 min at 0-5°C to obtain a solution turned from purple to yellow. The yellow solution was then stirred and reacted at low temperature for another 2 h. $Na_2CO_3$ (4.0 g, 38.0 mmol, 3.8 eq.), $As_2O_3$ (1.0 g, 5.0 mmol, 0.5 eq.), $CuSO_4 \cdot 5H_2O$ (150 mg, 0.6 mmol, 0.06 eq.) and $H_2O$ (13 mL) were added dropwise to a 50-mL round-bottomed reaction flask. The mixture was reacted at 95°C for 45 min to obtain a green solution. The green solution was cooled to 0-5°C. The azo hydrochloride prepared in step 1 was slowly added dropwise to the reaction liquid in step 2, and the temperature of the reaction system was controlled to be lower than 5°C. When foam was generated during the dropwise addition, a small amount of acetone was added to remove the foam. When the foam disappeared, the dropwise addition was continued. The dropwise addition was completed within 1 h, and then the temperature was naturally raised. The resulting product was stirred overnight and filtered with diatomite, and the filter cake was rinsed with ice water (2 mL × 2). The aqueous phase was concentrated to 10 mL under reduced pressure at 50°C. The pH was adjusted to 7-8 by dropwise adding 1.8 mL of 6N HCl in an ice-water bath, with a small amount of yellowish-brown solid appeared. Suction filtration was performed, and then the solid was washed with 2 mL of ice water and discarded. The pH was adjusted to 3 by dropwise adding 1.8 mL of 6N HCl to the filtrate, with a faint yellow solid appeared. Suction filtration was performed, and then the solid was washed with 2 mL of ice water and collected. The resulting filtrate was concentrated to 8 mL, and the pH was adjusted to 1 by adding 0.8 mL of 6N HCl, with a large amount of solids appeared. Suction filtration was performed, and the solids were collected to obtain 860 mg of 2,4,6-trideuterium-phenylarsonic acid as a solid in total (yield: 39%, $^1H$ NMR (δ, DMSO-d6): 7.56 (s, 2H)).

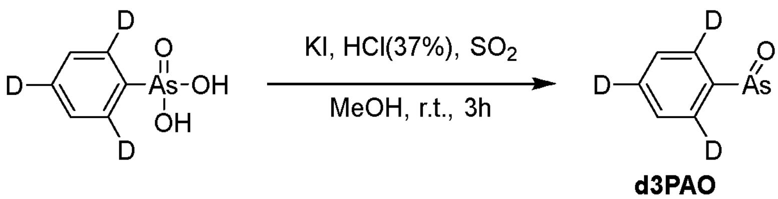


**d3PAO**

2,4,6-trideuterium-phenylarsonic acid (3.9 mmol, 1.0 eq.), KI (15 mg, 0.09 mmol, 0.023 eq), 37% HCl (1.3 mL, 18.0 mmol, 4.6 eq.) and methanol (5.3 mL) were sequentially added to a 25-mL three-necked flask and stirred at room temperature for 5-10 min. $SO_2$ was continuously passed, and the reaction was carried out for 3 h. TLC showed the reaction was completed. The reaction liquid was subjected to suction filtration and washed with methanol (1.5 mL × 2). The pH was adjusted to 7 by dropwise adding 4.3 mL of 17% NaOH solution in an ice-water bath, with yellow oily substances appeared on the flask wall. The mixture was extracted with ethyl acetate (25 mL × 2). The organic phases were combined, dried over anhydrous NaSO4, filtered and spin-dried at low temperature (20°C-28°C). About 3 mL of ethyl acetate was added to the obtained solid, and the mixture was pulped for about 30 min, filtered and dehumidified to obtain about 200 mg of d3PAO as an off-white solid. The mother liquor obtained in the previous step was spin-dried, and about 1.5 mL of $Et_2O$ was added to the obtained solid, and the mixture was pulped for about 30 min, filtered and dehumidified to obtain about 170 mg of d3PAO as an off-white solid. The d3PAO was combined (yield: about 55%, $^1H$ NMR (δ, DMSO-d6): 7.46 (s, 2H). MS $ES^+$ (*m*/*z*): 171.9 [$(M+H)^+$]).

**Example 2. Pharmacokinetic study of d5PAO and PAO**

d5PAO (d5-PAO) was prepared by the method described in example 1, and PAO was prepared by the company. After adult male rat subjects received single-dose intravenous injection (the dose is 0.1 mg/kg, and the compound is dissolved in 0.1% DMSO) or single-dose oral gavage (the dose is 0.2 mg/kg) of PAO and d5PAO, venous blood was drawn at 0, 0.083, 0.25, 0.5, 1, 2, 4, 6, 8, 24, 32 and 48 h post-administration for pharmacokinetic tests. The PAO and d5PAO in test samples were extracted by means of protein precipitation. The treated samples were loaded to a liquid chromatography-mass spectrometer/mass spectrometer (LC-MS/MS) and detected in an ESI negative ion mode after liquid-phase separation.

Sample treatment (blood samples):

1) 40 μL of unknown samples, a calibration standard , a quality control sample, a single blank sample and a double blank sample were added into a 96-well plate;
2) 120 μL of 0.1 mg/mL sodium dimercaptosulphonate in water was added to each sample;
3) 40 μL of 0.2% formic acid in water was added to each sample; the resulting mixture was mixed uniformly, then incubated with shaking at 45°C for 15 min and centrifuged at 4°C for 5 min (×3220 g);
4) each sample (except from the double blank sample) was quenched with 200 μL of IS1 (the double blank sample was quenched with 240 μL of MeOH), shaken and mixed for 15 min and then centrifuged at 4°C for 15 min (×3220 g); and
5) 50 μL of supernatants were transferred to the 96-well plate, centrifuged at 4°C for 5 min (×3220 g) and then analyzed by LC-MS/MS via the Triple Quad6500⁺LC-MS/MS (SCIEX) system.

Experimental results:

no significant difference was observed in the pharmacokinetic indexes of PAO and d5PAO at the same dose administered to male SD rats (n = 3) via single-dose intravenous injection (0.1 mg/kg) or single-dose oral gavage (0.2 mg/kg)(see table 3, table 4 and FIGs. 1 and 2). The bioavailability of the 0.1 mg/kg gavage group was 15.7%.

Table 3. Pharmacokinetic indexes of PAO and d5PAO at the same dose administered to male SD rats via single-dose intravenous injection (0.1 mg/kg)

| | PAO (IV, 0.1 mg/kg) | d5PAO (IV, 0.1 mg/kg) |
|---|---|---|
| PK Parameters | Mean ± SD | Mean ± SD |
| No. points used for $T_{1/2}$ | 3.00 | 3.00 |
| $C_0$ (ng/mL) | 1344.67 ± 210.97 | 1487.67 ± 141.76 |
| $T_{1/2}$ (h) | 11.4 ± 0.03 | 12.43 ± 0.65 |
| $Vd_{ss}$ (L/kg) | 0.16 ± 0.02 | 0.155 ± 0.02 |
| C1 (mL/min/kg) | 0.41 ± 0.03 | 0.38 ± 0.02 |
| $T_{last}$ (h) | 48.0 | 48.000 |
| $AUC_{0-last}$ (ng.h/mL) | 4010 ± 252.82 | 4337 ± 280.63 |
| $AUC_{0-inf}$ (ng.h/mL) | 4061.67 ± 258.10 | 4404.33 ± 275.54 |

(continued)

| | PAO (IV, 0.1 mg/kg) | d5PAO (IV, 0.1 mg/kg) |
|---|---|---|
| $MRT_{0\text{-last}}$ (h) | 5.84 ± 0.44 | 5.87 ± 0.049 |
| $MRT_{0\text{-inf}}$(h) | 6.58 ± 0.45 | 6.79 ± 0.65 |
| $AUC_{Extra}$(%) | 1.267 ± 0.05 | 1.54 ± 0.27 |
| $AUMC_{Extra}$(%) | 12.47 ± 0.67 | 14.87 ±1.46 |

Table 4. Pharmacokinetic indexes of PAO and d5PAO administered to male SD rats via single-dose oral gavage (0.2 mg/kg)

| | PAO (PO, 0.2 mg/kg) | d5PAO (PO, 0.2 mg/kg) |
|---|---|---|
| PK Parameters | Mean ± SD | Mean ± SD |
| No. points used for $T_{1/2}$ | 3.00 | 3.00 |
| $C_{max}$ (ng/mL) | 156 ± 12.53 | 161 ± 12.5 |
| $T_{max}$ (h) | 8.00 ± 0.00 | 7.33 ± 1.15 |
| $T_{1/2}$ (h) | 6.87 ± 0.94 | 7.19 ± 0.78 |
| $T_{last}$ (h) | 48.00 | 48.00 |
| $AUC_{0\text{-last}}$ (ng.h/mL) | 2313.67 ± 419.51 | 244.5.00 ± 474.53 |
| $AUC_{0\text{-inf}}$ (ng.h/mL) | 2341 ± 425.87 | 2478.67 ± 483.09 |
| $MRT_{0\text{-last}}$ (h) | 12.53 ± 0.61 | 12.63 ± 0.78 |
| $MRT_{0\text{-inf}}$(h) | 13.03 ± 0.75 | 13.27 ± 0.9 |
| $AUC_{Extra}$(%) | 1.16 ± 0.47 | 1.35 ± 0.44 |
| $AUMC_{Extra}$(%) | 5.09 ± 1.99 | 5.92 ± 1.8 |
| Bioavailability (%)[a] | 28.82 | 28.14 |

**Example 3. Comparison of effects on cell viability and pharmacodynamics of d5PAO and PAO**

Cell culture and drug administration: the complete culture system of SH-SY5Y was high-glucose DMEM (Gibco) supplemented with 15% FBS (Gibco). A plasmid was transfected by the Fugene HD transfection reagent (Promega, Beijing Biotech Co., Ltd. Catalog No. E2311). The plasmid was purchased from Obio Technology (Shanghai Corp., Ltd), and the vector map was shown in FIG. 3. The α-synuclein overexpression plasmid has a sequence of

ATGGATGTATTCATGAAAGGACTTTCAAAGGCCAAGGAGGGAGTTGTGGCTGCTGCTGAGAAAAC
CAAACAGGGTGTGGCAGAAGCAGCAGGAAAGACAAAAGAGGGTGTTCTCTATGTAGGCTCCAAAACCA
AGGAGGGAGTGGTGCATGGTGTGGCAACAGTGGCTGAGAAGACCAAAGAGCAAGTGACAAATGTTGG
AGGAGCAGTGGTGACGGGTGTGACAGCAGTAGCCCAGAAGACAGTGGAGGGAGCAGGGAGCATTGCA
GCAGCCACTGGCTTTGTCAAAAAGGACCAGTTGGGCAAGAATGAAGAAGGAGCCCCACAGGAAGGAAT
TCTGGAAGATATGCCTGTGGATCCTGACAATGAGGCTTATGAAATGCCTTCTGAGGAAGGGTATCAAGA
CTACGAACCTGAAGCCTAA （SEQ ID NO:1）

The stably-transformed APP (SW) HEK293 cell line was a human embryonic kidney cell line transfected with Swedish double-mutation APP695 cDNA. Before the cells were seeded, the plate was treated with 20 μg/mL poly-D-lysine (PDL) for 24 h. The culture solution was high-glucose DMEM supplemented with 10% FBS, and 200 μg/mL G418 was added for selection. After the cells were seeded into the plate for 48 h, starvation was performed, that is, the serum was removed and only the high-glucose DMEM medium was used. After 24 h of culturing , a complete culture system was used for replacement, and drugs were administered.

Cell viability was detected by Thiazolyl Blue (MTT) assay.

At 12 h post-administration, MTT with a final concentration of 0.5 mg/mL was added, and the mixture was

incubated for 4 h, followed by pipetting the culture solution. 100 μL of DMSO was added to dissolve the adsorbed MTT, the residue was shaken for 15 min, and then the absorbance value was read.

ELISA

a) The α-synuclein monoclonal antibody (Mouse monoclonal) for α-synuclein ELISA was purchased from Sigma-Aldrich (Shanghai, Catalog No. S5566); and the α-synuclein ELISA Kit was purchased from Thermo Fisher Scientific (Catalog No. KHB0061). 50 μL of Huα-synuclein Detection Antibody solution was added to each well (except from chromogen blanks empty, namely the chromogen blank wells), and 50 μL of samples and standard curve samples (see FIG. 4 for preparation of the standard curve samples) were added to each well (except from chromogen blanks empty). The resulting mixture was gently shaken and mixed uniformly. The plate was covered with a film and incubated at 4°C overnight. The wells were fully washed with 100 μL of 1× Wash buffer 4 times, and 100 μL of Anti-Rabbit IgG HRP to each well, except from the chromogen blank wells. The plate was covered with a film, incubated at room temperature for 30 min and then fully washed with 1 × Wash buffer four times. 100 μL of Stabilized Chromogen was added to each well. The solution turned blue and then was incubated at room temperature in the dark for 30 min. 100 μL of Stop Solution was added to each well. The resulting mixture was gently shaken and mixed uniformly. The solution turned yellow, and the NovoStar microplate reader (BMG company, Germany) was used to read at an absorption wavelength of 450 nm.

b) Aβ ELISA

The Amyloid beta42Human ELISA Kit was purchased from Thermo Fisher Scientific (Catalog No. KHB3544). 100 μL of diluted standard curve samples, 100 μL of blank controls and 100 μL of samples were added into the corresponding wells of an assay plate. The plate was covered with a film and incubated at 37°C for 2 h. The liquid in each well was discarded, and then 100 μL of Detection Reagent A Working Solution was added to each well. The plate was covered with a film and incubated at 37°C for 1 h. The supernatant was discarded. Then, each well was washed with 1 × Wash buffer 3 times, 2 min each time, keeping the liquid residue as little as possible. 100 μL of Detection Reagent B Working Solution was added to each well, and the plate was covered with a film and incubated at 37°C for 1 h. Washing was repeated 5 times. 90 μL of Substrate Solution was added to each well, and the plate was covered with a film and incubated at 37°C in the dark for 20 min. The solution turned blue. 50 μL of Stop Solution was added to each well. The resulting mixture was shaken gently and mixed uniformly. The solution turned yellow, and a microplate reader was used to read at an absorption wavelength of 450 nm as soon as possible.

Propidium Iodide (PI) staining

PI (Cell Signaling Technology, Catalog No. 4087) was added 15 min before immunofluorescent staining. The mixture was co-incubated in a cell incubator for 15 min, followed by immunofluorescent staining. The longest excitation and emission wavelengths of the PI/RNase staining solution were 535 nm and 617 nm, respectively.

Immunofluorescent staining

The supernatant was aspirated from the cells treated with a drug and the like. Next, the residue was washed with pre-cooled PBS three times, treated with 4% PFA, left to stand at room temperature for 30 min and then washed with PB-S three times (10 min each time). Triton-X was dissolved in PBS to prepare a 0.1% Triton-X solution, and treatment was performed for 15 min. The solution was blocked with 10% donkey serum for 1 h. Primary antibody: Mouse anti-Ki67 (Cell Signaling Technology, Catalog No. 9129); Secondary antibody: anti-Mouse Alex488.

Statistical analysis

Data were processed and analyzed by GraphPad Prism5 software. One-way ANOVA and mean ± SEM were involved, and $p < 0.03$ indicated a statistical difference.

Experimental results:

effects of d5PAO and PAO on the viability of SH-sy5y cells

The complete culture system for SH-sy5y cell lines is high-glucose DMEM supplemented with 15% FBS. In the cytotoxicity experiment, SH-sy5y cells were cultured for 48 h and treated with different concentrations of d5PAO and PAO for 24 h. Thiazolyl Blue (MTT) with a final concentration of 0.5 mg/mL was added, and the mixture was incubated for 4 h, followed by pipetting the culture solution. 100 μL of DMSO was added to dissolve the adsorbed MTT, the residue was shaken for 15 min, and then the absorbance value was read. The results showed that d5PAO at 6.25 nM, 25 nM, 50 nM, 100 nM and 200 nM significantly promoted the proliferation of SH-sy5y cells, while PAO at 200 nM showed

toxicity and caused cell death after 24 h of treatment. In order to further detect the toxic effects of different concentrations of d5PAO and PAO on SH-sy5y cells, the effects of d5PAO and PAO on the apoptosis/death and proliferation of SH-sy5y cells were investigated by PI staining and immunofluorescent staining of Ki67. PI (propidium iodide), as a fluorescent dye that can be inserted between DNA and RNA bases and dyes, cannot pass through living cell membranes, but can pass through damaged cell membranes to stain the nuclei of apoptotic/dead cells. However, Ki67 is an indispensable protein in cell proliferation, and its function is closely related to mitosis. Therefore, Ki67 is often used to mark cells in the proliferation cycle, and in clinical applications, cell tumors with a high Ki67-positive rate are generally considered to grow faster. PI staining and Ki67 staining were performed after 24 h of treatment with different concentrations of d5PAO and PAO. The results showed that PAO at 50 nM and 100 nM and d5PAO at 50 nM and 100 nM all did not significantly increase Ki67-positive rates, and PI-positive cells were reduced in comparison with the control group (ctrl), indicating that d5PAO and PAO reduced cell apoptosis or death, but did not significantly promote cell proliferation (FIG. 5).

**Example 4. Effects of d5PAO and PAO on viability and Aβ release of stably-transformed APP (SW) HEK293 cells**

The stably-transformed APP (SW) HEK293 cell line was a human embryonic kidney cell line that was transfected with Swedish double-mutation amyloid precursor protein (APP) 695 cDNA and carried a G418 selection marker. Before the cells were seeded, the plate was treated with 20 μg/mL poly-D-lysine (PDL) for 24 h. The culture solution was high-glucose DMEM supplemented with 10% FBS, and 200 μg/mL G418 was added for selection. After 48 h of culturing, different concentrations of d5PAO and PAO were added, and treatment was performed for 24 h. Thiazolyl Blue (MTT) with a final concentration of 0.5 mg/mL was added, and the mixture was incubated for 4 h, followed by pipetting the culture solution. 100 μL of DMSO was added to dissolve the adsorbed MTT, the residue was shaken for 15 min, and then the absorbance value was read.

In the experiments of detecting the effects of d5PAO and PAO on the Aβ release of APP(SW)HEK293 cells. After 48 h of culturing, starvation was performed for 24 h, and then a complete culture system was used for replacement. The cells were treated with compounds d5PAO and PAO for 4 h, and the Aβ level in the supernatant of the cell culture solution was detected by ELISA.

Experimental results:

The stably-transformed APP (SW) HEK293 cells were treated with d5PAO at 25 nM, 50 nM, 100 nM and 200 nM for 24 h, leading to a significantly increased cell viability in comparison with the control group. Previous studies have shown that PAO can promote the release of amyloid β-protein (Aβ) and other proteins. The Aβ in the supernatant of the stably-transformed APP (SW) HEK293 cells was detected with an ELISA kit, and the results showed that: d5PAO at 25 nM, 50 nM and 100 nM and PAO at 25 nM, 50 nM and 100 nM significantly increased extracellular Aβ contents (FIG. 6).

**Example 5. Comparison of effects of other deuterated compounds on Aβ release of stably-transformed APP (SW) HEK293 cells**

Three deuterated compounds, d1PAO, d2PAO and d3PAO, of PAO were selected. The culture method and administration method for APP(SW) HEK293 cells are the same as those in example 4. The groups involved: a control group (ctrl), a d5PAO50nM treatment group, a d5PAO100nM treatment group, a PAO50 nM treatment group, a PAO75nM treatment group, a d1PAO25nM treatment group, a d1PAO50 nM treatment group, a d1PAO75nM treatment group, a d2PAO25nM treatment group, a d2PAO50 nM treatment group, a d2PAO75nM treatment group, a d3PAO25nM treatment group, a d3PI0350 nM treatment group and a d3PAO75nM treatment group. The results showed that: in comparison with the control group, the extracellular Aβ contents in the 50 nM and 75 nM d5PAO treatment groups, the 50 nM and 75 nM PAO treatment groups, the 25 nM, 50 nM and 75 nM d1PAO treatment groups, the 25 nM, 50 nM and 75 nM d2PAO treatment groups and the 25 nM, 50 nM and 75 nM d3PAO treatment groups were all significantly increased. By comparing the d5PAO50 nM treatment group with other groups (except the control group), the 50 nM and 75 nM PAO treatment groups, the 25 nM, 50 nM and 75 nM d1PAO treatment groups, the 25 nM and 75 nM d2PAO treatment groups and the 25 nM and 75 nM d3PAO treatment groups had significant differences in terms of the Aβ contents in cell culture supernatants (FIG. 7A and FIG. 7B).

**Example 6. Effects of d5PAO and PAO on α-synuclein secretion**

An SH-SY5Y cell line was transfected with an α-synuclein overexpression (α-syn OE) plasmid by using the Fugene HD transfection reagent. Starvation was performed for 24 h, and then a complete culture system was used for replacement. The cells were treated with compounds d5PAO and PAO for 24 h, and the effects of d5PAO and PAO on the viability of SH-sy5y cells transfected with the α-synuclein plasmid were detected by MTT. The results showed that

the α-synuclein overexpression significantly decreased the viability of SH-sy5y cells, and d5PAO at 25 nM, 50 nM, 75 nM, 100 nM and 200 nM and PAO at 50 nM, 75 nM and 100 nM significantly increased the viability of SH-sy5y cells overexpressing α-synuclein in comparison with the α-synuclein overexpression group. The α-synuclein in cell supernatants was detected by an ELISA kit. The results showed that: 50 nM d5PAO significantly increased the α-synuclein content in the supernatant, and PAO and d5PAO showed a similar tendency to increasing α-synuclein (FIG. 8).

**Example 7. Therapeutic effects of d5PAO and PAO on Gaucher disease**

1. Inhibitory effects of d5PAO and PAO on CBE-induced apoptosis or death of SH-SY5Y cells

Conduritol Bepoxide (CBE) is an inhibitor of the GBA1 enzyme encoded by lysosomal glucocerebrosidase GBA genes, and is commonly used to construct cell and animal models of Gaucher disease (GD). SH-SY5Y cells were treated with CBE for 48 h, leading to a concentration-dependent decrease of cell viability of the SH-SY5Y cells (FIG. 9A). 100 μM of CBE was selected for subsequent experiments. SH-SY5Y cells were treated with 100 μM of CBE for 24 h and co-incubated with a certain concentration of d5PAO or PAO according to different groups for 24 h. The cell viability was detected by MTT. The experimental results showed that in comparison with the 100 μM CBE treatment group, the cell viability in the 100 μM CBE+25 nM, 50 nM and 100 nM d5PAO co-treatment groups and the 100 μM CBE+25 nM, 50 nM and 100 nM PAO co-treatment groups was significantly increased (FIGs. 9B and 9C), indicating that d5PAO or PAO have protective effects against CBE-induced apoptosis or death of SH-SY5Y cells.

2. Effect of PAO on reducing lysosomal storage and promoting glucosylceramide (GlcCer) efflux

GD is a common type of lysosomal storage disease. Whether d5PAO and PAO alleviate the CBE-induced lysosomal storage was investigated by Lyso-tracker red DND99, a lysosomal marker. The experimental results showed that in comparison with the control group (ctrl), the fluorescent intensity in the 100 μM CBE treatment group and 100 μM CBE Lyso-tracker was increased (FIGs. 10A and 10B), indicating that CBE treatment leads to lysosome storage in SH-SY5Y cells. In comparison with the 100 μM CBE treatment group, the fluorescent intensity of Lyso-tracker in the 100 μM CBE+50 nM and 100 nM PAO co-treatment groups was significantly decreased (FIGs. 10A and 10B). The fundamental defect of GD lies in lack of the activity of glucocerebrosidase. However, this enzyme mainly mediates the process of decomposing glucocerebroside into glucose and GlcCer. Therefore, the storage of substrates such as GlcCer occurs in GD patients or CBE-treated cells. To further investigate whether PAO has an effect on GlcCer storage in an SH-SY5Y cell model, the GlcCer contents of different side chains in cells and in the supernatants of cell culture mediums were detected by LC-MS. The detection results showed that the GlcCer concentrations of various side chains in the cells in the 100 μM CBE treatment group was much higher than that in the control group (ctrl), and the GlcCer concentration in the 100 μM CBE+50 nM PAO co-treatment group was lower than that in the 100 μM CBE treatment group (FIG. 10C). The 100 μM CBE treatment alone reduced the GlcCer in the supernatants of cell culture mediums, while 100 μM CBE+50 nM PAO co-treatment group increased the GlcCer concentration (FIG. 10D).

3. Effect of *PI4Ka* knockdown on promoting a reduction in lysosomal storage

Previous studies have shown that PAO at a low concentration (< 5 μM) mainly acts on phosphatidylinositol 4 kinase (PI4KIIIα). In order to further investigate the effect of PAO on the target spot PI4KIIIα during fibrosis, shRNA interference lentivirus vectors (with green fluorescent protein GFP expression sequence) were designed for a gene sequence PI4Ka encoding PI4KIIIα protein. The Western blot results showed that after SH-SY5Y cells were transfected with three shRNA interfering lentiviral vectors targeting PI4Ka for 48 h, interfering sequences sh1, sh2 and sh3 all significantly decreased the expression level of PI4KIIIα in the treatment groups (FIGs. 11A and 11B). After the cells were treated with shRNA interfering lentiviral vectors for 48 h, the fluorescent intensity of Lyso-tracker was observed by immunofluorescent staining. In comparison with the sh-ctrl group, the immunofluorescent intensity of Lyso-tracker in the sh-ctrl+100 μM CBE co-treatment group was significantly increased. In comparison with the sh-ctrl+100 μM CBE co-treatment group, the immunofluorescent intensity of Lyso-tracker in the sh1-PI4Ka+100 μM CBE co-treatment group was significantly decreased (FIG. 11C). The above results indicated that the PI4Ka knockdown promoted a reduction in lysosomal storage.

**Example 8. Inhibitory effects of d5PAO and PAO on pulmonary fibrosis**

Pulmonary fibrosis (PF), as a chronic fibrotic lung disease that may be caused by various factors, is mainly manifested by dry cough and progressive dyspnea. It has poor prognosis and is still difficult to cure. The main pathological feature of PF is excessive scar repair after the destruction of a normal lung tissue structure, which eventually leads to

respiratory insufficiency. Although research on the pathophysiological mechanism of pulmonary fibrosis has made great progress, its pathogenesis has not been fully elucidated, and effective therapeutic drugs are lacked clinically.

Human fetal lung fibroblasts, namely MRC-5 cells, are important cell tools for studying the pathological changes of PF and the drug development. During the development of PF, related transcription factors such as transforming growth factor-1 (TGF-β1) can regulate the abnormal activation, proliferation and migration of fibroblasts, resulting in abnormal deposition of an extracellular matrix (ECM) and destruction of an alveolar structure, which eventually leads to the formation of PF. TGF-β1, as one of the key factors in PF induction, can regulate the transformation of fibroblasts into myofibroblasts by binding to the corresponding receptors. Therefore, MRC-5 cells are usually treated with a certain concentration of TGF-β1 in an experimental process to promote the development of fibrosis. d5PAO and PAO are oxophenylarsine and a variant thereof, respectively. Preliminary studies showed that PAO has the potential to inhibiting PF.

**Experimental methods:**

Culture and treatment of MRC-5 cells

MRC-5 cells were purchased from the Center for Excellence in Molecular Cell Science, the Chinese Academy of Sciences. According to the cell culture instructions, the cells were incubated in MEM (Gibco) containing 10% fetal bovine serum (FBS) in a 37°C, 5% $CO_2$ constant-temperature incubator with saturated humidity for 24 h to adhere to the wall. The next day, 5 ng/mL TGF-β1 (Proteintech Group, HZ-1011) was added, and MEM (Gibco) containing different concentrations of PAO or d5PAO was added according to groups. The cells were cultured for further 24 h according to experimental needs. The groups are as follows: a control group (ctrl), a 5 ng/mL TGF-β1 group, a 5 ng/mL TGF-β1+50 nM d5PAO co-treatment group (5 ng/mL TGF-β1+50 nM d5PAO group), a 5 ng/mL TGF-β1+25 nM d5PAO co-treatment group (5 ng/mL TGF-β1+25 nM d5PAO group), a 5 ng/mL TGF-β1+50 nM PAO co-treatment group (5 ng/mL TGF-β1+50 nM PAO group) and a 5 ng/mL TGF-β1+25 nM PAO co-treatment group (5 ng/mL TGF-β1+25 nM PAO group).

Primary culture and treatment of rat mesenchymal stem cells

6-week-old Sprague-Dawley (SD) rats (Shanghai Slack Experimental Animal Co., Ltd.) were anesthetized with chloral hydrate, sterilized with 75% ethanol, and dissected on a super clean bench to take the tibia and femur out. Two ends of the tibia and femur were removed with a sterilized scissor to expose the marrow cavity. The marrow cavity was flushed with 5 mL of MEM containing 10% FBS. The flushing fluid containing the marrow was added to a culture dish, filtered through a 70-μm cell strainer and centrifuged at 2000 rpm for 3 min. The supernatant was removed, and the cells were resuspended in MEM containing 10% FBS. After the cells were seeded to a plate, the plate was incubated in a 37°C, 5% $CO_2$ constant-temperature incubator with saturated humidity for 6 h. Then, non-adherent cells were removed by means of medium change. When growing to 80% density, the cells were digested with 2.5% trypsin for 1 min and passaged at a ratio of 1 : 2. The mesenchymal stem cells for immunofluorescence experiments are seeded on a coverslip of a 24-well plate, 3000-5000 cells per well. When the cell density reached 70% as observed, MEM (free of FBS) containing different concentrations of PAO or d5PAO was added according to groups. Next, the plate was incubated in a 37°C, 5% $CO_2$ constant-temperature incubator with saturated humidity for 24 h. The groups are as follows: a control group (ctrl), a 50 nM d5PAO treatment group, a 25 nM d5PAO treatment group, a 50 nM PAO treatment group and a 25 nM PAO treatment group.

Immunofluorescent staining

After MRC-5 cells were treated for 24 h, the supernatant was aspirated. Phosphate buffer saline (PBS) was added for washing 3 times, and 4% paraformaldehyde (PFA) was added. The resulting mixture was left to stand at room temperature for 30 min. 4% PFA was discarded. Then, PBS was added for washing 3 times, 10 min each time, and 0.3% non-ionic detergent Triton-X (in PBS) was added. The resulting mixture was left to stand at room temperature for 30 min. 0.3% Triton-X was aspirated. PBS blocking buffer containing 10% goat serum (GS) was added. The mixture was left to stand at room temperature for 1 h. A primary antibody was incubated overnight at 4°C (the primary antibody was diluted with 10% GS blocking buffer at 1 : 200, α-Smooth Muscle Actin (rabbit anti-α-Smooth Muscle Actin, Cell Signaling Technology #19245), and the Calponin1 dilution ratio was 1 : 100).

The next day, PBS was added for washing 3 times, 10 min each time. A secondary antibody Alexa Flour555 goat-anti-rabbit IgG (Molecular Probes) was incubated, diluted with blocking buffer at 1 : 500, and 1 μg/mL 4',6-diamidino-2-phenylindole (DAPI) was added). The resulting mixture was left to stand at room temperature for 2 h. PBS was added for washing 3 times, 10 min each time. A mounting medium was used to adhere the coverslip to the slide, followed by observation under a microscope. The immunofluorescent staining process of bone marrow mesenchymal stem cells was the same as above.

Western blot experiment:

1) a cell culture dish was placed on ice, the supernatant was aspirated, and the cells were washed with pre-cooled PBS 3 times;
2) a cell lysate was added at 120 μL/well, and the cell culture dish was horizontally placed on ice for 30 min;
3) the lysate was scraped away by using a cell scraper and collected into a 1.5-mL EP tube;
4) centrifugation was performed at 15000 g for 15 min, the supernatant was collected into a new EP tube, 5 μL of samples was taken for protein content detection (BCA method), and 1/4 (volume) of loading buffer was added to the remaining lysate;
5) the resulting mixture was boiled in water for 5 min;
6) 10% SDS separation gel was prepared, and stacking gel was prepared;
7) proteins of different molecular weights were separated by SDS-PAGE gel electrophoresis;
8) wet transfer buffer was prepared, and a 0.45-μm PVDF membrane was used for a membrane transfer experiment;
9) 5% skim milk was added for blocking at room temperature for 1 h;
10) after washing, a primary antibody was incubated at 4°C overnight;
11) the next day, a TBST solution was added for washing 3 times, a secondary antibody was incubated and then placed at room temperature for 2 h; and
12) an ECL developer was added for color developing, and GE was added.

Enzyme-linked immunosorbent assay: Human Collagen Type IELISA Kit

8 samples in total, include: a control group (ctrl), a 5 ng/mL TGF-β1 group, a 5 ng/mL TGF-β1+50 nM d5PAO co-treatment group (5 ng/mL TGF-β1+50 nM d5PAO group), a 5 ng/mL TGF-β1+25 nM d5PAO co-treatment group (5 ng/mL TGF-β1+25 nM d5PAO group), a 5 ng/mL TGF-β1+50 nM PAO co-treatment group (5 ng/mL TGF-β1+50 nM PAO group) and a 5 ng/mL TGF-β1+25 nM PAO co-treatment group (5 ng/mL TGF-β1+25 nM PAO group).

The Human Collagen Type IELISA Kit was purchased from Novus Biologicals (Catalog No. NBP2-30102), and the following experimental steps were performed according to the instructions:

1) standard curve samples (4000 pg/mL, 2000 pg/mL, 1000 pg/mL, 500 pg/mL, 250 pg/mL, 125 pg/mL, 62.5 pg/mL and 0 pg/mL) were prepared by using Standard Diluent;
2) 100 μL of samples to be tested (3 replicate wells) or standard curve samples were added to each well, and the plate was sealed with a sealing film and incubated at 37°C for 2 h;
3) the supernatant was aspirated, and no washing was required;
4) 100 μL of Detection Reagent A solution was added to each well, and the plate was sealed with a sealing film and incubated at 37°C for 1 h;
5) the supernatant was discarded, 350 μL of 1 ×Wash solution was added to each well for washing in an oscillator for 2 min, and the plate was reversely placed on dust-free paper to remove the supernatant by tapping, and then consecutively washed 3 times;
6) 100 μL of Detection Reagent B solution was added to each well, and the plate was sealed with a sealing film and incubated at 37°C for 30 min;
7) the supernatant was discarded, 350 μL of 1 ×Wash solution was added to each well for washing in an oscillator for 2 min, and the plate was reversely placed on dust-free paper to remove the supernatant by tapping, and then consecutively washed 5 times;
8) after that, 90 μL of TMB substrate was added to each well, the liquid in the plate gradually turned blue, and the plate was sealed with a sealing film and left to stand at room temperature for 10 min;
9) 50 μL of Stop solution was added to each well, and the liquid in the plate turned yellow; and
10) a microplate reader was used to read at an absorption wavelength of 450 nM, and data was analyzed.

Construction and treatment of shRNA lentivirus vector

An shRNA lentiviral vector for *PI4Ka* interference was designed and produced by Genomeditech (Shanghai) Co., Ltd. Vector information: pGMI,V-SCS RNAi vector (FIG. 12).

The following target spots were designed:

| | TargetSeq |
|---|---|
| sh-ctrl | TTCTCCGAACGTGTCACGT |
| sh1 | GCTGATCTCTACTACACTTCC |
| sh2 | GGTTATCACCGGAAATCAATA |
| sh3 | GCAACATTATGCTGGACAAGA |

The MRC-5 cells were incubated in MEM containing 10% fetal bovine serum (FBS) in a 37°C, 5% $CO_2$ constant-temperature incubator with saturated humidity for 24 h to adhere to the wall. The next day, the lentiviral vector was added at 1 μL/well according to groups. 24 h later, 5 ng/mL TGF-β1 was added according to groups, and the mixture was co-incubated for 24 h. The proteins were collected for Western blot experiments or immunofluorescent staining.

**Statistics**

Fluorescent intensity processing was performed by using Image J software, data processing and statistics were performed by using GraphPad prism 5 software, and the data were expressed as mean ± standard error of the mean (mean ± SEM). The differences between the groups were compared and analyzed by one-way ANOVA, and $p < 0.03$ indicated a statistical difference.

**Experimental results:**

MRC-5 cells were treated with MEM (free of FBS) containing 5 ng/mL TGF-β1 for 24 h and treated with different concentrations of d5PAO or PAO according to groups. In comparison with the control group, TGF-β1 used alone or in combination with a certain concentration of d5PAO or PAO did not cause significant cell death or apoptosis (FIG. 13). α-smooth muscle actin (α-SMA) and actin-binding protein (Calponin1) are markers of myofibroblasts. To investigate whether d5PAO and PAO have regulatory effects on pulmonary fibrosis, α- SMA and calponin1 were separately detected. The Western blot experiment results showed that: in comparison with the control group (ctrl), the expression level of α-SMA in the 5 ng/mL TGF- β1 treatment group was significantly increased, indicating that MRC-5 cells treated with 5 ng/mL TGF- β1 for 24 h lead to cellular fibrosis, while d5PAO at 25 nM, 50 nM and 100 nM and PAO at 50 nM and 100 nM significantly inhibited the α-SMA overexpression induced by 5 ng/mL TGF-β1 in a dose-dependent relationship (FIGs. 14A and 14B). The Western blot experiment results of calponin1 were similar to the above results of α-SMA. In comparison with the ctrl group, the expression level of Calponin1 in the 5 ng/mL TGF- β1 treatment group was significantly increased, and the expression level of Calponin1 in a certain concentration of d5PAO or PAO+5 ng/mL TGF- β1 co-treatment groups was significantly higher than that in the 5 ng/mL TGF- β1 treatment group (FIGs. 14A and 14C). In addition, the immunofluorescence assay results were also consistent with the Western blot results, and the expression level of α-SMA in the 5 ng/mL TGF-β1 treatment group was significantly higher than that in the control group (FIGs. 15A and 15C). The expression level of α-SMA in the 5 ng/mL TGF-β1+25 nM d5PAO, 5 ng/mL TGF-β1+50 nM d5PAO, 5 ng/mL TGF-β1+25 nM PAO and 5 ng/mL TGF-β1+50 nM PAO co-treatment groups did not differ significantly from that in the control group. However, the expression level of α-SMA in the 5 ng/mL TGF-β1+25 nM d5PAO, 5 ng/mL TGF-β1+50 nM d5PAO, 5 ng/mL TGF-β1+25 nM PAO and 5 ng/mL TGF-β1+50 nM PAO co-treatment groups was significantly decreased in comparison with the 5 ng/mL TGF-β1 treatment group (FIGs. 15A and 15C). The immunofluorescence assay results of the actin-binding protein (Calponin1) were consistent with the above results of α-SMA (FIGs. 15B and 15D). These results indicated that d5PAO and PAO can significantly inhibit the fibrosis process of MRC-5 cells that is induced by TGF-β1.

***Inhibitory effects of d5PAO and PAO on expression of Calponin1 in bone mesenchymal stem cells (bMSC)***

Pulmonary fibrosis lacks effective therapeutic drugs and methods, and stem cells have become a hot research field in the treatment of pulmonary fibrosis in recent years due to their unique biological properties and potential biomedical application values. Mesenchymal stem cells (MSCs) have become ideal engineering cells for wound tissue repair, organ function reconstruction and cell therapy own to their advantages such as low immunogenicity, diverse differentiation potential, immune regulation, anti-inflammatory abilities, a wide source, easiness to culture in an isolated manner and less ethical disputes. Therefore, MSCs derived from rat bones were cultured in an isolated manner, and whether d5PAO and PAO regulate the expression of Calponin1 in MSCs was detected. The isolated MSCs were cultured in MEM containing 10% FBS and then were seeded on a coverslip of a 24-well plate in the immunofluorescence experiment, 3000-5000 cells per well. When the cell density reached 70% as observed, MEM (free of FBS) containing different

concentrations of PAO or d5PAO was added according to groups. Next, the plate was incubated in a 37°C, 5% $CO_2$ constant-temperature incubator with saturated humidity for 24 h. Immunofluorescence assay results showed that d5PAO at 25 nM and 50 nM and PAO at 25 nM and 50 nM treating MSCs for 24 h had a tendency to inhibiting the expression of Calponin1 in the MSCs (FIG. 16).

***Inhibitory effects of d5PAO and PAO on secretion of collagen type I (COL1) from MRC-5 cells treated with TGF-β1***

COL1 is one of the important components of the extracellular matrix. Studies have shown that TGF-β1 significantly increases the secretion of COL1 in the fibrosis process of MRC-5 cells. In order to further investigate whether d5PAO and PAO can regulate the secretion of COL1 in the process of inhibiting pulmonary fibrosis, the COL1 level in the cellular supernatant was detected by ELISA. MRC-5 cells were treated with MEM (free of FBS) containing 5 ng/mL TGF-β1 for 24 h, and treated with different concentrations of d5PAO or PAO according to groups for 24 h. The supernatant was collected for ELISA. The experiment results showed that: in comparison with the ctrl group, the COL1 concentration in the cellular supernatant in the 5 ng/mL TGF-β1 treatment group was significantly increased, and the COL1 level in the cellular supernatant in the 5 ng/mL TGF-β1+25 nM d5PAO, 5 ng/mL TGF-β1+50 nM d5PAO, 5 ng/mL TGF-β1+100 nM d5PAO, 5 ng/mL TGF-β1+25 nM PAO, 5 ng/mL TGF-β1+50 nM PAO and 5 ng/mL TGF-β1+100 nM PAO co-treatment groups was significantly decreased in comparison with the 5 ng/mL TGF-β1 treatment group (FIGs. 17A and 17B), indicating that d5PAO and PAO can inhibit the secretion of COL1 in an MRC-5 cell model.

***Inhibitory effect of PI4Kα knockdown on fibrosis of MRC-5 cells***

Previous studies have shown that PAO at a low concentration (< 5 μM) mainly acts on phosphatidylinositol 4 kinase (PI4KIIIα). In order to further investigate the effect of PAO on the target spot PI4KIIIα during fibrosis, shRNA interference lentivirus vectors (with green fluorescent protein GFP expression sequence) were designed for a gene sequence PI4Ka encoding PI4KIIIα protein. The Western blot results showed that after MRC-5 cells were transfected with three shRNA interfering lentiviral vectors targeting PI4Ka for 48 h, interfering sequences sh1, sh2 and sh3 all significantly decreased the expression level of PI4KIIIα in the treatment groups (FIGs. 18A and 18B). Treatment with shRNA interfering lentiviral vectors was performed for 48 h, and then the expression of Calponin1 and α-SMA was observed by means of immunofluorescent staining. In comparison with the vector control group (sh-ctrl), the expression level of α-SMA in the sh-ctrl+5 ng/mL TGF-β1 co-treatment group was significantly increased. In comparison with the sh-ctrl+5 ng/mL TGF-β1 co-treatment group, the expression level of α-SMA in the sh1+5 ng/mL TGF-β1 co-treatment group and the sh3+5 ng/mL TGF-β1 co-treatment group was significantly decreased, and the expression level of α-SMA in the sh2+5 ng/mL TGF-β1 co-treatment group also showed a decreased tendency (FIGs. 19A and 19C). The observation results of Calponin1 are consistent with those of α-SMA (FIGs. 19B and 19D), which indicate that the *PI4Kα* knockdown inhibits the fibrosis of MRC-5 cells.

**Example 9. Comparison of anti-inflammatory effects of d5PAO and PAO**

**Inhibitory effects of d5PAO and PAO on release of inflammatory factors in murine microglial cells BV2**

BV2 cells are immortalized by retrovirus-mediated transfection of murine microglial cells with v-raf/v-myc, and retain many morphological, representational and functional features of microglial cells. In experiments related to nervous system inflammation, lipopolysaccharide (LPS) is commonly used to stimulate the BV2 cells to obtain an inflammatory cell model for the experiment. In this experiment, LPS was used to stimulate BV2 cells to obtain an inflammatory cell model. The effects of d5PAO and PAO on the secretion of inflammatory factors such as tumor necrosis factor a (TNF-α) and interleukin-6 (IL-6) were observed, and indomethacin, a commonly used anti-inflammatory drug, was used as a positive control in the experiment.

Cell culture and treatment

BV2 cells were cultured in high-glucose DMEM supplemented with 10% FBS in a 37°C, 5% $CO_2$ cell incubator for 48 h. The culture medium was removed and replaced with high-glucose DMEM (free of FBS) containing 1 μg/mL LPS (lipopolysaccharide, purchased from Sigma, Catalog No. L2880). Different concentrations of d5PAO or PAO were incubated with the cells for 24 h according to groups. The supernatant was collected and centrifuged for use in subsequent experiments. The groups are as follows: a control group (ctrl), a 1 μg/mL LPS group, a 1 μg/mL LPS+50 nM d5PAO co-treatment group (1 μg/mL LPS+50 nM d5PAO group), a 1 μg/mL LPS+25 nM d5PAO co-treatment group (1 μg/mL LPS+25 nM d5PAO group), a 1 μg/mL LPS+12.5 nM d5PAO co-treatment group (1 μg/mL LPS+12.5 nM d5PAO), a 1 μg/mL LPS+50 nM PAO co-treatment group (1 μg/mL LPS+50 nM PAO group), a 1 μg/mL LPS+25 nM PAO co-treatment

group (1 μg/mL LPS+25 nM PAO group), a 1 μg/mL LPS+12.5 nM PAO co-treatment group (1 μg/mL LPS+12.5 nM PAO group) and a 1 μg/mL LPS+100 μM indometacin co-treatment group.

Mouse tumor necrosis factor alpha (TNF-α) ELISA

The mouse tumor necrosis factor alpha (TNF-α) ELISA kit was purchased from Signalway Antibody LLC, Catalog No. EK16997. The following experiments were performed according to product instructions:

1) Diluent buffer was used to prepare standards at 10 ng/mL, 5 ng/mL, 2.5 ng/mL, 1.25 ng/mL, 0.625 ng/mL, 0.312 ng/mL, 0.156 ng/mL and 0.
2) Detection Reagent A and Detection Reagent B were gently oscillated, and Diluent buffer was used to dilute Reagent A and Reagent B to 1/100 of the mother liquor to reach a working solution concentration.
3) Wash solution was diluted with deionized water to 1/30 of the mother liquor to form a working solution.
4) 100 μL of samples to be tested or standards were added to each well, and the plate was sealed with a sealing film and incubated at 37°C for 2 h;
5) the supernatant was removed, and no washing was required;
6) 100 μL of Detection Reagent A working solution was added to each well, and the plate was sealed with a sealing film and incubated at 37°C for 1 h;
7) the supernatant was discarded, 300 μL of Wash solution working solution was added for washing 3 times, standing for 2 min each time, and then the plate was reversely placed on absorbent dust-free paper to remove the Wash solution as much as possible;
8) 100 μL of Detection Reagent B working solution was added to each well, and the plate was sealed with a sealing film and incubated at 37°C for 1 h;
9) the plate was washed 5 times according to the method in step 7);
10) 90 μL of Substrate solution was added to each well, the liquid turned blue as observed in the plate, and the plate was sealed with a sealing film and incubated at 37°C in the dark for 20 min;
11) 50 μL of Stop solution was added to each well, the blue liquid turned yellow as observed in the plate, the plate was sealed with a sealing film, and the resulting mixture was rotated and shaken using a shaker and mixed uniformly for 10 min; and
12) the absorbance value of the sample at a wavelength of 450 nM was read by using a microplate reader (NOVOstar liquid transfer type microplate reader).

Mouse IL-6 ELISA experiment

The mouse IL-6 ELISA kit was purchased from Proteintech group, Catalog No. KE10007. The following experiments were performed according to product instructions:
100 μL of standard curve samples or test samples at each concentration was added to the corresponding wells of an assay plate, chromogen blank wells were reserved; and a cover film was placed in a humidifying box, and the plate was incubated at 37°C for 2 h. 350 μL of Wash Solution was added to each well for washing 4 times, 1-2 min each time, keeping the liquid residue as little as possible. 100 μL of Diluent Antibody Solution (Detection Antibody Solution) was added to each well; and a cover film was placed in a humidifying box, and the plate was incubated at 37°C for 1 h. Washing was repeated 4 times, 1-2 min each time, keeping the liquid residue as little as possible. 100 μL of HRP-Conjugate Antibody was added to each well; and a cover film was placed in a humidifying box, and the plate was incubated at 37°C for 40 min. Washing was repeated 4 times, keeping the liquid residue as little as possible. 100 μL of TMB Substrate Solution was added to each well, and the plate was covered with a film and incubated at 37°C in the dark for 15 min. The solution turned blue. 100 μL of Stop Solution was added to each well, and the mixture was shaken gently and mixed uniformly. The solution turned yellow, and the NOVOstar (liquid transfer type) microplate reader was used to read at an absorption wavelength of 450 nm as soon as possible.

Statistics

Data processing and statistics were performed by using GraphPad prism 5 software, and the data were expressed as mean ± standard error of the mean (mean ± SEM). The differences between the groups were compared and analyzed by one-way ANOVA, and $p < 0.03$ indicated a statistical difference.

Experimental results

BV2 cells were cultured in high-glucose DMEM supplemented with 10% FBS in a 37°C 5% $CO_2$ cell incubator

for 48 h, and then the culture medium was removed and replaced with high-glucose DMEM (free of FBS) containing 1 μg/mL LPS. The cells were co-incubated with different concentrations of d5PAO or PAO for 24 h, the supernatant was collected, and the concentrations of IL-6 and TNF-α in the culture mediums were detected by means of ELISA (enzyme-linked immunosorbent assay). The experimental results showed that in comparison with the control group (ctrl), the concentrations of IL-6 and TNF-α in the supernatant of BV2 cells in the 1 μg/mL LPS treatment group were significantly increased, indicating that the treatment of BV2 cells with 1 μg/mL LPS promoted the release of inflammatory factors (FIGs. 20A to 20D). In comparison with the 1 μg/mL LPS treatment group, the 100 μM positive drug (indomethacin) significantly inhibited the secretion of TNF-α (FIGs. 20A to 20B). Similar to the results of the positive drug, the concentrations of TNF-α in the supernatant in the 1 μg/mL LPS+12.5 nM, 25 nM and 50 nM d5PAO co-treatment groups, and the 1 μg/mL LPS+12.5 nM, 25 nM and 50 nM PAO co-treatment groups were significantly reduced in comparison with the 1 μg/mL LPS treatment group, indicating that d5PAO and PAO can inhibit the LPS-induced release of TNF-α. The ELISA results of IL-6 showed that treatment with a certain concentration of d5PAO and PAO had a tendency to inhibiting the LPS-induced secretion of IL-6 from BV2 cells (FIGs. 20C to 20D). The above experiments showed that a certain concentration of d5PAO and PAO can inhibit the LPS-induced release of inflammatory factors from BV2 cells.

**Example 10. Comparison of anti-tumor effects of d5PAO and PAO**

1. Pharmacodynamic experiment of PAO and d5PAO on tumor cell model

Experimental method

Cell plating:

a complete medium was prepared and mixed uniformly. Thawed primary tumor cells (Shanghai ChemPartner) were passaged for about two generations to select cell lines with good growth conditions. Cell adhering: the culture medium was aspirated, trypsin was added for washing, the waste liquid was discarded, and 3 mL of fresh trypsin was added to a culture flask for digestion. When the cells were about to detach from a flask wall, 8 mL of complete mediums were added to stop digestion with trypsin , followed by gentle mixing. The cell suspension was pipetted into a centrifuge tube with a pipette and centrifuged at 1000 rpm for 4 min. Cell suspending: the cell suspension was pipetted into a centrifuge tube and centrifuged at 1000 rpm for 4 min. The supernatant was discarded. An appropriate volume of culture medium was added to the centrifuge tube, and the cells were resuspended uniformly by means of gentle blowing. The cells were counted by using the Vi-Cell XR cell counter. The cell suspension was adjusted to an appropriate concentration.

Preparation and addition of compound plate

Compounds to be tested: a 10 mM solution of compounds in DMSO was prepared, and compounds PAO and d5PAO were diluted into a 0.5 mM solution in DMSO. The compounds were added to the corresponding cell wells by using an HPD300 instrument and incubated in a $CO_2$ incubator for 72 h.

Reagent preparation and testing

CellTiter-Glo Buffer was melted at room temperature. The lyophilized CellTiter Glo substrate was equilibrated to room temperature. CellTiter-Glo Buffer was added to the CellTiter Glo Substrate and fully mixed uniformly. A cell plate was taken out and equilibrated to room temperature. 100 microlitres of uniformly mixed CellTiter Glo reagent was added to each well, and the mixture was shaken in the dark for 10 min, followed by incubation for 10 min. A culture plate was placed into the Envision reading plate, and the luminescence reading results were recorded. The inhibition rate was calculated according to the following formula: inhibition rate (%) = (1-(RLU compound-RLU blank)/(RLU DMSO-RLU blank)) × 100%. A pharmacodynamic inhibition rate curve was plotted by using XLFit, and the IC50 value was calculated. The 4-parameter model [fit = (a+((B-A)/(1+((C/x) ^ D)))] was used.

**Experimental results:**

as shown in table 5 below, both d5PAO and PAO had inhibitory effects on the tumor cells tested, and their inhibitory effects ($IC_{50}$) were similar. The $IC_{50}$ of d5PAO was slightly low. They had the strongest inhibitory effects on U2-OS and A-375 cells, with the $IC_{50}$ less than 50 nM, and had relatively strong inhibitory effects on HeLa, SK-HEP-1, Daudi, EL4, HL-60, Jurkat, Clone E6-1 and NAMALWA cells, with the $IC_{50}$ of 50-100 nM. The inhibitory effects on A-431 cells were the weakest, with the $IC_{50}$ of about 300 nM. However, the $IC_{50}$ was 100-200 nM for other cells. In comparison with PAO (original compound) and d5PAO (pentadeuterated compounds), the effective inhibition concentration of the monodeuterated compound d1PAO on most tumor cells was greater than 200 nM, indicating that the inhibitory effect of the monodeuterated compound on tumor cells was not as good as that of PAO and d5PAO.

Table 5. List of inhibitory effects of d5PAO and PAO on tumor cells (cultured for 72 h)

| **Cell line** | | **PAO** | **d5PAO** | **d1PAO** | **Paclitaxe l** |
|---|---|---|---|---|---|
| | | $IC_{50}$ (nM) | $IC_{50}$ (nM) | Abs$IC_{50}$ (nM) | $IC_{50}$ (nM) |
| HeLa | Cervical cancer | 87.01 | 87.78 | > 200 | 10.66 |
| SK-HEP-1 | Liver cancer | 85.73 | 83.09 | | 4.69 |
| MCF7 | Breast cancer | 137.09 | 151.95 | > 200 | 23.36 |
| MDA-MB-231 | Breast cancer | 105.55 | 106.42 | > 200 | > 10000 |
| A549 | Lung cancer | 216.39 | 225.21 | > 200 | 3.95 |
| NCI-H1299 | Lung cancer | 116.97 | 120.85 | > 200 | 17.69 |
| HT-29 | Colon cancer | 175.72 | 168.06 | > 200 | 4.19 |
| A-431 | Skin cancer | 344.36 | 312.71 | > 200 | 3.08 |
| NCIN87[N87] | Gastric cancer | 161.28 | 153.93 | > 200 | 7.76 |
| HCC827 | Lung cancer | 180.53 | 179.63 | > 200 | 5.88 |
| EL4 | T-cell lymphoma | 60.22 | 52.68 | | 55.00 |
| Daudi | Leukemia | 63.42 | 59.30 | 97.66 | 4.71 |
| U-2OS | Osteosarcoma | 49.51 | 48.49 | 71.17 | 16.77 |
| Jurkat, Clone E6-1 | Leukemia | 93.01 | 91.06 | 175.35 | 3.83 |
| K-562 | Myeloma | 51.92 | 52.10 | | 6.53 |
| HL-60 | Leukemia | 49.94 | 51.14 | > 200 | 2.82 |
| SK-OV-3 | Ovarian cancer | 207.68 | 199.59 | > 200 | 9.90 |
| U-937 | Lymphoma | 85.09 | 85.79 | 149.63 | 3.97 |
| NAMALWA | Leukemia | 61.09 | 60.73 | 68.66 | 3.66 |
| KG-1 | Myeloma | 151.10 | 151.32 | 176.84 | 76.80 |
| A-375 | Melanoma | 33.23 | 34.96 | 50.76 | 3.98 |
| BxPC-3 | Pancreatic cancer | > 200 | > 200 | - | - |
| ZR-75-1 | Breast ductal carcinoma | 28.54 | 50-100 | | |
| 4T1 | Breast cancer | 47.54 | 50-100 | | |
| B 6F 10 | Melanoma | 20.88 | < 50 | | |
| A20 | B cell lymphoma | 64.42 | < 50 | | |
| Raji-Luc | Lymphoblast | 110 | > 100 | | |
| SU-DHL-1 | Lymph node large cell lymphoma | 100-200 | 100-200 | | |
| LOVO | Colon cancer | > 200 | 100-200 | | |
| HepG2 | Liver cancer | 105 | 100-200 | | |
| NCI-H358 | Non-small cell lung cancer | 75.4 | ~100 | | |
| OVCAR-3 | Ovarian cancer | | 50-100 | | |
| SNU-5 | Gastric cancer | | 100-200 | | |
| LLC1 | Lung cancer | 162.3 | | | |

2. Pharmacodynamic experiment of PAO and d5PAO on mouse tumor model

Experimental animals were raised in an SPF barrier facility at the animal center of Beijing Biocytogen Co., Ltd. The temperature of the barrier system was controlled to 20°C-26°C and the humidity was controlled to 40%-70%. The light-dark cycle was shifted every 12 h. The SPF-grade growth and reproduction feed was purchased from Beijing Keao Xieli Feed Co., Ltd. Drinking water was acidified water (pH 2.5-3.0) which has been sterilized by autoclaving. Animals have free access to sterile food and water

2.1 Inhibitory effect of PAO on breast cancer

Experimental methods:

Inoculation and grouping of PDX models

After the PDX tumor for inoculation grew to about 800-1000 $mm^3$, the tumor was taken out under aseptic conditions and placed in an RPMI1640 culture medium, non-tumor tissues such as calcifications and secretions were removed, and then the tumor was cut into small pieces of uniform size (3 × 3 × 3 mm) and used for subcutaneous inoculation of a breast cancer PDX model (BP1395). The skin of the right lateral thoraxes of mice was disinfected with iodophor and cut with a scissor to make an incision of about 3-5 mm, and the tumor pieces were inoculated subcutaneously on the right lateral thoraxes of the mice by using an inoculation needle. When the mean tumor volume reached about 100 $mm^3$, mice with a moderate tumor volume were picked and randomly divided to 4 experimental groups according to tumor volumes, 8 mice per group. The administration began on the day of grouping. All groups were administered orally (P.O.) the test product PAO on the day of grouping, once a day for a total of 20 doses. Paclitaxel was administered intravenously (i.v.) once a week for a total of 4 doses. Animals were euthanized with excess $CO_2$ at the end of the experiment or at the humane endpoint.

Tumor volume: After grouping, the tumor volume was measured by using a vernier caliper twice a week. Before euthanasia, the tumor volume was measured, and the long and short diameters of the tumor were measured. The volume calculation formula is as follows: tumor volume = 0.5 × long diameter × short diameter$^2$.

Weight measurement: Animals were inoculated, grouped (i.e., before the first dose), weighed twice a week during administration and weighted before euthanasia.

Drug evaluation index:

tumor volume inhibition rate ($TGI_{TV}$)

$$TGI\ (\%) = [1-(Ti-T0)/(Vi-V0)] \times 100\%$$

(Ti: the mean tumor volume of the treatment groups on day i post-administration, T0: the mean tumor volume of the treatment groups on day 0 of administration, Vi: the mean tumor volume of the solvent control group on day i post-administration, and V0: the mean tumor volume of the solvent control group on day 0 of administration)

Tumor weight inhibition rate ($TGI_{TW}$):

at the end of the experiment, the living animals were euthanized, and then the tumor tissues were excised. The tumor weight was weighed, and the tumor weight differences of each group were calculated to further calculate the tumor weight inhibition rate ($TGI_{TW}$). The calculation formula is as follows:

$$\text{tumor weight inhibition rate } TGI_{TW}\% = (W_{\text{solvent control group}} - W_{\text{treatment group}})/W_{\text{solvent control group}} \times 100\%,$$

Data collection and statistical analysis:

analysis was performed on the basis of the original data, and the results were expressed as mean ± standard error of the mean (Mean ± SEM). At the same time, the tumor volume was statistically analyzed, and $P < 0.05$ indicated a statistical difference. Both statistical and biological significance were considered when the results were analyzed.

Experimental results:

in the experiment, all animals had good activity and eating situations during administration. The weights of non-tumor-bearing mice were increased to a certain extent, and the weights of tumor-bearing mice were decreased slightly, indicating that the animals had a good tolerance to the test product. On day 21 after grouping and administration, the mean tumor volume of the solvent control group G1 was 436 $\pm$ 40 $mm^3$. The mean tumor volumes of the treatment groups G2 (PAO, 2 mg/kg), G3 (paclitaxel, 7 mg/kg) and G4 (paclitaxel, 7 mg/kg+PAO, 2 mg/kg) were 519 $\pm$ 96 $mm^3$, 290 $\pm$ 63 $mm^3$ and 162 $\pm$ 26 $mm^3$ respectively, and the tumor volume inhibition rates ($TGI_{TV}$) were -25.7%, 44.8% and 84.1% (P = 0.01), respectively. The results showed that the combined use of PAO and paclitaxel can effectively inhibit the tumor growth (FIG. 21).

2.2 Inhibitory effect of PAO on lymphoma

Experimental methods:

mouse lymphoma SU-DHL-1 cells, purchased from ATCC, were cultured in a Dulbecco's Modified Eagle's culture medium containing 10% inactivated fetal bovine serum in a 37°C, 5% $CO_2$ incubator.

Inoculation and grouping of tumor cells:

the SU-DHL-1 lymphoma cells resuspended in PBS were subcutaneously inoculated on the right side of the B-NDG humanized mice, at 1 $\times$ $10^7$ cells/0.1 mL and 0.1 mL/mouse. When the mean tumor volume reached about 100 $mm^3$, 36 mice with a moderate tumor volume were picked and randomly divided to 6 experimental groups according to tumor volumes, 6 mice per group. The administration began on the day of grouping. The test product PAO was administered once a day for a total of 17 doses. Cyclophosphamide was subcutaneously (i.v.) injected once a week for a total of 4 doses. Animals were euthanized with excess $CO_2$ at the end of the experiment or at the humane endpoint.

Experimental results:

in the experiment, all animals had good activity and eating situations during administration. The weights of non-tumor-bearing mice were increased to a certain extent, and the weights of tumor-bearing mice were decreased slightly, indicating that the animals had a good tolerance to the test product. On day 17 after grouping and administration, the mean tumor volume of the solvent control group G1 was 3899 $\pm$ 272 $mm^3$. The mean tumor volumes of the treatment groups G2 (cyclophosphamide, 50 mg/kg), G3 (PAO, 0.3 mg/kg), G4 (PAO, 0.6 mg/kg), G5 (PAO, 1.2 mg/kg) and G6 (cyclophosphamide, 50 mg/kg+PAO 0.6, mg/kg) were 367 $\pm$ 79 $mm^3$, 3427 $\pm$ 128 $mm^3$, 3784$\pm$114 $mm^3$, 3735$\pm$205 $mm^3$ and 497$\pm$106 $mm^3$, respectively. The tumor volume inhibition rates $TGI_{TV}$ were -93% (**P < 0.001), 17.2%, 3%, 4.3% and 89.9% (P < 0.001 **), respectively. The experimental results of the tumor weight were also confirmed. Cyclophosphamide can effectively inhibit the growth of lymphoma, and the combined use of PAO and cyclophosphamide cannot further improve the inhibitory effect (FIG. 22).

2.3 Inhibitory effect of d5PAO on melanoma

Experimental methods:

A2058 cells were subcutaneously inoculated on the right sides of B-NDG mice, at 1 $\times$ $10^7$ cells/0.1 mL and 0.1 mL/mouse. When the mean tumor volume reached about 100 $mm^3$, 48 mice with a moderate tumor volume were picked and randomly divided to 6 experimental groups according to tumor volumes, 8 mice per group. The groups involved G3 (normal saline/vehicle), G4 (d5PAO, 0.5 mg/kg), G5 (d5PAO, 1.5 mg/kg), G6 (temozolomide, 30 mg/kg+d5PAO, 0.5 mg/kg), G7 (temozolomide, 30 mg/kg+d5PAO, 1.5 mg/kg) and G8 (temozolomide, 30 mg/kg). Meantime, 16 non-tumor-bearing mice were picked according to their weights and divided equally into 2 experimental groups (8 mice per group), namely G1 (normal saline/vehicle) and G2 (d5PAO, 1.5 mg/kg). The test product d5PAO was intragastrically administered to all groups on the day of grouping, once a day for a total of 23 doses. The test product temozolomide was administered 4 times a week for a total of 12 doses. The weights and tumor volumes of the mice were measured twice a week during administration and observation, and the measurements were recorded (FIG. 23). At the end of the experiment, the animals were euthanized, the tumors were excised, weighed and photographed, and the tumor growth inhibition rate (TGI%) was calculated.

Experimental results:

in the experiment, all animals had good activity and eating situations during administration. The weights of non-tumor-bearing mice were increased to a certain extent, and the weights of tumor-bearing mice were decreased slightly, indicating that the animals had a good tolerance to the test product. On day 25 after grouping and administration, the mean tumor volume of the solvent control group G3 was 2806 ± 240 $mm^3$. The mean tumor volumes of the treatment groups G4 (d5PAO 0.5 mg/kg), G5 (d5PAO 1.5 mg/kg), G6 (temozolomide, 30 mg/kg+d5PAO, 0.5 mg/kg), G7 (temozolomide, 30 mg/kg+d5PAO, 1.5 mg/kg) and G8 (temozolomide, 30 mg/kg) were 2907 ± 295 $mm^3$, 2180 ± 312 $mm^3$, 1064 ± 164 $mm^3$, 1213 ± 155 $mm^3$ and 1480 ± 136 $mm^3$, respectively. The tumor volume inhibition rates $TGI_{TV}$ were - 3.7%, 23.2%, 64.5%, 58.9% and 49.1%, respectively. The experimental results of the tumor weight were also confirmed. At the end of the experiment, the tumor tissue weights of the mice were as follows: 3.413 ± 0.253 g for group G3 (vehicle/normal saline); 3.557 ± 0.379 g for group G4 (d5PAO 0.5 mg/kg); 2.744 ± 0.459 g for group G5 (d5PAO 1.5 mg/kg); 1.413 ± 0.233 g for group G6 (temozolomide, 30 mg/kg+d5PAO, 0.5 mg/kg); 1.442 ± 0.251 g for group G7 (temozolomide, 30 mg/kg+d5PAO, 1.5 mg/kg); and 1.884 ± 0.217 g (FIG. 24) for group G8 (temozolomide, 30 mg/kg).

In comparison with the control group G3, temozolomide at 30 mg/kg used alone and temozolomide at 0.5 mg/kg and 1.5 mg/kg used in combination with d5PAO both had extremely significant inhibitory effects on the growth of subcutaneous transplanted tumor of A2058 ($P < 0.001$). In comparison with temozolomide used alone, temozolomide used in combination can further enhance the inhibitory effect.

## Example 11. Comparison of anti-tumor-cachexia effects of d5PAO and PAO

Cancer is the second leading cause of human death, and nearly one-sixth of global deaths are caused by cancer. Cancer is mainly treated by means of chemotherapy, radiation therapy, surgery, immunotherapy, gene therapy, hormone therapy and the like. At present, chemotherapy is one of the most effective means. But the main problem with chemotherapy is its side effects: when a chemotherapy drug kills cancer cells, it also kills fast-growing cells in the body, including cells in the blood, mouth, digestive system and hair follicles, which can cause digestive reactions, hair loss, bone marrow suppression and functional decline of other systems.

Cachexia, also known as dyscrasia, is manifested by extreme emaciation, weight loss, fat loss and reduced dissolution of skeletal muscle and cardiac muscle, which leads to progressive dysfunction and finally to systemic failure and other syndromes. Cachexia is mostly caused by severe chronic wasting diseases, including tumors, AIDS, severe trauma, post-surgery, malabsorption, severe sepsis and the like. Among them, cachexia accompanying tumor is the most common situation and also known as tumor cachexia. 31%-87% of patients with malignancy are accompanied by cachexia, and the direct cause of death of about 20% of tumor patients is malnutrition caused by cachexia, rather than the disease itself. Cachexia is highly correlated with pancreatic cancer, gastric cancer, lung cancer and liver cancer. Cachexia directly affects the cancer treatment effect, increases the incidence of complications, reduces the quality of life, shortens the survival time, prolongs the treatment time and increases the medical cost.

The causes of cachexia have not been fully elucidated, but recent studies have gradually revealed various pathogenic factors that are released from tumor cells or cells in the surrounding environment of tumor cells. Slowing or preventing the development of tumor cachexia can improve the quality of life for patients and prolong the survival time and is the main part of an anti-cancer treatment plan. Study on animal experimental models have shown that preventing weight loss during tumor development can prolong the survival rate. The main approach to treat cachexia of tumor patients is to inhibit weight loss and muscle loss by drugs. So far, most of the first-line anti-cachexia drugs have very limited effects on the prevention and treatment of tumor cachexia.

### 1. Effects of PAO and d5PAO on weights of healthy mice

22 (2 month old) and 20 (6 month old) male C57B/6 mice were taken to mouse rearing cages, 5-6 mice each cage. Among them, 12 (2 month old) and 10 (6 month old) mice received d5PAO in MCT at 2.1 mg/kg every day, the remaining 10 (2 month old) and 10 (6 month old) mice received PAO in MCT at 2.0 mg/kg every day. From the first day of administration, on the first day every 4 days, all mice were weighed before administration and the weights were recorded. In the next 4 days, on the basis of the weights, corresponding doses of PAO or d5PAO were given intragastrically. The number of living mice was recorded every 4 days.

The results are shown in FIG. 25. For 2-month-old mice in the d5PAO (2M-d5PAO) and PAO (2M-PAO) treatment groups, the average weights of mice were exactly the same on the first 24 days of intragastric administration and gradually increased. After day 24, the mice in the two groups all lost weight, wherein the weight loss of the 2M-PAO group was significantly faster than that of the 2M-d5PAO group. On day 44-48 of administration, one mouse was died in the group, whereas no death occurred in the 2M-d5PAO group. For 6-month-old mice in the d5PAO (6M-d5PAO) and PAO (6M-PAO) treatment groups, on day 48 of intragastric administration, the average weights of mice in the two groups were

slowly decreased in a fluctuant manner as a whole. However, from day 32 of administration, the weight loss of the 6M-PAO group was significantly faster than that of the 6M-d5PAO group, and the two groups both involved died mice, with 5 in the 6M-PAO group (50%) and two in the 6M-d5PAO group (20%). Therefore, the toxicity caused by intragastric administration of d5PAO was significantly lower than that of 6M-PAO.

**2. Effect of PAO on weight of tumor animal model**

2.1 Effect of PAO on weight of breast cancer mouse model

Injection of paclitaxel can cause weight loss in animals. On day 21 of administration, the animal weight was decreased from 101.4% ± 1.6% (the vehicle group, relative to the average weight on day 0) to 94.3% ± 2.6% (the 7 mg/kg paclitaxel group, relative to the average weight on day 0, P = 0.036*). The addition of PAO prevented the weight loss caused by paclitaxel (105.3% ± 2.4%, P = 0.187). It was worth noting that PAO itself can also increase the weights of tumor-bearing animals (106.1% ± 2.5%, P = 0.128) (FIG. 26).

2.2 Effect of PAO on weight of pancreatic cancer model

The establishment of a pancreatic cancer model was the same as above. On day 28 of administration, the average weight (104.3% ± 4.0%, relative to the average weight on day 0) of the gemcitabine (1.5 mg/kg)+paclitaxel (7 mg/kg) group was not significantly different from that of the vehicle group (99.0% ± 2.2%, relative to the average weight on day 0). However, the addition of PAO can increase the weight to 116.7% ± 3.9% (P = 0.002 *vs.* the vehicle group and the gemcitabine+paclitaxel group, FIG. 27).

2.3 Effect of PAO on weight of lymphoma animal model

The establishment of lymphoma model was the same as above. The addition of PAO failed to further inhibit the tumor growth, but can alleviate the weight loss caused by cyclophosphamide injection. On day 17 of administration, the animal weight was decreased from 117.4% ± 1.4% (the vehicle group G1, relative to the average weight on day 0) to 104.7% ± 1.6% (the 50 mg/kg cyclophosphamide group G2, relative to the average weight on day 0, P < 0.01*). PAO (0.6-0.9 mg/kg) used in combination with cyclophosphamide alleviated the weight loss caused by cyclophosphamide (G6, 111.1% ± 1.9%, P = 0.032 *vs.* the vehicle group, P = 0.05 *vs.* the cyclophosphamide group) (FIG. 28).

2.4 Effect of d5PAO on weight of melanoma animal model

In comparison with healthy mice, the weights of the tumor-bearing mice were all decreased. In comparison with the tumor-bearing mice in the vehicle group, temozolomide at 30 mg/kg used alone reduced the mouse weight (88% ± 1.5%, relative to day 0), and temozolomide used in combination with high-dose d5PAO (1.5 mg/kg) alleviated the weight loss (95% ± 2%, P = 0.01 *vs.* the temozolomide group, P = 0.038 *vs.* the vehicle group). d5PAO used alone had no significant effect on the weights of tumor-bearing animals (FIG. 29).

**Example 12. Inhibitory effects of PAO and d5PAO on HCoV229E (influenza coronavirus)**

**Test compound and control compound**

The control compound Remdesivir was provided by WuXi AppTec. The compound was prepared into 20 mM stock solutions with a DMSO solution. The test sample and the control compound were tested at 8 concentrations and diluted at 2-fold or 3-fold gradient, with replicate wells.

**Cell strains, virus strains and reagents**

MRC5 cells and HCoV229E strains were purchased from ATCC. The cells were cultured in EMEM (Sigma) culture solution supplemented with 10% fetal bovine serum (Hyclone), 1% double antibody (Hyclone), 1% L-glutamine (Gibco) and 1% nonessential amino acid (Gibco). The EMEM (Sigma) culture solution supplemented with 5% fetal bovine serum (Hyclone), 1% double antibody (Hyclone), 1% L-glutamine (Gibco) and 1% nonessential amino acid (Gibco) was used as an experimental culture solution. The main reagent used in this project is a cell viability detection kit CellTiter-Glo (Promega).

**Test method**

MRC5 cells were seeded into a 96-well assay plate, 20000 cells per well, and cultured overnight in a 37°C, 5% $CO_2$ incubator. The following day, the fold-diluted compound (8 concentration points, diluted at 2-fold or 3-fold gradient, replicate wells) was added, and then viruses were added to the cells at 200$TCID_{50}$ per well. A cell control (cells without compound treatment or virus infection), a virus control (cells infected with viruses, without compound treatment) and a culture solution control (only culture solution) were set. The final concentration of DMSO in the culture solution was 0.5%. The cells were cultured in an incubator for 3 days. The cytotoxicity experiment and the antiviral experiment were performed simultaneously under the same experimental conditions except without virus infection. The cell viability was detected by using a cell viability assay kit CellTiter Glo (Promega). The antiviral activity and cytotoxicity of the compound were expressed as inhibition rate (%) of the compound at different concentrations on cytopathic effects caused by viruses and viability (%) of MRC5 cells, respectively. The calculation formulas are as follows:

$$\text{Inhibition rate (\%)} = (\text{reading value of test well} - \text{average value of virus control})/(\text{average value of cell control} - \text{average value of virus control}) \times 100$$

$$\text{Cell viability (\%)} = (\text{reading value of test well} - \text{average value of culture solution control})/(\text{average value of cell control} - \text{average value of culture solution control}) \times 100$$

Nonlinear fitting analysis was performed on the inhibition rate and cell viability of the compound by using GraphPad Prism (version 5), and the half effective concentrations ($EC_{50}$) and half cytotoxic concentrations ($CC_{50}$) of the compound were calculated. The fitting formula is as follows: log(inhibitor) vs. response--Variable slope.

**Results**

The dose-response fitting curves of PAO and d5PAO drugs shown in FIG. 30. The control compound Remdesivir showed the expected antiviral activity and cytotoxicity.

The test results showed that the test compounds PAO and P100 had antiviral activity against HCoV229E, with the $EC_{50}$ values of 55.35 nM and 47.21 nM, respectively. The test compounds PAO and P100 had obvious toxicity to MRC5 cells, with the $CC_{50}$ values of 256.8 nM and 317.5 nM, respectively.

**Example 13. Comparison of anti-anxiety and anti-depression effects of low-dose d5PAO and PAO**

Test animals

30 male ICR mice (2 month old) were randomly divided into 3 groups after reared for 8 weeks in a clean-grade room under normal circadian rhythm and other conditions. The carrier, PAO and d5PAO groups were given chronic unpredictable multiple stimulations (CUMS), two cages a group, 5 mice a cage.

CUMS depression modeling

CUMS depression modeling: Mice were given various stimulations alternately every day every week, so that the duration of each stimulation given to mice and the pattern and duration of the next stimulation were unpredictable. The stimulation and schedule of the first week were shown in table 6. The stimulation pattern and time of each day in the table form a module, 7 modules in total. From the second week, the 7 modules were randomly picked for stimulation on Monday, the remaining 6 modules were randomly picked for stimulation on Tuesday, the remaining 5 modules were randomly picked for stimulation on Wednesday, and so on. If a test trial was scheduled on a certain day, appropriate adjustments were made to the stimulation for the previous two days and the day of the trial.

Table 6. CUMS stimulation schedule

| | Time | Stimulation schedule |
|---|---|---|
| Monday | 9: 00 | Clip the tail |

(continued)

| | Time | Stimulation schedule |
|---|---|---|
| | 12: 00 | Stop clipping the tail, place a camphor wood block, and tilt the mouse cage |
| Tuesday | 10: 00 | Remove the camphor wood block and place foreign objects (glass or plastic cups) |
| | 16: 00 | Take away food and the foreign objects, and keep lighting (switch on) |
| Wednesday | 9: 00 | 3 h of white noise |
| | 12: 00 | Ice pellets (200 mL)/cage |
| Thursday | 9: 00 | Change the cage, and give white noise for about 7 h |
| | 16: 00 | Supply food, tilt the mouse cage, and turn on a flash |
| Friday | 9: 00 | Turn off the flash and clip the tail for 3 h |
| | 12: 00 | Supply food, and give 6 h of white noise |
| Saturday | 16: 00 | Stop white noise and keep lighting at night (switch on) |
| | 10: 00 | Tilt the mouse cage |
| | 16: 00 | Take away water, stop tilting the mouse cage, and turn on the flash |
| Sunday | 9: 00 | Supply 45°C-50°C hot water (150 mL/cage) |

Administration method

After completing the first week of CUMS stimulation, mice in the PAO and d5PAO groups were intragastrically given solutions of compounds PAO and d5PAO every day at 0.05 mg/kg/day, respectively. The mice in the carrier group were intragastrically given MCT (MIGLYOL812N, supplied by IOI Oleo GmbH) with a volume corresponding to the mouse weight every day, wherein MCT was a carrier used to prepare solutions of compounds PAO and d5PAO. The concentrations of solutions of PAO and d5PAO in MCT were 0.005 mg/mL.

Novelty suppressed feeding (NSF) test

In the novelty suppressed feeding (NSF) test, there was a 25 cm × 25 cm × 20 cm (length × width × height) box that is made of opaque plexiglass. The box only has an open top-end and a small platform in the center, and a piece of mouse feed was placed on the platform. In the test, a mouse was put into the box from any corner of the box, with its head facing the corner of the box, and allowed to move freely for 5 min without interference, and the latency of the first feeding within 5 min was recorded. If the mouse did not eat within 5 min, the mouse was taken out, and the latency of the first feeding of the mouse was recorded as 300 s. The length (in seconds) of the latency of the first feeding is an index of anxiety of mice.

Depression behavior index: Sugar water preference test

In the sugar water preference test, two identical bottles weighed before were placed at a water supply place of a clean mouse cage, one bottle containing water and the other one containing an aqueous solution of 1% sucrose. In the test, a mouse was put to the end of the clean cage away from the bottle, with its head facing the opposite direction of the bottle. The mouse had free access to sugar water or water within 1 h without interference. After 1 h, the mouse was taken out, and the two bottles were carefully taken out and weighed to calculate the weight of sugar water and water that the mouse drinks, which were counted as $W_{water}$ and $W_{sugar}$ water respectively. The sugar water preference of mouse = $W_{sugar\ water}/(W_{water}$ and $W_{sugar\ water}) \times 100\%$.

Statistical methods

Statistical analysis was performed by using *SPSS* software, and data were expressed as mean ± standard error of the mean ($\bar{x} \pm$ s.e.m.). Since it is know that PAO has an anti-depression effect, the differences in the sugar water preference after administration between the PAO and d5PAO groups and the carrier group were tested for significance by means of One-tail unpaired *t*test, P < 0.05 marked as *, P < 0.01 marked as **.

Results

Three groups of mice were subjected to 3 weeks of CUMS and were intragastrically given carrier (MCT), PAO and d5PAO preparations for 15 days, respectively, followed by the novelty suppressed feeding test (NSF). The results

showed that only 2 of 10 mice in the vector group ate food, while the other 8 mice did not eat food within the limited 5 min, wherein the latency was recorded as 300 s, and the average anxiety index of this group was 282 ± 12 (sec). The average anxiety indexes of the PAO and d5PAO groups were 227 ± 29 (sec) and 181 ± 36 (sec), respectively, which were significantly lower than that of the carrier group. This indicated that low-dose (0.05 mg/kg/day) PAO and d5PAO also had obvious anti-anxiety effects, and d5PAO had more significant anti-anxiety effects than d5PAO (FIG. 31).

48 h after the three groups of mice completed the NSF test, a sugar water preference test was performed to detect the depression degrees of the three groups of mice. As shown in FIG. 27, the sugar water preference of the mice in the carrier, PAO and d5PAO groups were 66% ± 3.8%, 75% ± 4.3% and 78% ± 2.4%, respectively. The sugar water preference of the PAO and d5PAO groups was significantly higher than that in the carrier group, indicating that low-dose (0.05 mg/kg/day) PAO and d5PAO also had significant anti-depression effects, and d5PAO had a more significant anti-depression effect than PAO (FIG. 32). After the first sugar water preference test was completed, the three groups of mice were further given CUMS for a total of 38 days, and then the sugar water preference test was performed. The results showed that the sugar water preference of the mice in the carrier, PAO and d5PAO groups were 71% ± 3.5%, 77% ± 2.0% and 82% ± 2.9% respectively; The sugar water preference of the PAO and d5PAO groups was higher than that in the carrier group, but the difference between the PAO group and the carrier group was not significant, indicating that low-dose (0.05 mg/kg/day) PAO and d5PAO still had anti-depression effects, and d5PAO had a more significant and stable anti-depression effect than d5PAO (FIG. 32).

**Example 14. Study on therapeutic effects of d5PAO and PAO on NPC**

U18666A, an inhibitor of intracellular cholesterol transport, is often used to construct a cell model of Niemann-Pick disease type C (NPC).

1. Cell culture and compound treatment

SH-SY5Y cells were cultured in a complete medium containing high-glucose DMEM and 15% FBS in a 37°C, 5% $CO_2$ incubator. When cell confluence reached 70%, 10 μM U18666A (purchased from Absin (Shanghai) Biotechnology Co., Ltd., Catalog No. abs819512) was added, and different concentrations of d5PAO and PAO were added according to groups, followed by culturing for 24 h.

2. Filipin staining

1) the culture medium in the 24-well plate was discarded, 1 mL of PBS buffer was added and left to stand for 1 min, the liquid in the 24-well plate was discarded, and those steps were repeated twice;
2) 1 mL of 4% paraformaldehyde was added to each well, and fixation was carried out for 30 min at room temperature;
3) 4% paraformaldehyde in the 24-well plate was discarded, 1 mL of PBS buffer was added, the 24-well plate was gently shaken for 1 min, the liquid in the 24-well plate was discarded, and those steps were repeated twice;
4) 1 mL of 1.5 mg/mL glycine solution was added to each well, followed by incubation at room temperature for 10 min;
5) the liquid in the 24-well plate was discarded, and 1 mL of Filipin staining solution at a final concentration of 50 μg/mL (purchased from sigma Aldrich, Catalog No. SAE0087) was added, followed by incubation in the dark at room temperature for 1 h;
6) the liquid in the 24-well plate was discarded, 1 mL of PBS buffer was added, the 24-well plate was gently shaken for 1 min, the liquid in the 24-well plate was discarded, and those steps were repeated twice;
7) a glass slide was taken, 5 microlitres of mounting medium (containing DAPI) was added dropwise to the center of the glass slide, a cell slide was taken out and dried, the side on which cells grew was placed facing downward to cover the glass slide, which brought the cells in full contact with the mounting medium, followed by incubation in the dark at room temperature for 30 min.
8) observation was performed by using a laser confocal microscope.

Experimental results

SH-SY5Y cells were treated with 10 μM U18666A and co-incubated with different concentrations of d5PAO and PAO according to groups for 24 h, and observation was performed after Filipin staining. The immunofluorescent staining results showed that the fluorescent intensity of Filipin in the 10 μM U18666A treatment group was stronger than that in the control group (ctrl), indicating that 10 μM U18666A treatment led to an increase in the amount of cholesterol binding to Filipin, that is, cholesterol storage was caused. In comparison with the 10 μM U18666A treatment group, the

fluorescent intensity of Filipin in the 10 μM U18666A+35 nM d5PAO co-treatment group, 10 μM U18666A+70 nM d5PAO co-treatment group, 10 μM U18666A+35 nM PAO co-treatment group and 10 μM U18666A+70 nM PAO co-treatment group was decreased (FIG. 33). The above results indicated that certain concentrations of d5PAO and PAO can inhibit the cholesterol storage caused by U18666A.

**Example 15. Effects of PAO and *PI4Ka* knockdown on activating autophagy-lysosome pathway (ALP)**

Previous studies showed that ALP was blocked during the development of GD and other lysosomal storage diseases. SH-SY5Y cells were treated with CBE for 48 h and co-incubated with different concentrations of PAO or mTOR inhibitor rapamycin (RAPA) which were added as positive controls according to groups for 24 h. Western Blot or immunofluorescence assay was performed. The effects of PAO and other compounds on the autophagy-lysosomal pathway were studied by observing common markers of ALP. The common markers LC3B and p62 of ALP were detected, and the Western blot showed that: in an SH-SY5Y cell model constructed by CBE, PAO dose-dependently promoted the expression of LC3B proteins and inhibited the level of p62 proteins, indicating that PAO activated the ALP pathway and activated the autophagic flux (FIGs. 34A to 34C). The results were similar to the positive control 500 nM RAPA (FIGs. 34A to 34C). The immunofluorescence assay results were consistent with the Western blot results (FIG. 34D). In addition, the $H^+$-ATPase inhibitor Bafilomycin A1 (Baf-A1) is a commonly used ALP inhibitor. By blocking ALP signaling with Baf-A1, whether the protective effect of compounds such as PAO on SH-SY5Y cells constructed by CBE is associated with ALP can be further verified. SH-SY5Y cells were treated with CBE for 48 h and co-incubated with different concentrations of PAO or 50 nM Baf-A1 according to groups for 24 h. The cell viability was detected by MTT. The experimental results showed that in comparison with the control group (ctrl), 100 μM CBE significantly inhibited the viability of SH-SY5Y cells. In comparison with the 100 μM CBE treatment group, the 100 μM CBE+25 nM, 50 nM and 75 nM PAO co-treatment groups significantly increased the cell viability. The 50 nM Baf-A1 treatment decreased the protective effect of PAO in the corresponding groups, which resulted in that the cell viability of 50 nM Baf-A1 and 100 μM CBE+25 nM, 50 nM and 75 nM 1PAO co-treatment groups was not significantly different from that of the 100 μM CBE treatment group (FIG. 34E), indicating that Baf-A1 blocked the protective effect of PAO on CBE-treated SH-SY5Y cells by inhibiting ALP signaling. The above results confirmed that PAO activated ALP to activate autophagic flux and exerted a protective effect on the GD cell model by means of ALP. The SH-SY5Y cells treated with shRNA interfering lentiviral vectors were detected for the ALP pathway marker LC3B. The results showed that: in comparison with the sh-ctrl group, the level of LC3B proteins was significantly increased following PI4Ka knockdown (FIG. 35), and the PI4Ka knockdown in CBE-treated SH-SY5Y cells also promoted the expression of LC3B proteins, indicating that similar to results of the PI4Ka inhibitor PAO, the PI4Ka knockdown also activated the ALP pathway.

**Example 16. Resistance of PAO and d5PAO to chemical-factor-induced pneumonia and pulmonary fibrosis**

The lung and the upper respiratory tract are the tissues and organs that most frequently suffer from an inflammatory reaction that is caused by various etiologies, such as pathogens, chemical factors (including drugs), foreign bodies, physical damages, allergic reactions and autoimmune abnormalities. The inflammatory reaction caused is manifested by an increase in leukocytes (such as neutrophils, macrophages and lymphocytes) in local tissue or systemic blood and an increase in various inflammatory factors or cytokines. Pathogens include microorganisms and parasites. The microorganisms include bacteria, viruses, chlamydia, mycoplasma, spirochetes, fungi and the like. Lung inflammation sometimes may lead to fibrosis of lung tissues, which damages lung structure and function and especially damages the ventilation and diffusion of oxygen.

Bleomycin is a drug or chemical that can clearly cause pneumonia and pulmonary fibrosis. The interstitial pneumonia and pulmonary fibrosis, caused by bleomycin, in the lungs of animals is a common model of idiopathic pulmonary fibrosis (IPF). IPF is a fatal disease characterized by progressive and irreversible pulmonary fibrosis, which currently has no specific treatment method. The curative effects of existing treatment methods are not significant. Most patients died of progressive respiratory failure within 3-8 years following the onset of symptoms. Although we know little about the underlying mechanisms of IPF pathogenesis, the symbolic pathological features include: inflammatory reaction, hyperproliferation of fibroblasts, and abnormal deposition of an extracellular matrix.

The efficacy of PAO and d5PAO on bleomycin-induced pneumonitis and pulmonary fibrosis in mice is an example of the pharmaceutical application of PAO and d5PAO in inflammation reactions and tissue fibrosis.

Experimental methods:

Bleomycin induction

1. Preparation of bleomycin

Bleomycin was dissolved in normal saline, and the final concentration was adjusted according to the dose.

2. Induction method

On day 1, animals were anesthetized by inhalation of 2-5% isoflurane. Based on weights, the animals were intratracheally given bleomycin (2 mg/kg, the specific volume administered was calculated and recorded based on the animals' weights).

3. Administration

The day of receiving bleomycin induction was regarded as day 1 of the test. On day 3, animals were screened and grouped. On day 8 of the test, all animals were administered once a day until the end of the test. See table 7 for the specific administration regimen.

Table 7. Grouping and administration regimen

| Group | Test object | Animal | Administration route | Concentr ation mg/mL | Administration concentration | | Administration regimen |
|---|---|---|---|---|---|---|---|
| | | | | | mg/kg | mL/kg | |
| 1 | Normal group [a] | 6 | Orally taken | / | - | 10 | D8-D21, q.d |
| 2 | model group [a] | 10 | Orally taken | / | - | 10 | D8-D21, q.d |
| 3 | Nintedanib | 10 | Orally taken | 5 | 50 | 10 | D8-D21, q.d |
| 4 | PAO | 10 | Orally taken | 0.03 | 0.3 | 10 | D8-D21, q.d |
| 5 | d5PAO | 10 | Orally taken | 0.03 | 0.3 | 10 | D8-D21, q.d |
| a: MCT | | | | | | | |

4. Sample collection and analysis

On day 21, after tested for bronchial responsiveness post administration, all animals were anesthetized by inhalation of 2-5% isoflurane, and a minimum 0.5 mL of whole blood was collected from the orbit. The blood was anticoagulated with EDTA-2K, and plasma was centrifuged at 10000 rpm at 4°C for 10 min and stored at -80°C. After the blood samples were collected from the animals, the animals were anesthetized with Zoletil (intraperitoneal injection, 25-50 mg/kg, containing 1 mg/mL xylazine), a trachea cannula was inserted, and 0.5 mL of PBS (containing 1% FBS) was used for a first lavage of the lungs. Another 0.5 mL of PBS (containing 1% FBS) was taken for a second lavage of the lungs. 100 μL of suspension was taken to count the total number of cells in BALF. The BALF was centrifuged at 300 g for 5 min at 4°C, the bronchoalveolar lavage fluid (BALF) supernatant free of cell masses was collected. The concentrations of inflammatory factors (such as TNF-α, IL-1β, IL-6 and IFN-γ) and cytokines in BALF of the mice were detected by electrochemiluminescence immunoassay (a mouse Factor X detection kit from Merck MSD, V-PLEX Proinflammatory Panel1Mouse Kit, Catalog No. 15048D-X). Cell masses obtained after centrifugation were resuspended for smear preparation and stained with a Wright-Giemsa staining solution to differentiate eosinophils, neutrophils, macrophages and lymphocytes. The cells were counted under a light microscope.

After lavage, the animals were euthanized via cervical dislocation. The lung tissue was collected, the right lung was cryopreserved, and the total proteins were extracted after homogenization. The contents of collagen type I, hyaluronic acid and α-SMA were detected by commercial ELISA kits, and all the samples were loaded in replicate wells.

The left lung was collected and fixed in neutral formaldehyde. The left lung of each animal was cut into three sections and embedded in a paraffin block, so paraffin-embedded blocks and ultrathin sections with a thickness of 5 microns were prepared. After Masson's staining, histopathological evaluation was performed.

ELISA for detecting the levels of hyaluronic acid and collagen in plasma

The hyaluronic acid and collagen type I in plasma of the mice in each group were detected according to product instructions of a hyaluronic acid ELISA kit (Mouse Quantikine ELISA Kit, Biotechne, Catalog No. DHYAL0) and a collagen type I ELISA kit (Mouse TypeI I Colagen Detection ELISA Kit, Chondrex, Catalog No. 6012).

Test indexes

1. Weight

In the whole test period, animals were weighed once on the day of modeling, once on the day of grouping and three times a week after grouping, and the weights of the animals were recorded.

2. Pulmonary function test

bronchial hyperresponsiveness assay was performed on test mice by using an unconstrained whole-body plethysmograph (WBP) system to determine the lung function. First, mice received a PBS solution via aerosol inhalation and then methacholine (Mch) at 1.5625, 3.125, 6.25, 12.5, 25 and 50 mg/mL via continuous aerosol inhalation, the enhanced pause (Penh) at the corresponding concentration was determined, and stimulation was performed at each concentration for 90 s. A curve of change rate of Penh relative to a baseline-Mch concentration was plotted, and the area under the curve was calculated.

3. Pathological evaluation

The left lung of each animal was cut into three sections and embedded in a paraffin block, so paraffin-embedded blocks and ultrathin sections with a thickness of 5 microns were prepared. One section was prepared from each paraffin block, and Masson staining was performed for fibrosis evaluation. See table 8 for the scoring criteria.

**Table 8. Fibrosis scoring criteria**

| Scores | Standard |
|---|---|
| 1 | Normal |
| 3 | Minimal fibrotic thickening |
| 5 | Moderate fibrotic thickening |
| 7 | Fibrosis with lung tissue damage (thick bundle) |
| 8 | Large fiber area, showing "honeycomb shape" |

Detection of contents of collagen type I, hyaluronic acid, α-SMA and ten factors (such as TNF-α, IL-1β and IL-6)

The collected right lung tissue was homogenized and treated according to the instructions of commercial detection kits, and the contents of collagen type I, hyaluronic acid and α-SMA were detected. The expression of cytokines in BALF was measured by MSD.

The BALF supernatant without cell masses was collected, the expression of cytokines (the contents often factors such as TNF-α, IL-1β and IL-6) in BALF was measured by MSD, and the samples were loaded in replicate wells.

Test observation

The health states of the animals were observed near the cage twice a day and recorded in a log about animal rooms.

At the same time of weighing, the animal states were observed by members in the project team. Any abnormal appearance or behavior should be recorded in the PharmaLegacy biological test observation table in detail. For example, if the animal weights were significantly decreased (more than 15%) or other side effects (such as lethargy, immobility and mental malaise) occurred post administration, such events should be reported to the client immediately, and whether to change the dose or administration regimen should be discussed with the client.

Statistical analysis:

Test data were expressed as mean ± standard error of the mean (mean ± S.E.M). Data were analyzed by using SPSS or Graphpad Prism. The specific analysis methods used were described in the figure legends and in the notes below the tables. $P < 0.05$ indicated a statistic difference.

Experimental results:

Bronchial hyperresponsiveness assay was performed on test mice by using a WBP system. First, mice received a PBS solution via aerosol inhalation and then methacholine (Mch) at 1.5625, 3.125, 6.25, 12.5, 25 and 50 mg/mL via continuous aerosol inhalation, the enhanced pause (Penh) at the corresponding concentration was determined, and stimulation was performed at each concentration for 90 s. The percentage of Penh of each mouse at PBS and different concentrations of Mch relative to a baseline was calculated. The results are shown in table 9. A curve of change rate of Penh relative to a baseline-Mch concentration was plotted (FIG. 36), and the area under the curve was calculated (Table 10). These results indicated that PAO and d5PAO can well improve the damage of the lung function caused by pulmonary fibrosis, wherein the effect of d5PAO is stronger than that of PAO.

10 inflammatory factors (such as TNF-α, IL-1β and IL-6) and cytokines in BALF of animals of each group were detected by electrochemiluminescence immunoassay. As shown in table 11, both PAO and d5PAO had inhibitory effects on the up-regulation of IFN-γ, IL-1β, IL-2, IL-5, IL-6 and TNF-α, and especially had strong inhibitory effects on the up-regulation of IL-6.

The BALF cells of mice in each group were used to smear the slide, stained by a Wright-Giemsa staining solution to distinguish eosinophils, neutrophils, macrophages and lymphocytes and counted under a light microscope. The total count results of the 4 types of cells in each group are shown in table 9, and the respective count results of the 4 types of cells in each group are shown in FIG. 38. It was shown that PAO and d5PAO have different inhibitory effects on the total number of inflammatory cells caused by pulmonary fibrosis and on the increase of 4 types of inflammatory cells, and especially have significant inhibitory effects on the increase of neutrophils.

Pulmonary fibrosis was often accompanied by elevated levels of hyaluronic acid and collagen in the blood. Therefore, the levels of hyaluronic acid and collagen in plasma were tested by ELISA. Some results were shown in FIG. 39 and FIG. 40, indicating that PAO and d5PAO have inhibitory effects on the increase of plasma hyaluronic acid and collagen caused by pulmonary fibrosis, wherein the inhibitory effect on the increase of hyaluronic acid is obviously higher in comparison with the positive control drug (nintedanib).

Table 9. Percentage of enhanced pause (Penh) induced by methacholine relative to a baseline

| | Grouping of mice | Methacholine (mg/mL) | | | | | | |
|---|---|---|---|---|---|---|---|---|
| | | PBS | 1.563 | 3.125 | 6.25 | 12.5 | 25 | 50 |
| ***% basel ine Penh* Mean ± SEM** | Normal group (6) | 120 ± 10 | 132 ± 13 | 150 ± 14 | 177 ± 14 | 190 ± 16 | 289 ± 45 | 358 ± 29 |
| | Model group (10) | 140 ± 7 | 166 ± 13 | 173 ± 15 | 214 ± 24 | 213 ± 15 | 357 ± 38 | 643 ± 77### |
| | Nintedanib group (10) | 109 ± 8 | 121 ± 12 | 142 ± 16 | 219 ± 37 | 254 ± 39 | 352 ± 50 | 490 ± 115 |
| | PAO group (10) | 127 ± 8 | 150 ± 18 | 189 ± 26 | 203 ± 30 | 264 ± 46 | 285 ± 49 | 431 ± 126** |
| | d5PAO group (10) | 127 ± 19 | 152 ± 28 | 179 ± 29 | 208 ± 37 | 233 ± 41 | 312 ± 48 | 383 ± 95*** |
| Two-way ANOVA, ### < 0.001 *vs.* the normal group; ** < 0.01 and *** < 0.001 *vs.* the model group. | | | | | | | | |

Table 10. Area under the curve of change rate of Penh relative to a baseline-Mch concentration (AUC)

| Group | AUC of penh value | Inhibition rate |
|---|---|---|
| Normal group (6) | 13166 | -- |
| Model group (10) | 18522 | -- |

(continued)

| concentration (AUC) | | |
|---|---|---|
| Group | AUC of penh value | Inhibition rate |
| Nintedanib (10) | 16732 | 9.67% |
| PAO group (10) | 14921 | 19.45% |
| d5PAO (10) | 14549 | 21.45% |

Table 11. Contents (pg/mL) of inflammatory factors and cytokines in bronchoalveolar lavage fluid (BALF) of animals in each group

| Factors | Normal group (6) | Model group (10) | Positive drug group (10) | PAO (10) | d5PAO (9) |
|---|---|---|---|---|---|
| IFN-γ | 0.07 ± 0.01 | 0.08 ± 0.00 | 0.13 ± 0.05 | 0.07 ± 0.00 | 0.07 ± 0.01 |
| IL-10 | 1.87 ± 0.09 | 2.4 ± 0.23 | 2.17 ± 0.16 | 2.19 ± 0.16 | 2.37 ± 0.24 |
| IL-12p70 | 31.39 ± 2.27 | 23.31 ± 1.03 | 25.09 ± 1.12 | 23.30 ± 2.52 | 24.32 ± 2.41 |
| IL-1β | **0.36 ± 0.02** | **0.77 ± 0.14** | 1.24 ± 0.34 | 0.71 ± 0.12 | 0.73 ± 0.15 |
| IL-2 | 0.30 ± 0.02 | 0.31 ± 0.02 | 0.31 ± 0.04 | 0.30 ± 0.02 | 0.29 ± 0.04 |
| IL-4 | 0.28 ± 0.02 | 0.22 ± 0.01 | 0.24 ± 0.01 | 0.23 ± 0.02 | 0.23 ± 0.02 |
| IL-5 | **0.52 ± 0.07** | **1.64 ± 0.61** | 5.67 ± 3.38 | 1.57 ± 0.27 | 1.20 ± 0.27 |
| IL-6 | **9.69 ± 0.81** | **290.24 ± 251.49** | **371.98 ± 216.68** | **34.59 ± 6.64** | **82.83 ± 49.05** |
| KC/GRO | **3.13 ± 0.62** | **7.97 ± 0.99** | 9.01 ± 1.89 | 7.15 ± 1.00 | 11.45 ± 3.41 |
| TNF-α | **0.61 ± 0.06** | **5.51 ± 0.81** | 3.83 ± 0.61 | 5.16 ± 0.79 | 4.82 ± 0.92 |

It should be apparent to a person skilled in the art that although specific embodiments of the present invention are described herein for the purpose of illustration, various modifications can be made thereto without departing from the spirit and scope of the present invention. Therefore, the specific embodiments and examples of the present invention should not be construed as limiting the scope of the present invention. The present invention is limited only by the appended claims. All documents cited herein are incorporated herein by reference in their entirety.

## Claims

1. A compound of formula I or a pharmaceutically acceptable salt thereof,

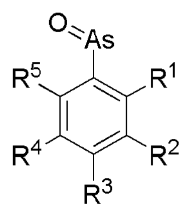

formula (I)

wherein $R^1$, $R^2$, $R^3$, $R^4$ and $R^5$ are independently selected from hydrogen, deuterium, halogen, methyl, mono-deuterated methyl, di-deuterated methyl or tri-deuterated methyl, and at least one of $R^1$, $R^2$, $R^3$, $R^4$ and $R^5$ is deuterium or deuterated methyl.

2. The compound or the pharmaceutically acceptable salt thereof according to claim 1, wherein $R^1$, $R^2$, $R^3$, $R^4$ and $R^5$ are independently selected from hydrogen or deuterium, and at least one, at least two, and preferably at least three, four or five of $R^1$, $R^2$, $R^3$, $R^4$ and $R^5$ are deuterium.

3. The compound or the pharmaceutically acceptable salt thereof according to claim 2, wherein the compound is selected from the group consisting of:

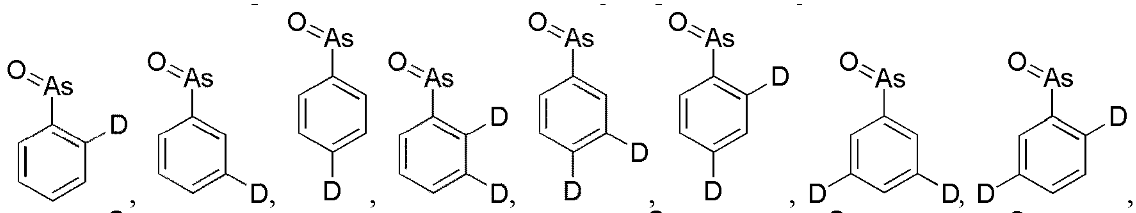

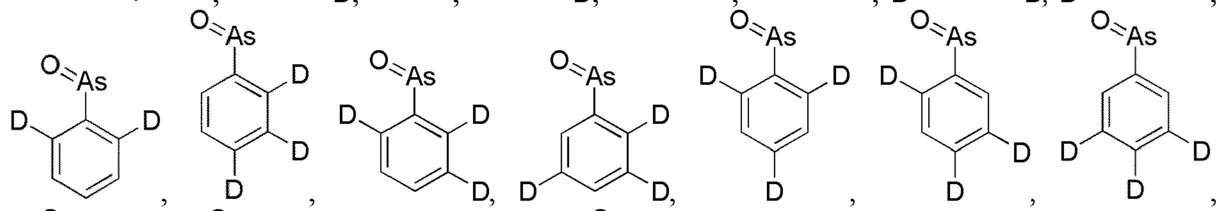

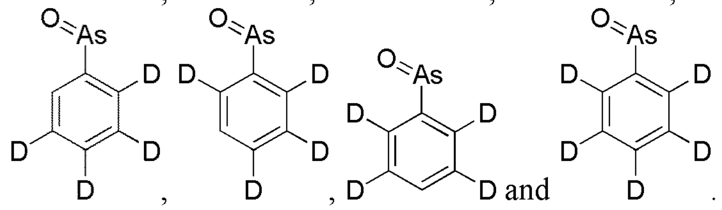

4. Use of the compound or the pharmaceutically acceptable salt thereof according to any one of claims 1 to 3 in the preparation of a drug for preventing or treating a disease or pathological reaction in a subject.

5. The use according to claim 4, wherein the disease is selected from a tumor, cachexia such as malignancy or cachexia caused by a chemotherapeutic drug for treating tumor, Alzheimer's disease, a disease related to intracellular protein misfolding, a lysosomal storage disease, an inflammatory reaction, tissue and organ fibrosis, an infectious disease caused by a virus and neurosis.

6. The use according to claim 4, wherein the subject is a human or a non-human mammal.

7. The use according to claim 5, wherein the tumor is selected from lymphoma, cervical cancer, liver cancer, breast cancer such as triple negative breast cancer, lung cancer such as non-small cell lung cancer or small cell lung cancer, colorectal cancer, gastric cancer, skin cancer such as melanoma, osteocarcinoma, osteosarcoma, myeloma, leukemia or ovarian cancer.

8. The use according to claim 5, wherein the disease related to intracellular protein misfolding is Parkinson's disease, Lewy body dementia, multiple system atrophy, inclusion body myositis, frontotemporal dementia, Huntington's disease, a polyglutamine disease, amyotrophic lateral sclerosis or a prion disease.

9. The use according to claim 5, wherein the lysosomal storage disease is a sphingolipid metabolism disorder such as Gaucher disease, Niemann-Pick disease type C, mucopolysaccharidosis, a glycogen storage disease, a glycoprotein storage disease, a lipid storage disease, post-translational modification deficiency, an integral membrane protein deficiency disorder, neuronal ceroid lipofuscinosis or a disorder of lysosome-related organelles.

10. The use according to claim 5, wherein the inflammatory reaction is manifested by an increase in inflammatory factors such as TNF-$\alpha$ or IL-6 in local tissue or systemic blood.

11. The use according to claim 5, wherein the tissue and organ fibrosis is selected from pulmonary fibrosis or hepatic fibrosis.

12. The use according to claim 5, wherein the virus comprises a coronavirus and a non-coronavirus, and preferably, the coronavirus is selected from avian infectious bronchitis virus, porcine epidemic diarrhea virus, porcine transmissible gastroenteritis virus, porcine hemagglutinating encephalomyelitis virus, porcine delta coronavirus, canine respiratory coronavirus, mouse hepatitis virus, feline coronavirus, human coronavirus, severe acute respiratory syndrome virus, Middle East respiratory syndrome virus or novel coronavirus, and the non-coronavirus is selected from hepatitis C virus or HIV.

**13.** The use according to claim 5, wherein the neurosis is selected from neurasthenia, anxiety, depression or mania.

**14.** The use according to any one of claims 5-13, further comprising administering a second agent to a subject in need thereof.

**15.** The use according to claim 14, wherein the disease is selected from a tumor, and the second agent is an agent for treating tumor, wherein the disease is selected from pulmonary fibrosis, and the second agent is an agent for treating pulmonary fibrosis, such as a vascular endothelial growth factor receptor tyrosine kinase inhibitor, preferably nintedanib.

**16.** The use according to claim 15, wherein the second agent is an agent for treating tumor, and the agent for treating tumor is selected from at least one of paclitaxel, gemcitabine, cyclophosphamide and temozolomide.

**17.** The use according to claim 14, wherein the compound or the pharmaceutically acceptable salt thereof according to any one of claims 1 to 3 is administered prior to, subsequent to or concurrently with administration of the second agent.

**18.** A pharmaceutical composition, comprising the compound or the pharmaceutically acceptable salt thereof according to any one of claims 1 to 3 and a pharmaceutically acceptable carrier.

**19.** The pharmaceutical composition according to claim 18, wherein the pharmaceutical composition further comprises a drug for treating tumor.

**20.** The pharmaceutical composition according to claim 19, wherein the drug for treating tumor is selected from at least one of paclitaxel, gemcitabine, cyclophosphamide and temozolomide.

**21.** A method for preparing the compound or the pharmaceutically acceptable salt thereof according to any one of claims 1 to 3, comprising the following steps:

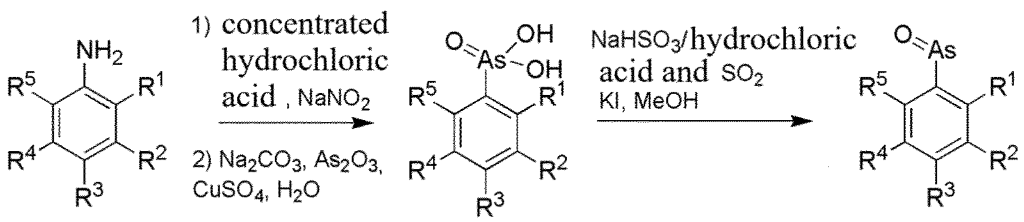


1) adding concentrated hydrochloric acid and an aqueous solution of sodium nitrite sequentially to an aqueous solution of aniline or a salt thereof that has a structure corresponding to formula (I) at 0-10°C, and maintaining the temperature below 5°C;
2) heating an aqueous solution of sodium carbonate, arsenic trioxide and copper sulfate to 90°C-100°C and then cooling the aqueous solution, adding the solution prepared in step 1) to the cooled aqueous solution, stirring and filtering the resulting mixture, adjusting a pH value of a filtrate by adding an acid, and separating the precipitated solid; and
3) stirring the precipitated solid, potassium iodide, sodium hydrogen sulfite or hydrochloric acid and sulfur dioxide in methanol until the reaction is complete, and then performing post-treatment to obtain the compound.

**22.** Use of oxophenylarsine and a derivative thereof in the preparation of a drug for preventing or treating tissue and organ fibrosis such as pulmonary fibrosis or hepatic fibrosis.

**23.** Use of oxophenylarsine and a derivative thereof in the preparation of a drug for preventing or treating an inflammatory reaction, wherein the inflammatory reaction is manifested by an increase in inflammatory factors such as TNF-$\alpha$ or IL-6 in local tissue or systemic blood.

**24.** Use of oxophenylarsine and a derivative thereof in the preparation of a drug for preventing or treating cachexia such as malignancy or cachexia caused by a chemotherapeutic drug for treating tumor.

**25.** Use of oxophenylarsine and a derivative thereof in the preparation of a drug for preventing or treating tumor.

**26.** The use according to any one of claims 22 to 25, wherein the oxophenylarsine and the derivative thereof have a structure of formula (II) or a pharmaceutically acceptable salt thereof,

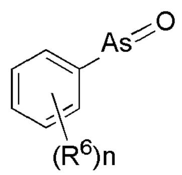

formula (II)

wherein (a) $R^6$ is each independently selected from H, halogen, nitro, cyano, hydroxyl, amino, carbamoyl, C1-6 alkylsulfuryl, C1-6 alkyl, C1-6 cycloalkyl, C2-6 alkynyl, C2-6 alkenyl, C1-6 alkoxy, C1-6 haloalkyl, C1-6 alkylene-NH2, C1-6 alkylene-NH-C(O)H, -As(O), -N=NH, N-(C1-6 alkyl)amino, N,N-(C1-6 alkyl)2amino, -NH-C(O)H, -NH-S(O)2H, -C(O)OH, - OC(O)H, -SH, -S(O)2H, -S(O)2-NH2 or heterocyclyl and is optionally substituted with $R^7$ or $R^8$, wherein the $R^7$ and $R^8$ are each independently selected from amino, C1-6 alkyl, C1-6 alkoxy, C1-6 haloalkyl, N-(C1-6 alkyl)amino, N-(6-12 membered aryl)amino, N,N-(C1-6 alkyl)2amino, C3-6 cycloalkyl, 6-12 membered aryl or 3-12 membered heterocyclyl and are optionally substituted with one or more of halogen, nitro, cyano, hydroxyl, amino, carbamoyl, -NH-C(O)-$R^{10}$, -C(O)O$R^9$, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-, wherein the $R^9$ is C1-6 alkyl and is optionally substituted with one or more of halogen, nitro, cyano, hydroxyl, amino, carbamoyl, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-, wherein the $R^{10}$ is selected from H, C1-6 alkyl, C2-6 alkynyl, C2-6 alkenyl, C1-6 alkoxy or C1-6 haloalkyl; and/or

(b) $R^6$ on two adjacent carbon atoms forms a 5-12 membered cycloalkyl, aryl or heterocyclyl, which is optionally substituted with one or more of halogen, nitro, cyano, hydroxyl, amino, carbamoyl, 6-12 membered aryl, C1-6 alkyl, C2-6 alkynyl, C2-6 alkenyl, C1-6 alkoxy, C1-6 haloalkyl, 3-6 membered heterocyclyl, C3-6 cycloalkyl or Bn-O-,
wherein n is an integer from 0 to 5.

**27.** The use according to claim 26, wherein n is an integer from 0 to 2, and the $R^6$ is each independently selected from H, halogen, nitro, cyano, hydroxyl, amino, carbamoyl, C1-6 alkylsulfuryl, C1-6 alkyl, C1-6 cycloalkyl, C1-6 alkoxy, C1-6 haloalkyl, -As(O), N-(C1-6 alkyl)amino, N,N-(C1-6 alkyl)2amino, -NH-C(O)H or -NH-S(O)2H and is optionally substituted with the $R^7$ or $R^8$.

**28.** The use according to claim 26, wherein n is an integer from 0 to 2, and the R1 is each independently selected from H, halogen, nitro, cyano, hydroxyl, amino, C1-6 alkylsulfuryl, C1-6 alkyl, C1-6 cycloalkyl, C1-6 alkoxy, C1-6 haloalkyl, -As(O), -NH-C(O)H or -NH-S(O)2H and is optionally substituted with the $R^7$ or $R^8$.

**29.** The use according to claim 26, wherein n is 1 or 2, and the $R^6$ is each independently selected from H, halogen, amino, C1-6 alkylsulfuryl, C1-6 cycloalkyl, C1-6 alkoxy, C1-6 haloalkyl, -NH-C(O)$R^7$ or -NH-S(O)2$R^8$, wherein the $R^7$ is C1-6 alkyl which is optionally substituted with 6-12 membered aryl, and the $R^8$ is 6-12 membered aryl which is optionally substituted with one of halogen, C1-6 alkoxy or C1-6 haloalkyl.

**30.** The use according to claim 29, wherein the $R^6$ is located at an ortho position and/or a para position to a -As(O) group.

**31.** The use according to claim 26, wherein n is 0.

**32.** The use according to claim 26, wherein the compound is selected from the group consisting of:

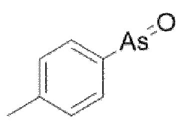 , 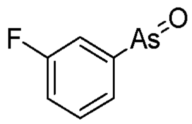 , 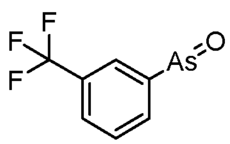 , 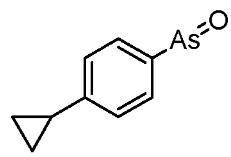 ,

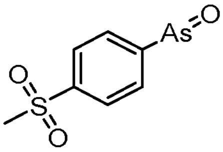,
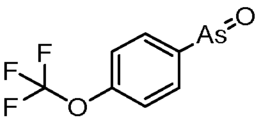,
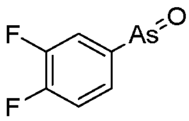,
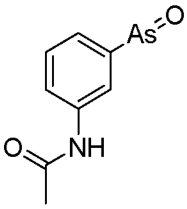,
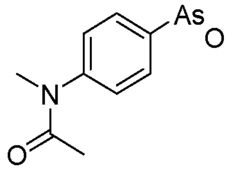,

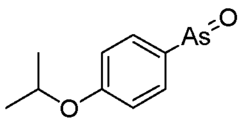,
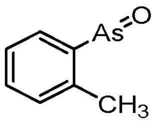,
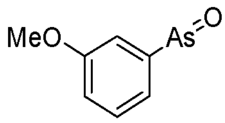,
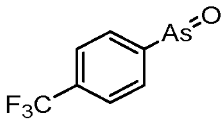,
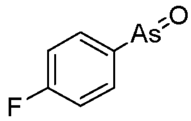,

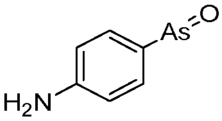,
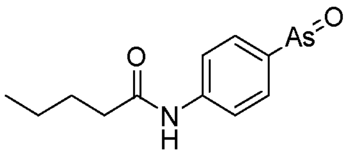,
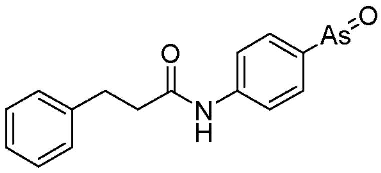,

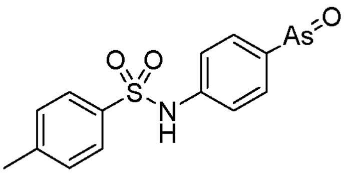,
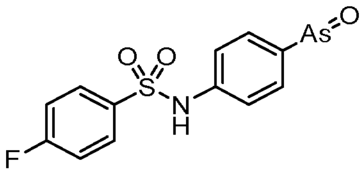,
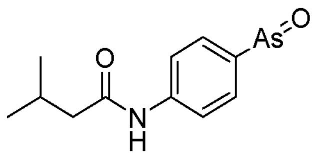,

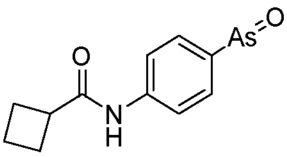,
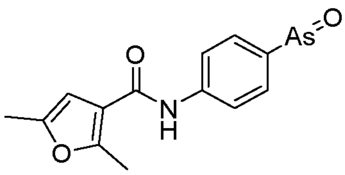,
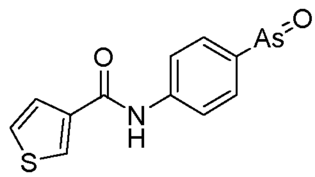,

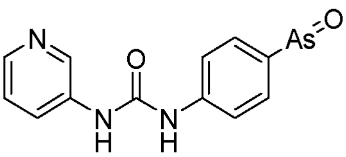,
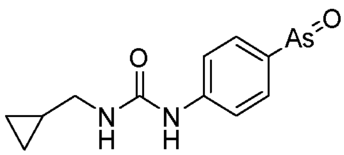,
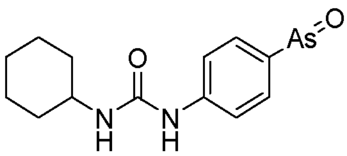,

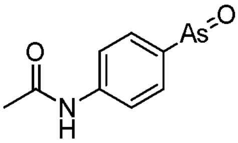,
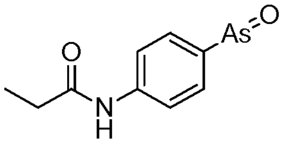,
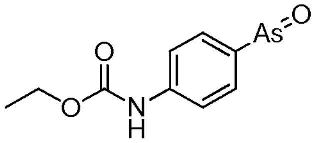,

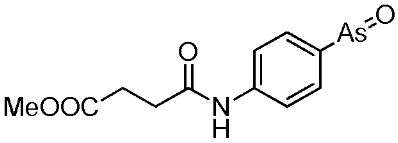,
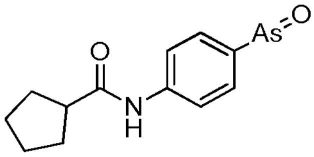,
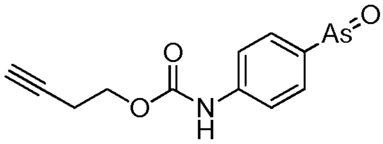,

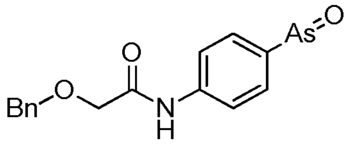,
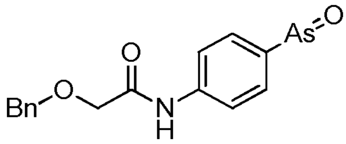,
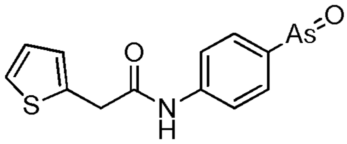,

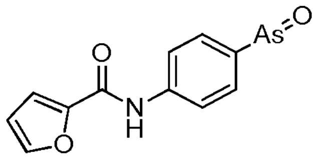,
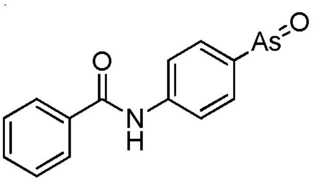,
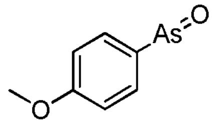,
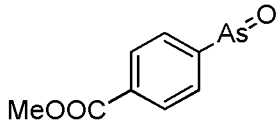,

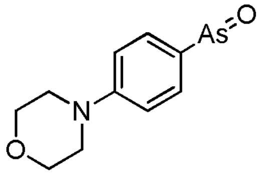,
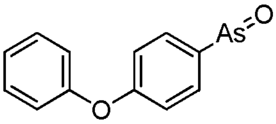,
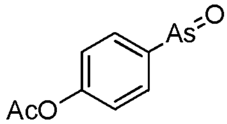,
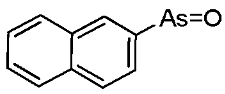,

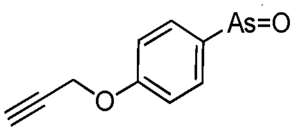,
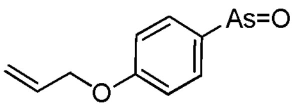,
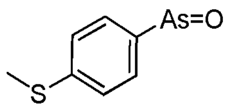,
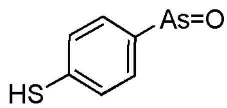,

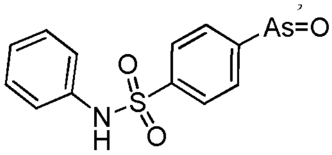,
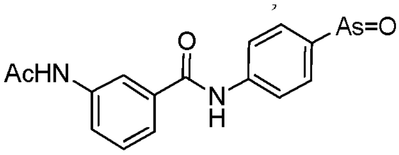,
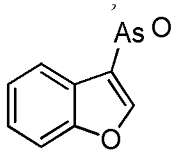,
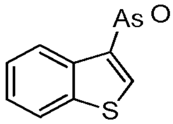,

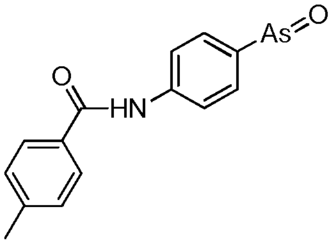,
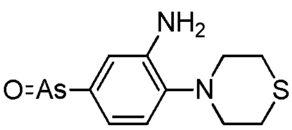,
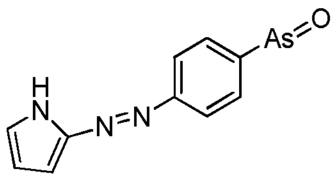,

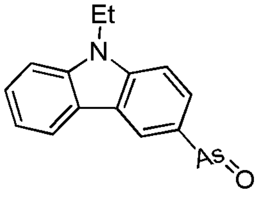,
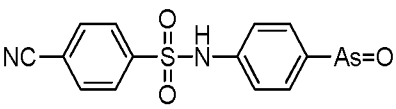,
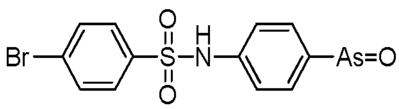,

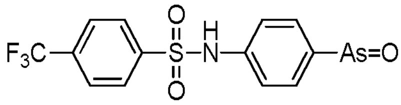,

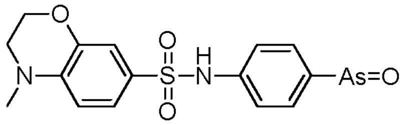,

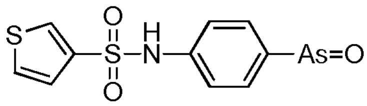,

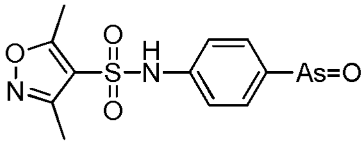,

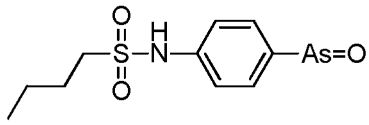,

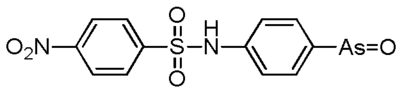,

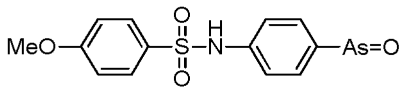,

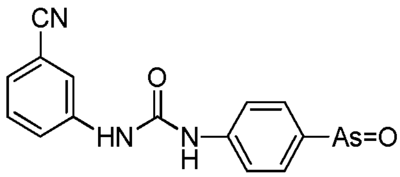,

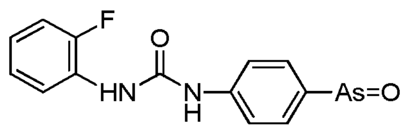,

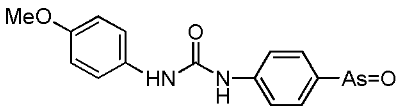,

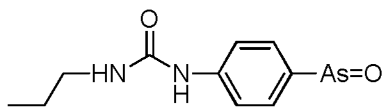,

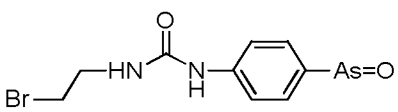,

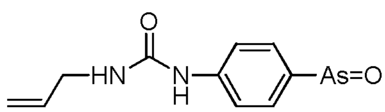,

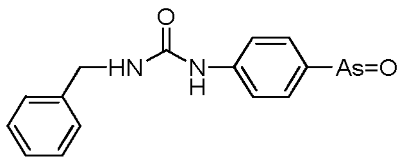,

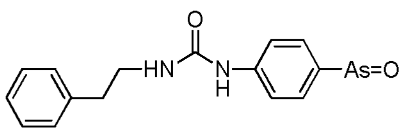,

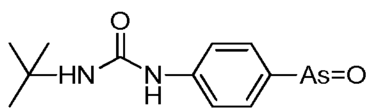,

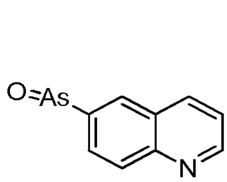,

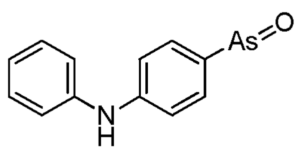,

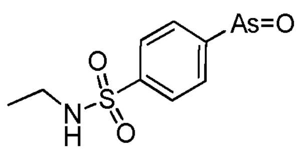,

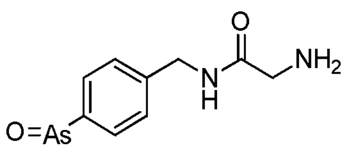,

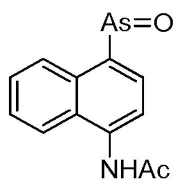,

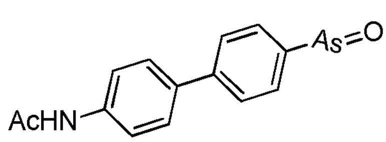,

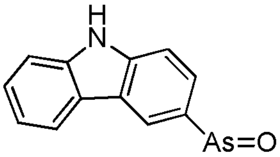, 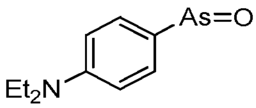, 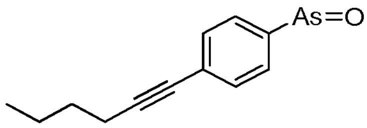,

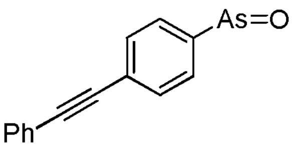, 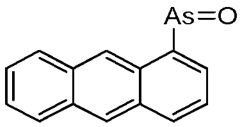, 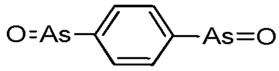, 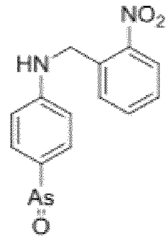,

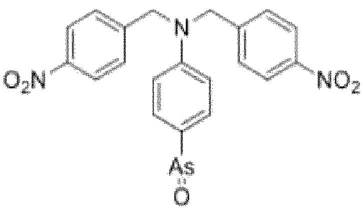, 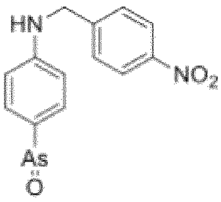, 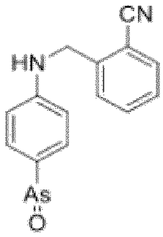, 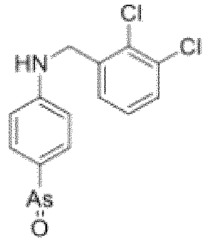,

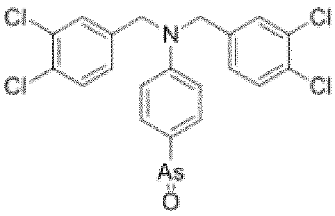 and 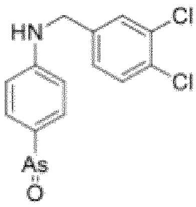.

**33.** The use according to any one of claims 22 to 25, wherein the object is a human or a mammal.

**34.** The use according to claim 25, wherein the tumor is selected from lymphoma, cervical cancer, liver cancer, breast cancer such as triple negative breast cancer, lung cancer such as non-small cell lung cancer or small cell lung cancer, colorectal cancer, gastric cancer, skin cancer such as melanoma, osteocarcinoma, osteosarcoma, myeloma, leukemia or ovarian cancer.

**35.** The use according to claim 25, further comprising administering a second agent to a subject in need thereof, wherein the second agent is preferably an agent for treating tumor.

**36.** The use according to claim 35, wherein the second agent is an agent for treating tumor.

**37.** The use according to claim 35, wherein the compound is administered prior to, subsequent to or concurrently with administration of the second agent.

**38.** The pharmaceutical composition according to claim 37, wherein the agent for treating tumor is selected from at least one of paclitaxel, gemcitabine, cyclophosphamide and temozolomide.

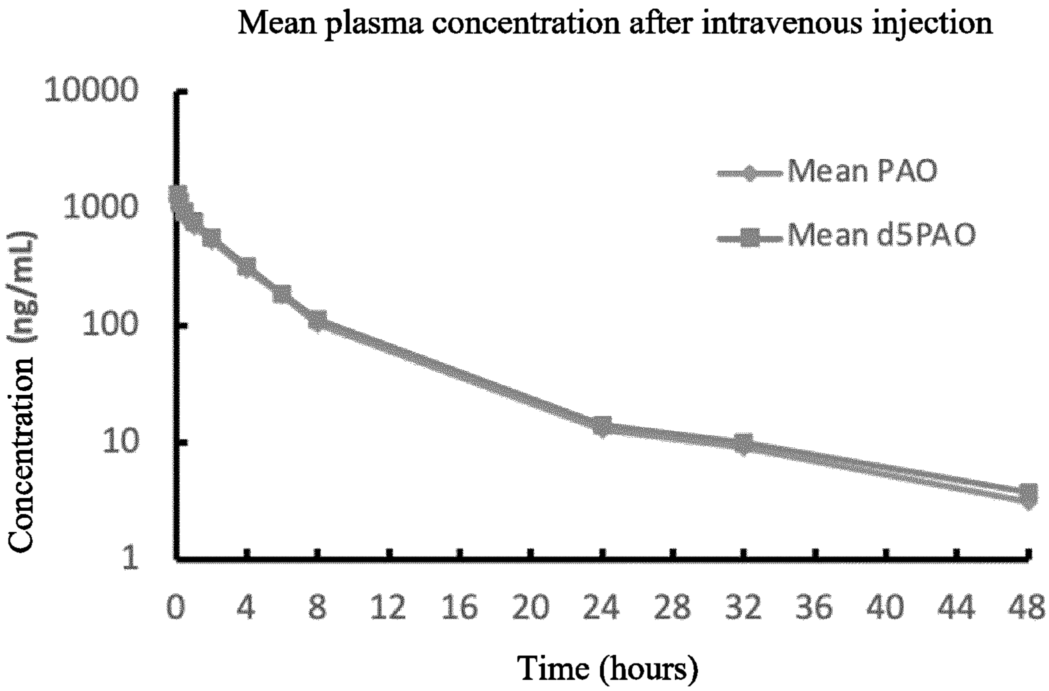
Mean plasma concentration after intravenous injection
10000
1000
100
10
1
Concentration (ng/mL)
Mean PAO
Mean d5PAO
0 4 8 12 16 20 24 28 32 36 40 44 48
Time (hours)

*FIG. 1*

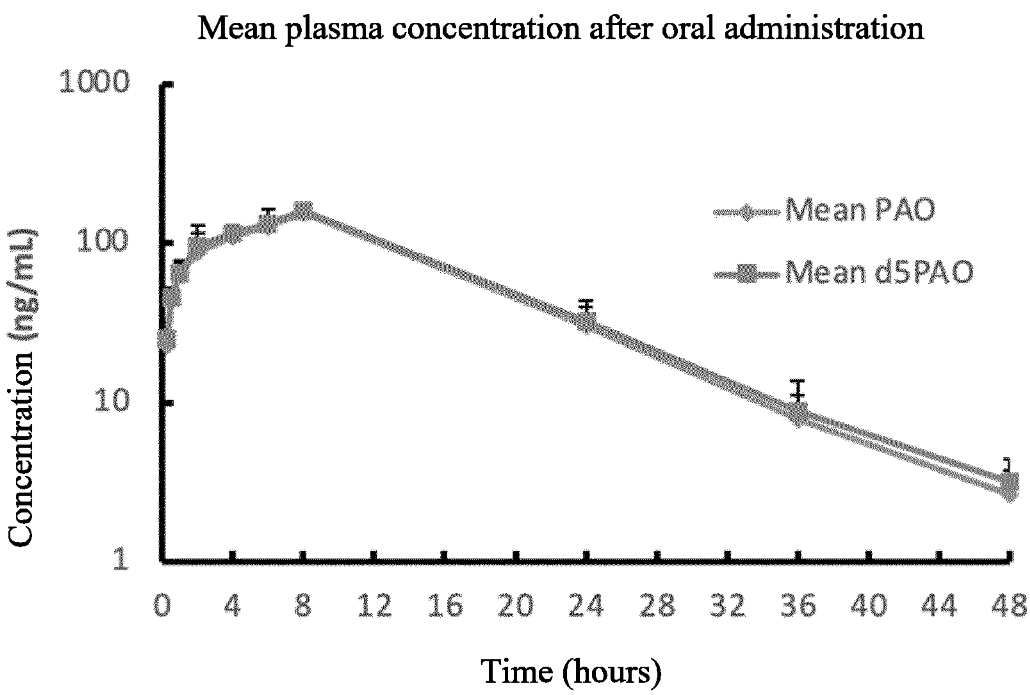
Mean plasma concentration after oral administration
1000
100
10
1
Concentration (ng/mL)
Mean PAO
Mean d5PAO
0 4 8 12 16 20 24 28 32 36 40 44 48
Time (hours)

*FIG. 2*

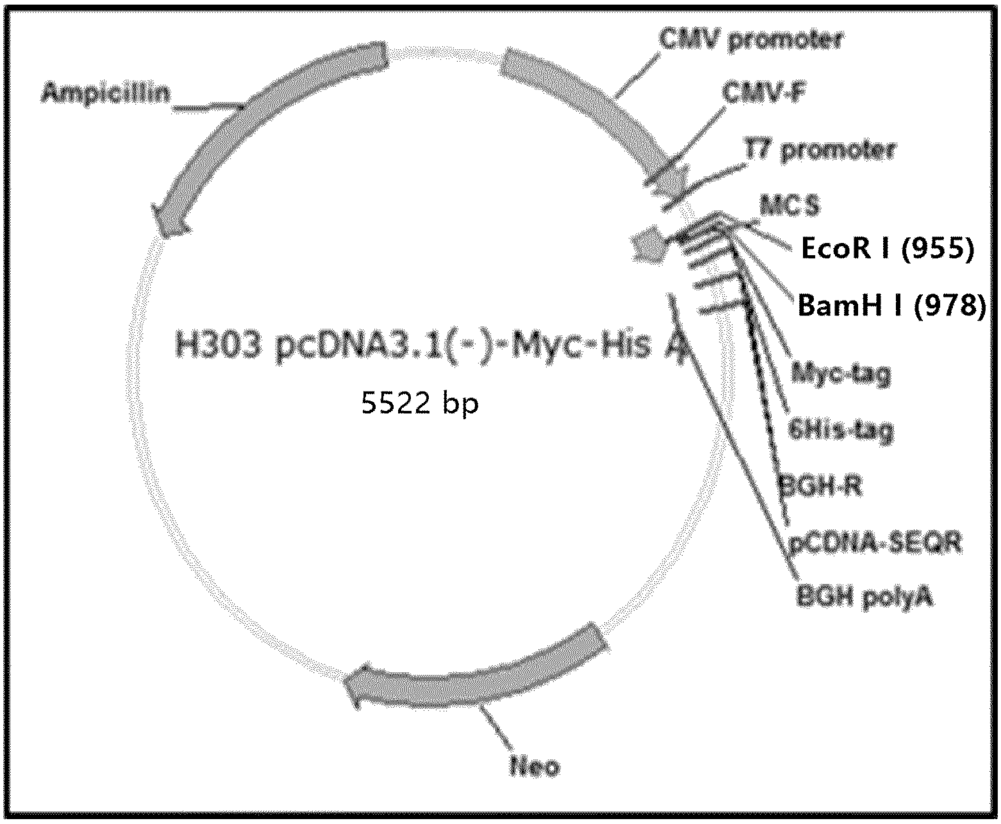
Ampicillin
CMV promoter
CMV-F
T7 promoter
MCS
EcoR I (955)
BamH I (978)
H303 pcDNA3.1(-)-Myc-His A
5522 bp
Myc-tag
6His-tag
BGH-R
pCDNA-SEQR
BGH polyA
Neo

*FIG. 3*

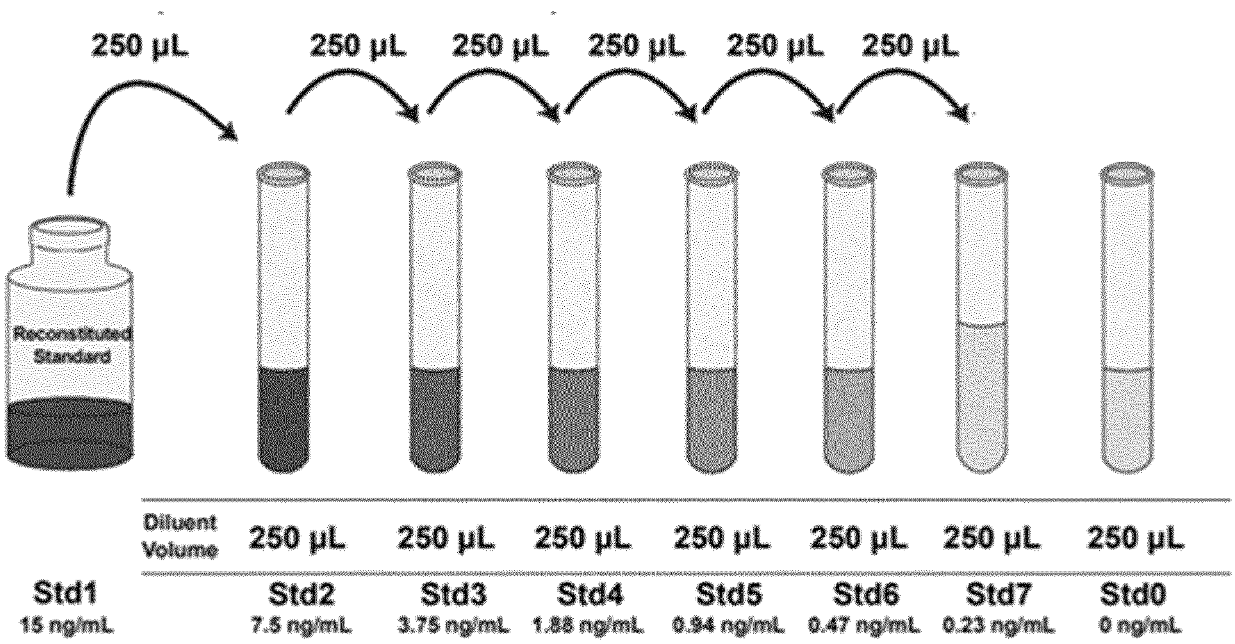
250 μL
250 μL
250 μL
250 μL
250 μL
250 μL
Reconstituted Standard
Diluent Volume
250 μL
250 μL
250 μL
250 μL
250 μL
250 μL
250 μL
Std1
15 ng/mL
Std2
7.5 ng/mL
Std3
3.75 ng/mL
Std4
1.88 ng/mL
Std5
0.94 ng/mL
Std6
0.47 ng/mL
Std7
0.23 ng/mL
Std0
0 ng/mL

*FIG. 4*

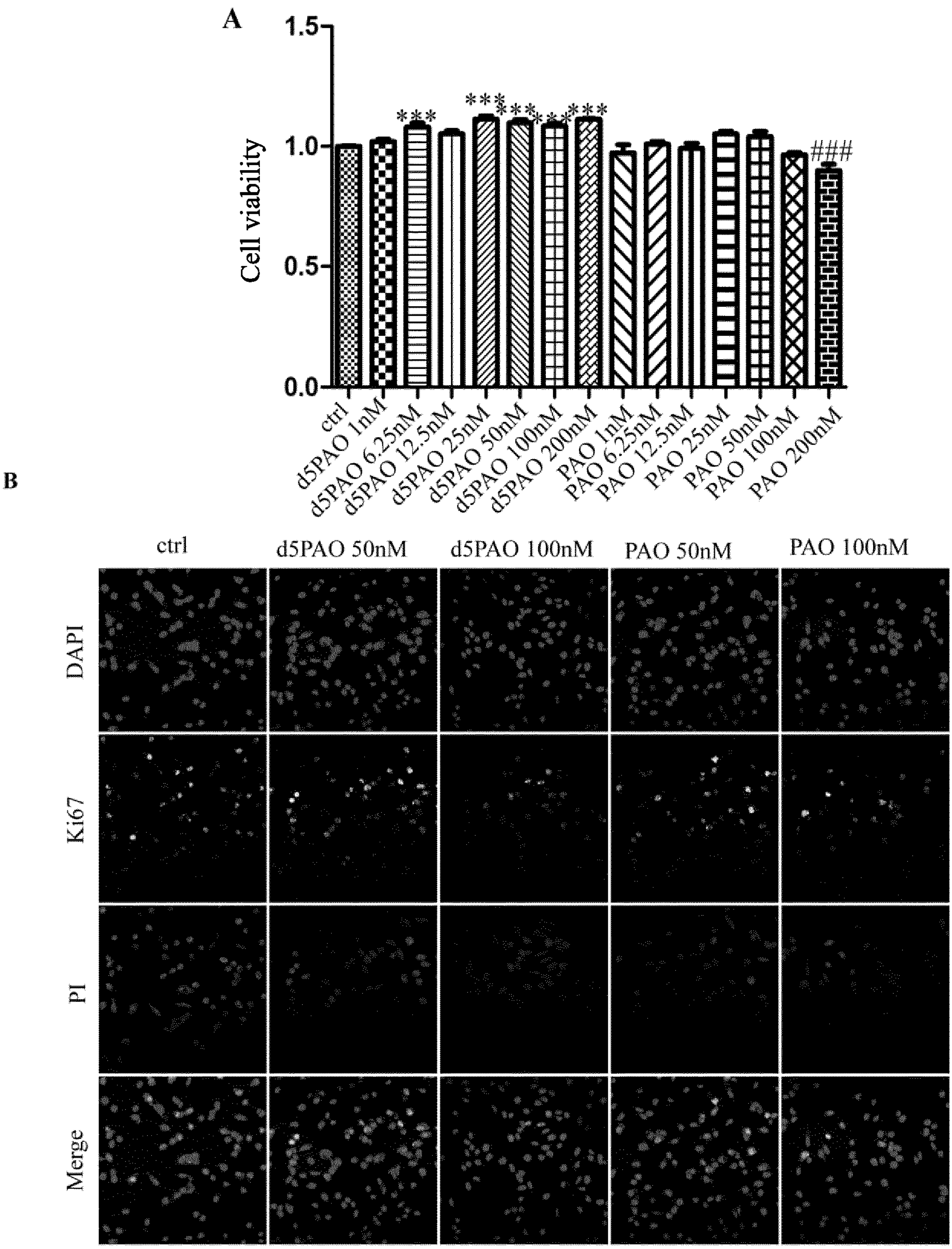
A
Cell viability
1.5
1.0
0.5
0.0
***
***
***
***
***
###
ctrl
d5PAO 1nM
d5PAO 6.25nM
d5PAO 12.5nM
d5PAO 25nM
d5PAO 50nM
d5PAO 100nM
d5PAO 200nM
PAO 1nM
PAO 6.25nM
PAO 12.5nM
PAO 25nM
PAO 50nM
PAO 100nM
PAO 200nM
B
ctrl
d5PAO 50nM
d5PAO 100nM
PAO 50nM
PAO 100nM
DAPI
Ki67
PI
Merge

*FIG. 5*

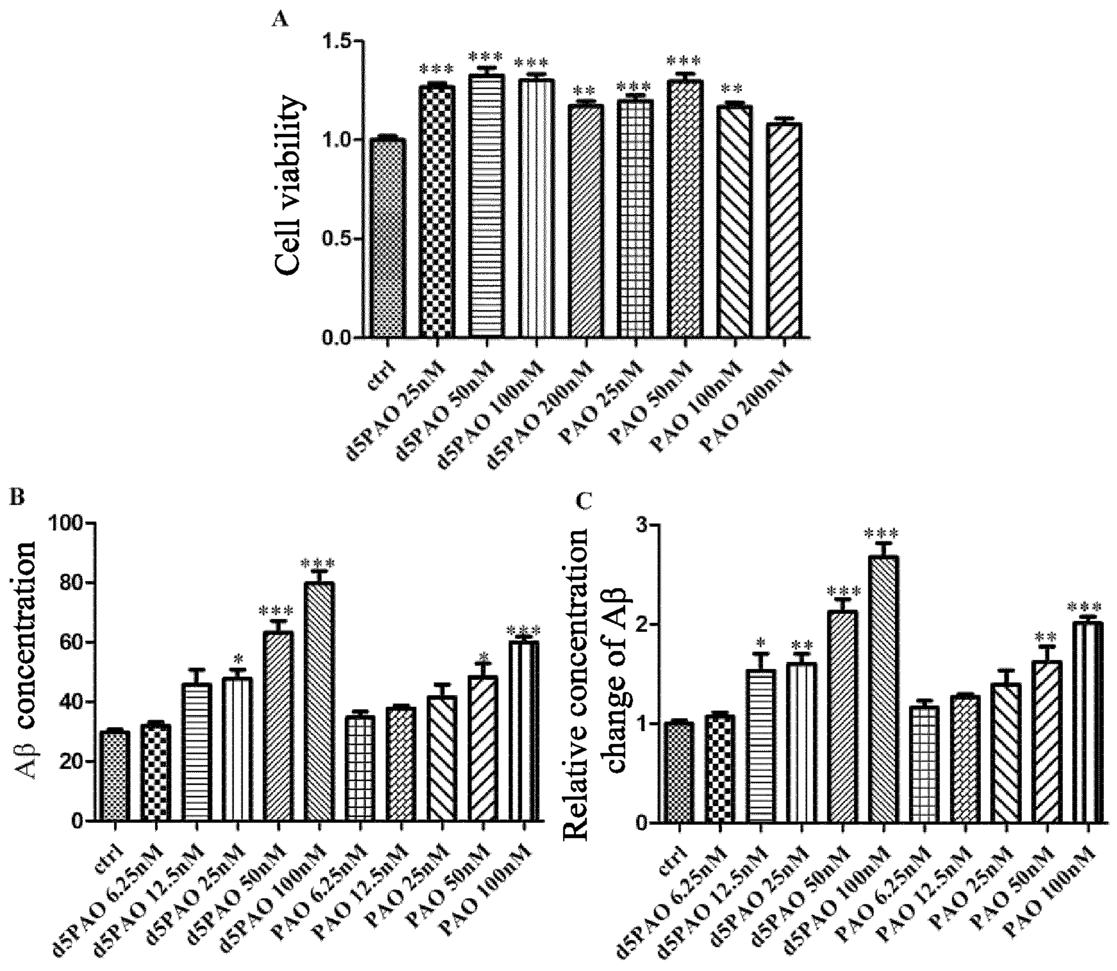
A
Cell viability
1.5
1.0
0.5
0.0
ctrl
d5PAO 25nM
d5PAO 50nM
d5PAO 100nM
d5PAO 200nM
PAO 25nM
PAO 50nM
PAO 100nM
PAO 200nM
B
Aβ concentration
100
80
60
40
20
0
ctrl
d5PAO 6.25nM
d5PAO 12.5nM
d5PAO 25nM
d5PAO 50nM
d5PAO 100nM
PAO 6.25nM
PAO 12.5nM
PAO 25nM
PAO 50nM
PAO 100nM
C
Relative concentration change of Aβ
3
2
1
0
ctrl
d5PAO 6.25nM
d5PAO 12.5nM
d5PAO 25nM
d5PAO 50nM
d5PAO 100nM
PAO 6.25nM
PAO 12.5nM
PAO 25nM
PAO 50nM
PAO 100nM

*FIG. 6*

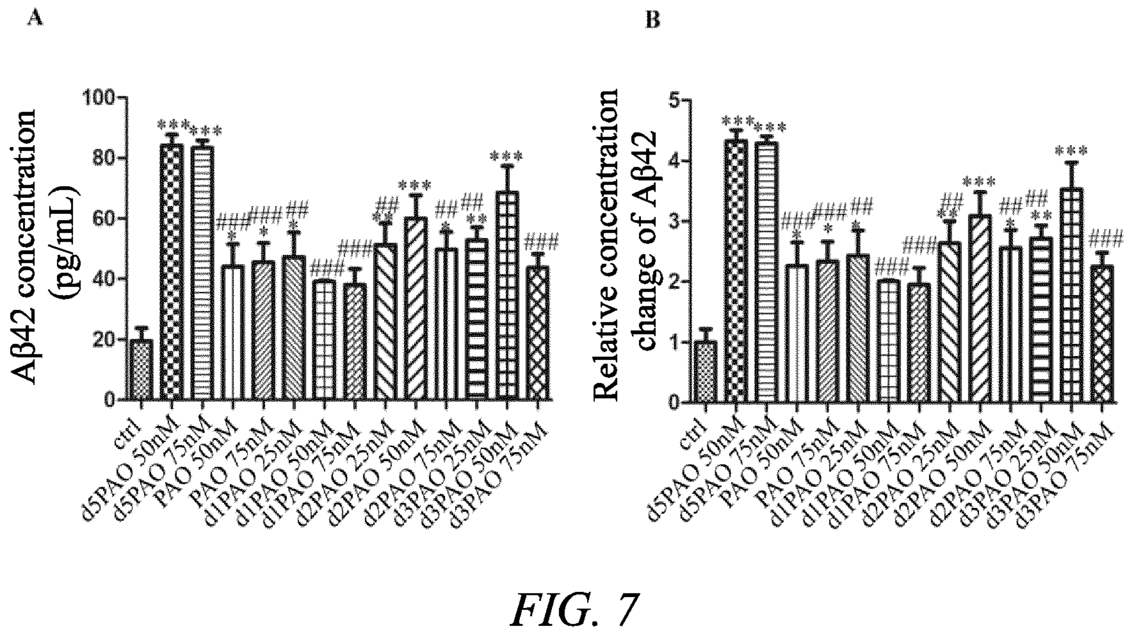
A
Aβ42 concentration (pg/mL)
100
80
60
40
20
0
ctrl
d5PAO 50nM
d5PAO 75nM
PAO 50nM
PAO 75nM
d1PAO 25nM
d1PAO 50nM
d1PAO 75nM
d2PAO 25nM
d2PAO 50nM
d2PAO 75nM
d3PAO 25nM
d3PAO 50nM
d3PAO 75nM
B
Relative concentration change of Aβ42
5
4
3
2
1
0
ctrl
d5PAO 50nM
d5PAO 75nM
PAO 50nM
PAO 75nM
d1PAO 25nM
d1PAO 50nM
d1PAO 75nM
d2PAO 25nM
d2PAO 50nM
d2PAO 75nM
d3PAO 25nM
d3PAO 50nM
d3PAO 75nM

*FIG. 7*

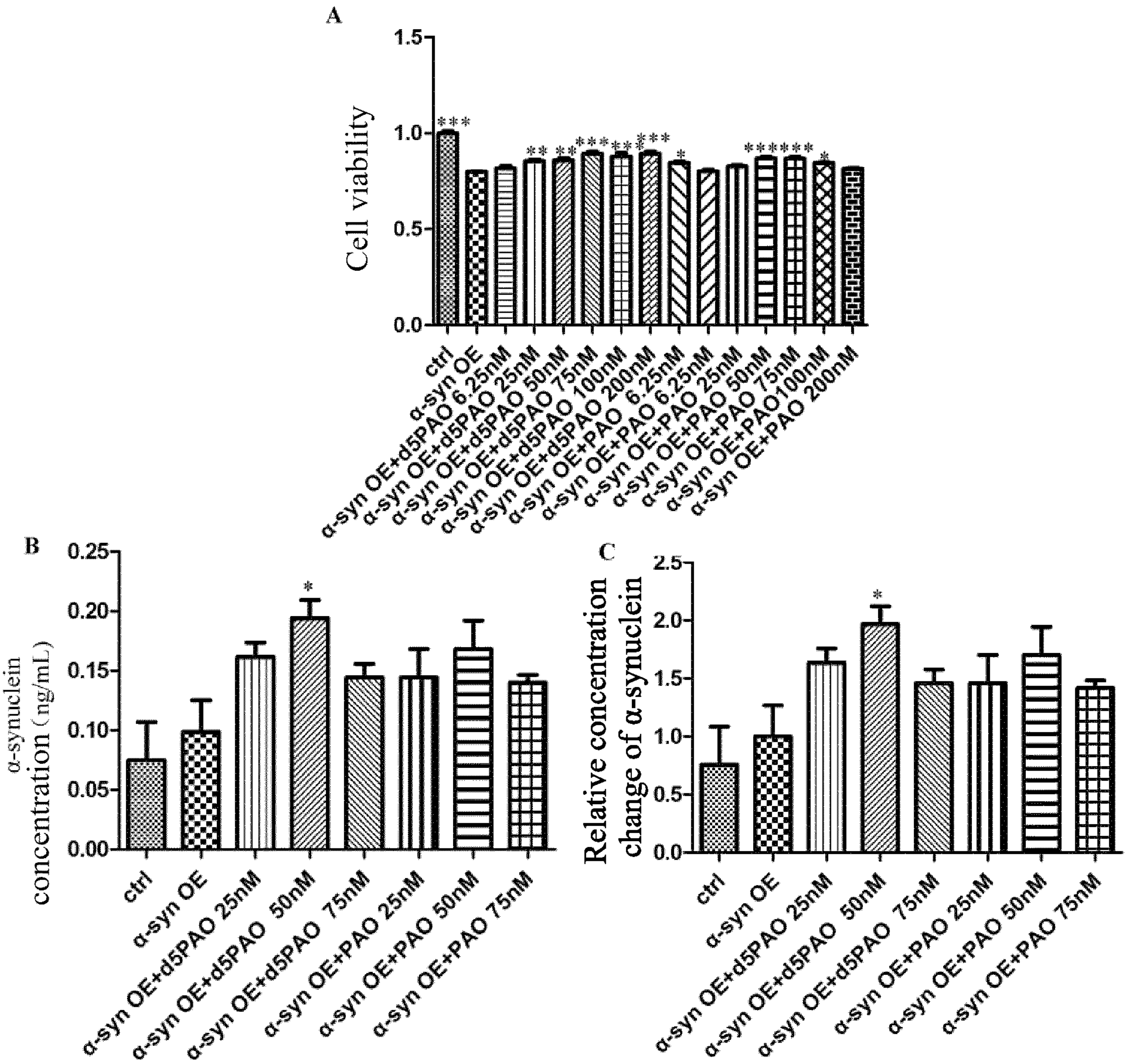
A
Cell viability
1.5
1.0
0.5
0.0
ctrl
α-syn OE
α-syn OE+d5PAO 6.25nM
α-syn OE+d5PAO 25nM
α-syn OE+d5PAO 50nM
α-syn OE+d5PAO 75nM
α-syn OE+d5PAO 100nM
α-syn OE+d5PAO 200nM
α-syn OE+PAO 6.25nM
α-syn OE+PAO 25nM
α-syn OE+PAO 50nM
α-syn OE+PAO 75nM
α-syn OE+PAO100nM
α-syn OE+PAO 200nM
B
α-synuclein concentration（ng/mL）
0.25
0.20
0.15
0.10
0.05
0.00
ctrl
α-syn OE
α-syn OE+d5PAO 25nM
α-syn OE+d5PAO 50nM
α-syn OE+d5PAO 75nM
α-syn OE+PAO 25nM
α-syn OE+PAO 50nM
α-syn OE+PAO 75nM
C
Relative concentration change of α-synuclein
2.5
2.0
1.5
1.0
0.5
0.0
ctrl
α-syn OE
α-syn OE+d5PAO 25nM
α-syn OE+d5PAO 50nM
α-syn OE+d5PAO 75nM
α-syn OE+PAO 25nM
α-syn OE+PAO 50nM
α-syn OE+PAO 75nM

*FIG. 8*

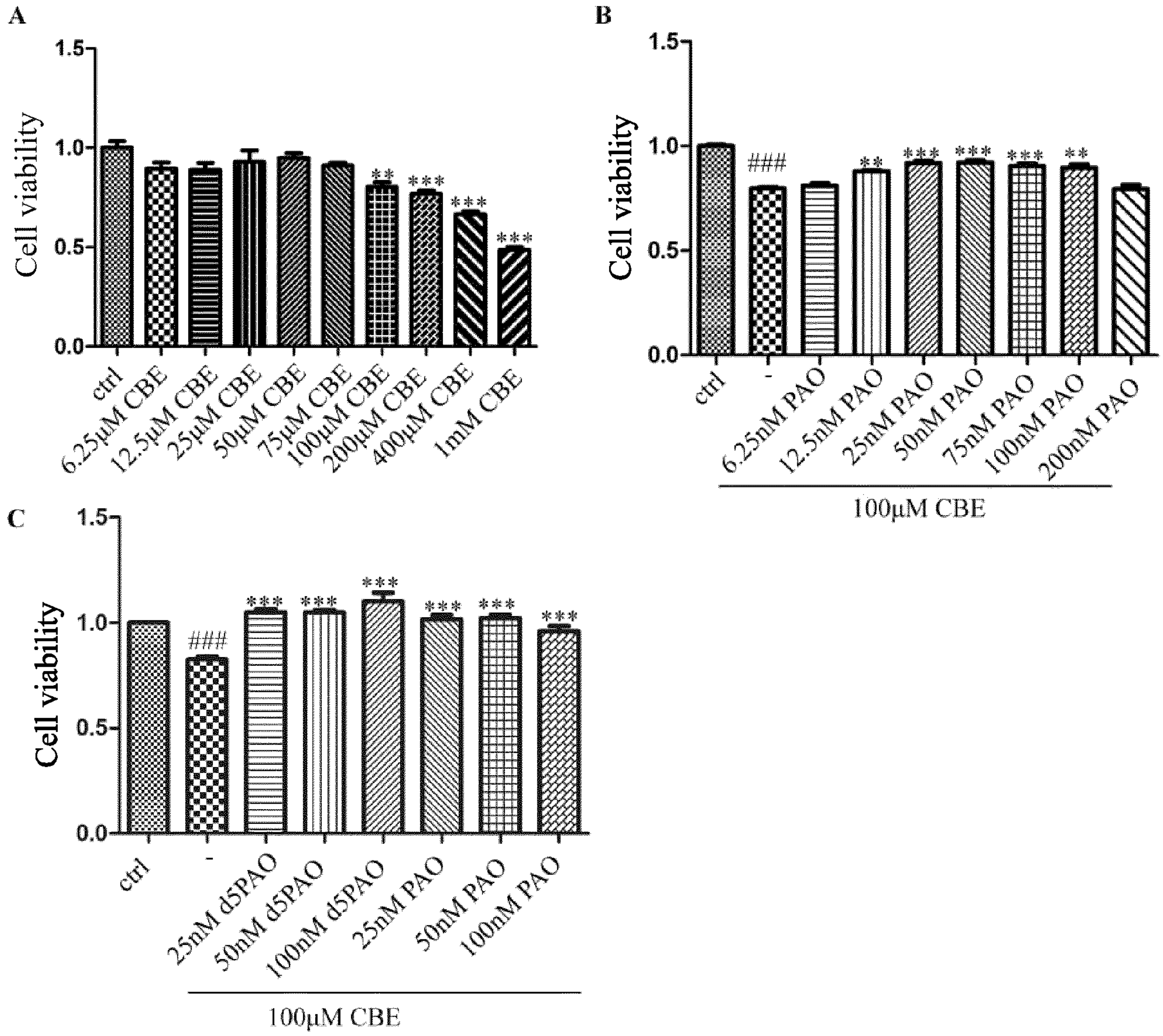
A
Cell viability
1.5
1.0
0.5
0.0
ctrl
6.25μM CBE
12.5μM CBE
25μM CBE
50μM CBE
75μM CBE
100μM CBE
200μM CBE
400μM CBE
1mM CBE
**
***
***
***
B
Cell viability
ctrl
-
6.25nM PAO
12.5nM PAO
25nM PAO
50nM PAO
75nM PAO
100nM PAO
200nM PAO
100μM CBE
###
C
Cell viability
ctrl
-
25nM d5PAO
50nM d5PAO
100nM d5PAO
25nM PAO
50nM PAO
100nM PAO
100μM CBE

*FIG. 9*

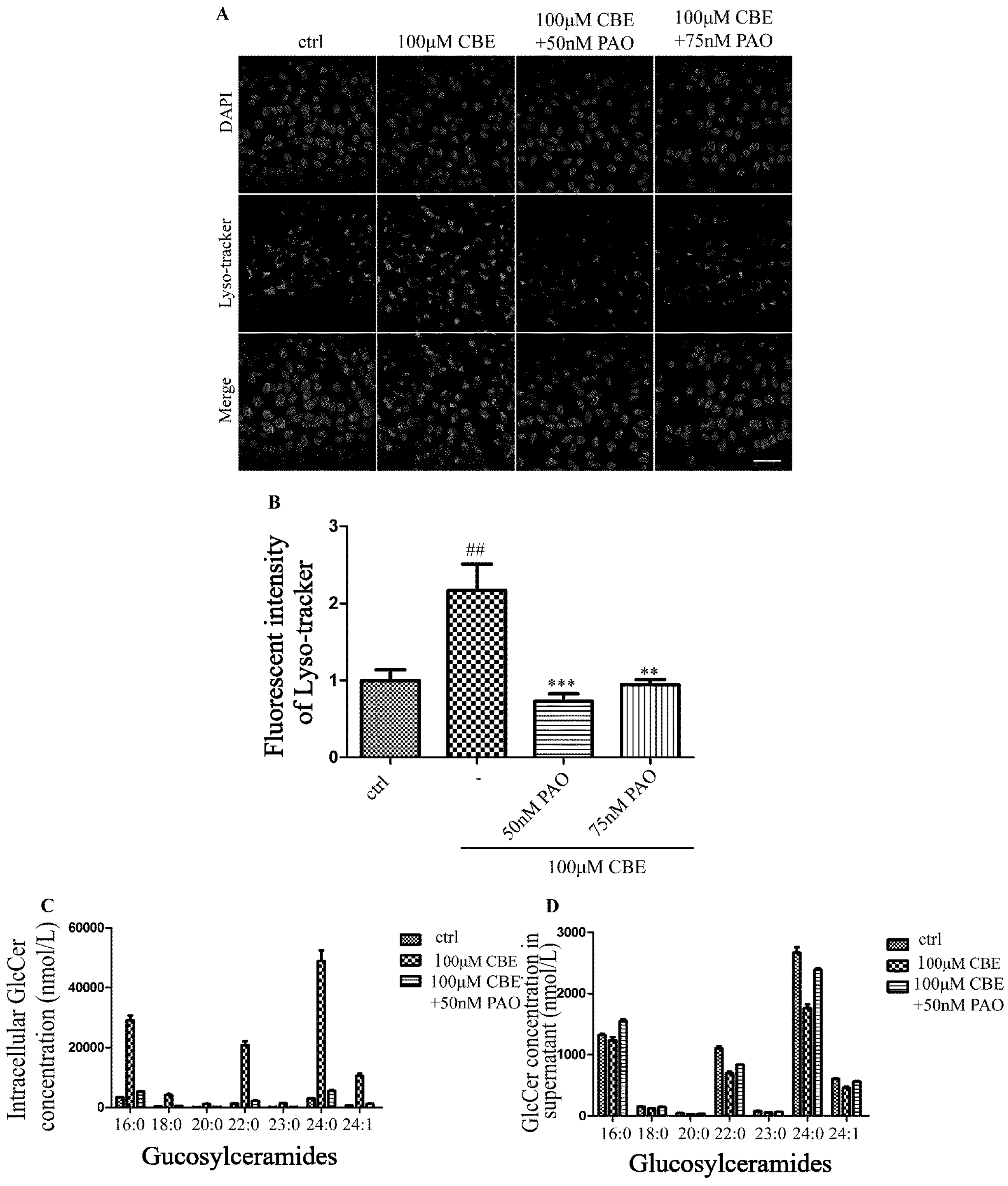
A
ctrl
100μM CBE
100μM CBE +50nM PAO
100μM CBE +75nM PAO
DAPI
Lyso-tracker
Merge
B
Fluorescent intensity of Lyso-tracker
##
***
**
ctrl
-
50nM PAO
75nM PAO
100μM CBE
C
Intracellular GlcCer concentration (nmol/L)
ctrl
100μM CBE
100μM CBE +50nM PAO
16:0 18:0 20:0 22:0 23:0 24:0 24:1
Gucosylceramides
D
GlcCer concentration in supernatant (nmol/L)
ctrl
100μM CBE
100μM CBE +50nM PAO
16:0 18:0 20:0 22:0 23:0 24:0 24:1
Glucosylceramides

*FIG. 10*

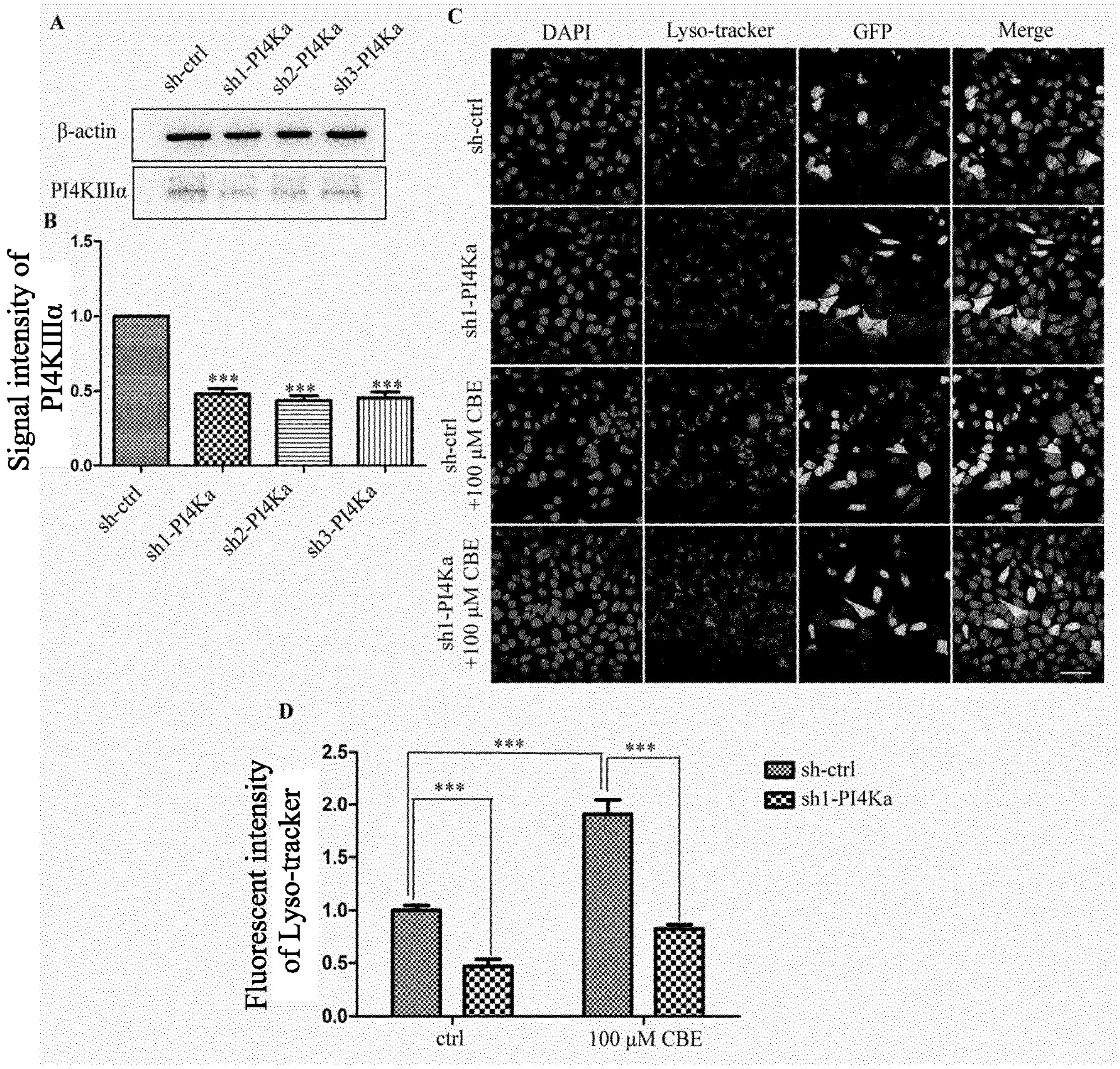
A
sh-ctrl
sh1-PI4Ka
sh2-PI4Ka
sh3-PI4Ka
β-actin
PI4KIIIα
B
Signal intensity of PI4KIIIα
1.5
1.0
0.5
0.0
***
sh-ctrl
sh1-PI4Ka
sh2-PI4Ka
sh3-PI4Ka
C
DAPI
Lyso-tracker
GFP
Merge
sh-ctrl
sh1-PI4Ka
sh-ctrl +100 μM CBE
sh1-PI4Ka +100 μM CBE
D
Fluorescent intensity of Lyso-tracker
2.5
2.0
1.5
1.0
0.5
0.0
***
ctrl
100 μM CBE
sh-ctrl
sh1-PI4Ka

*FIG. 11*

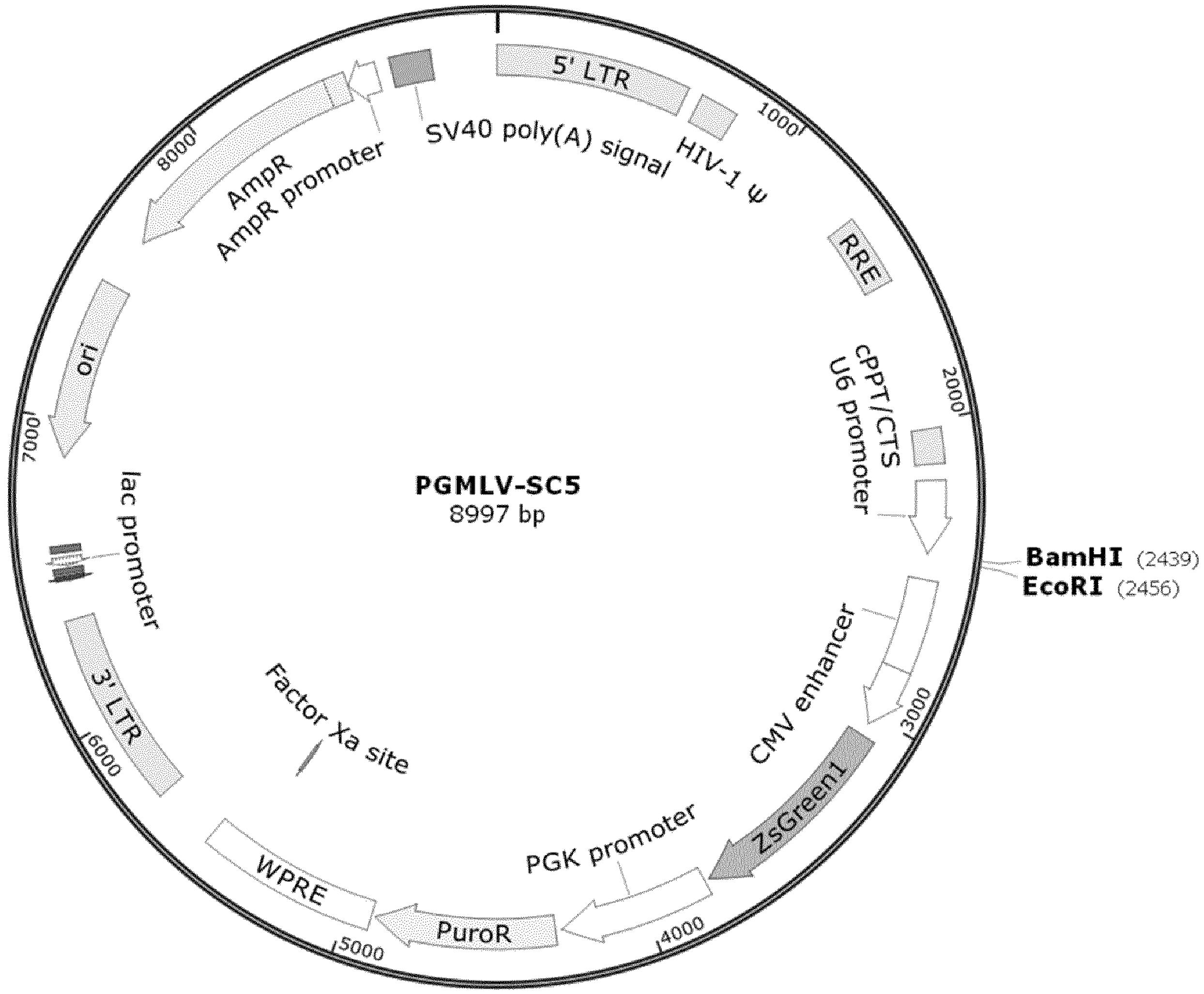
PGMLV-SC5
8997 bp
5' LTR
1000
HIV-1 Ψ
RRE
cPPT/CTS
U6 promoter
2000
BamHI (2439)
EcoRI (2456)
CMV enhancer
3000
ZsGreen1
PGK promoter
4000
PuroR
5000
WPRE
Factor Xa site
6000
3' LTR
lac promoter
7000
ori
8000
AmpR
AmpR promoter
SV40 poly(A) signal

*FIG. 12*

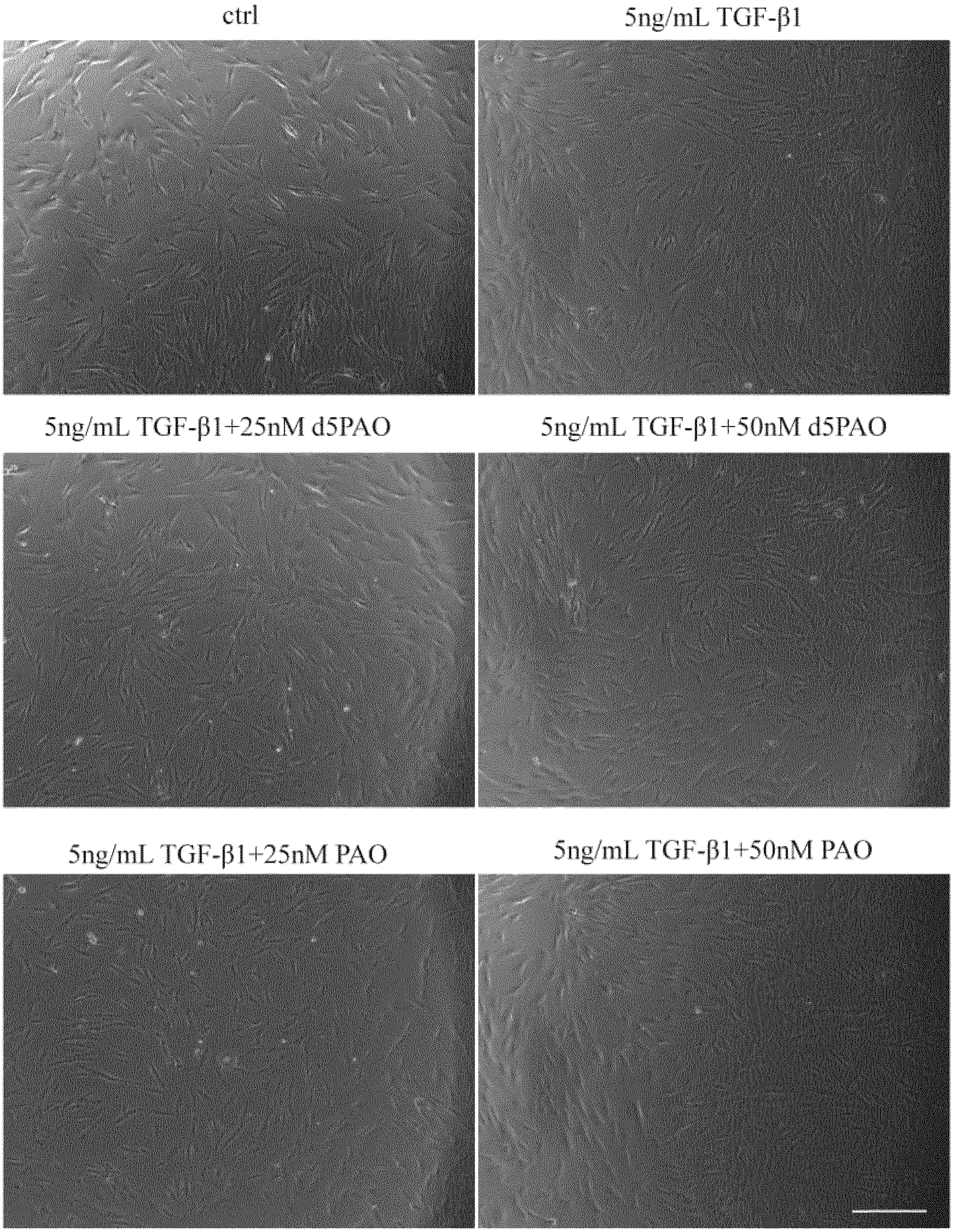
ctrl
5ng/mL TGF-β1
5ng/mL TGF-β1+25nM d5PAO
5ng/mL TGF-β1+50nM d5PAO
5ng/mL TGF-β1+25nM PAO
5ng/mL TGF-β1+50nM PAO

*FIG. 13*

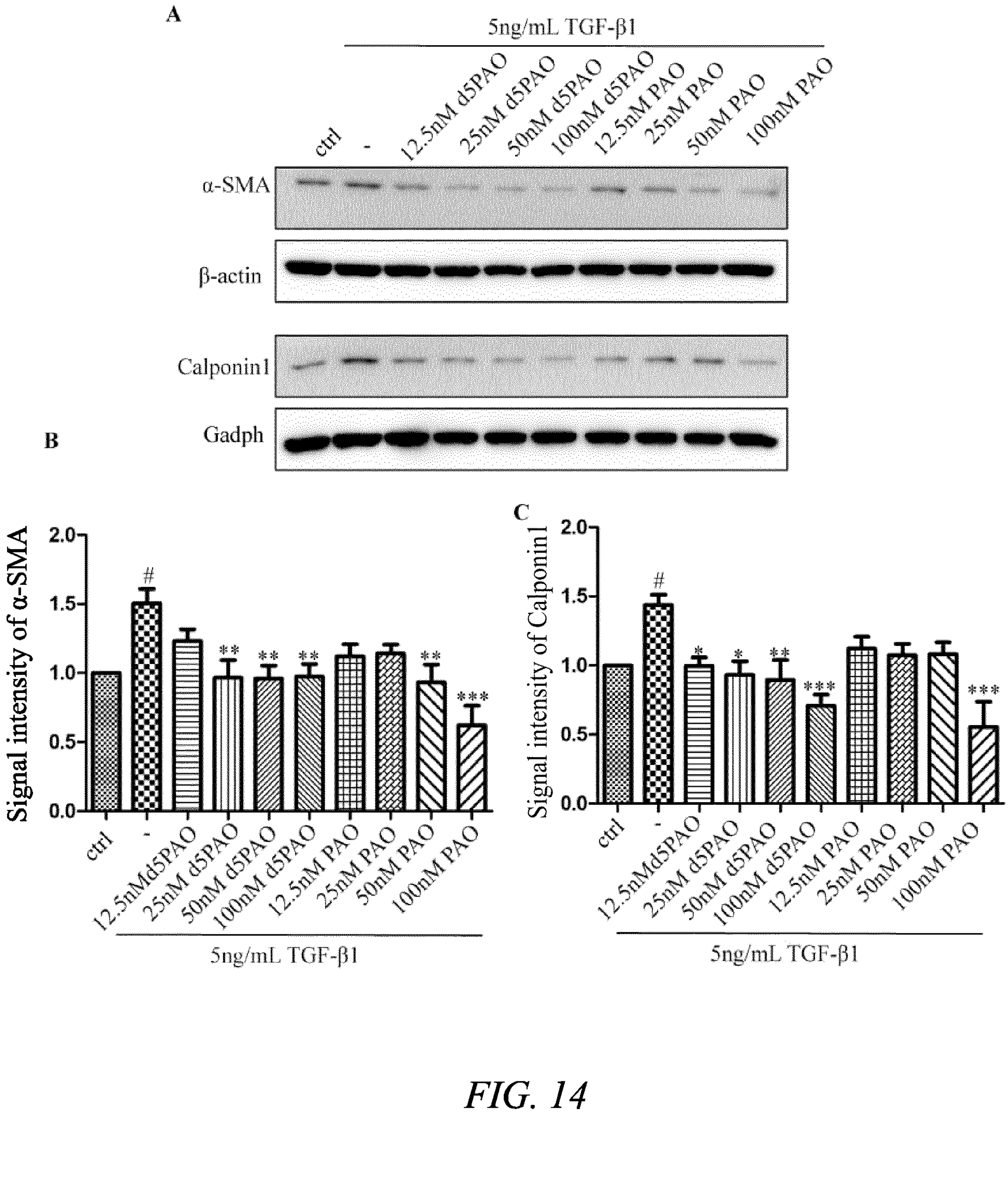
A
5ng/mL TGF-β1
ctrl
-
12.5nM d5PAO
25nM d5PAO
50nM d5PAO
100nM d5PAO
12.5nM PAO
25nM PAO
50nM PAO
100nM PAO
α-SMA
β-actin
Calponin1
Gadph
B
Signal intensity of α-SMA
2.0
1.5
1.0
0.5
0.0
#
**
**
**
**
***
ctrl
-
12.5nMd5PAO
25nM d5PAO
50nM d5PAO
100nM d5PAO
12.5nM PAO
25nM PAO
50nM PAO
100nM PAO
5ng/mL TGF-β1
C
Signal intensity of Calponin1
2.0
1.5
1.0
0.5
0.0
#
*
*
**
***
***
ctrl
-
12.5nMd5PAO
25nM d5PAO
50nM d5PAO
100nM d5PAO
12.5nM PAO
25nM PAO
50nM PAO
100nM PAO
5ng/mL TGF-β1

*FIG. 14*

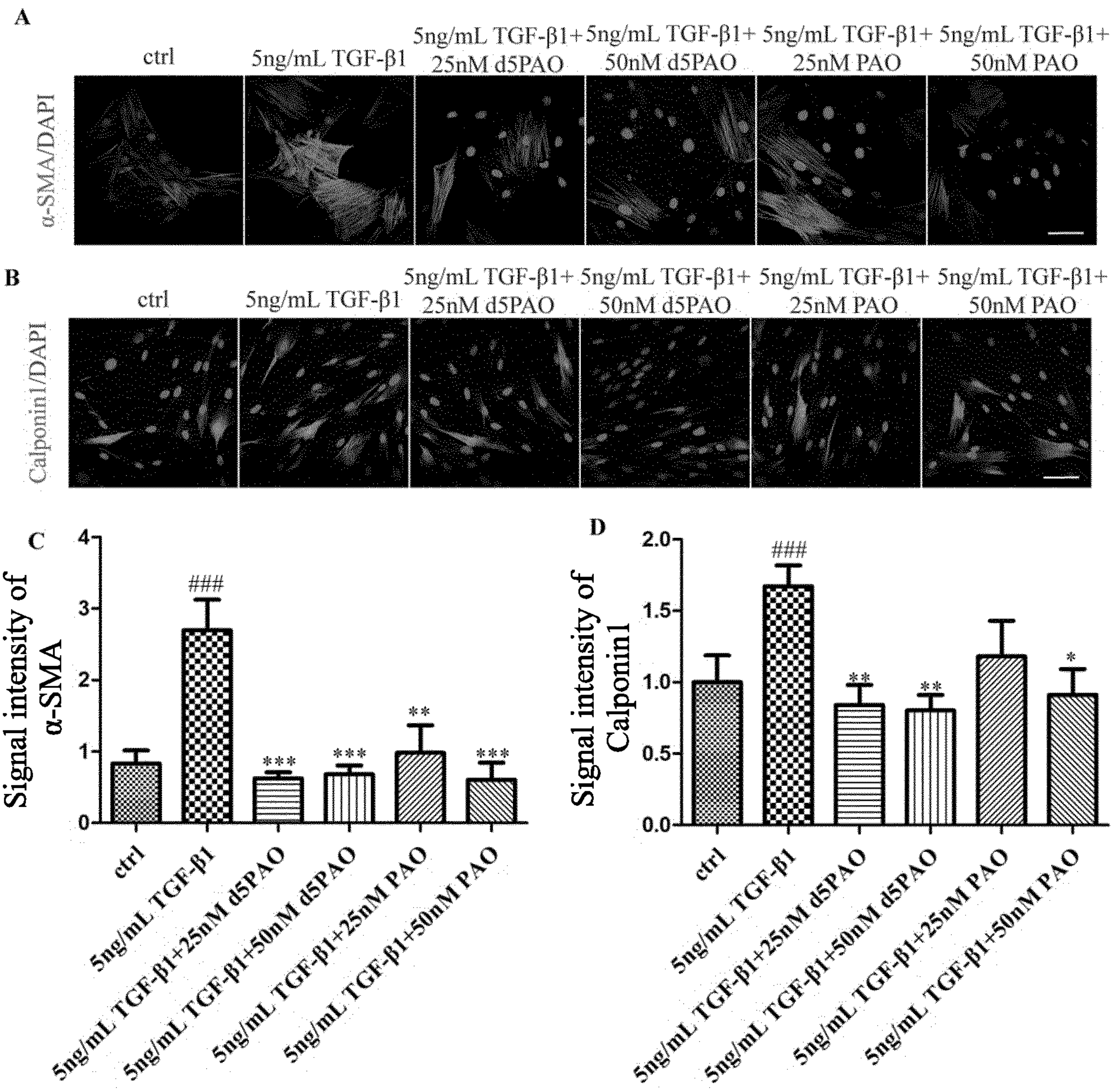
A
ctrl
5ng/mL TGF-β1
5ng/mL TGF-β1+ 25nM d5PAO
5ng/mL TGF-β1+ 50nM d5PAO
5ng/mL TGF-β1+ 25nM PAO
5ng/mL TGF-β1+ 50nM PAO
α-SMA/DAPI
B
Calponin1/DAPI
C
Signal intensity of α-SMA
###
***
**
ctrl
5ng/mL TGF-β1
5ng/mL TGF-β1+25nM d5PAO
5ng/mL TGF-β1+50nM d5PAO
5ng/mL TGF-β1+25nM PAO
5ng/mL TGF-β1+50nM PAO
D
Signal intensity of Calponin1
*

*FIG. 15*

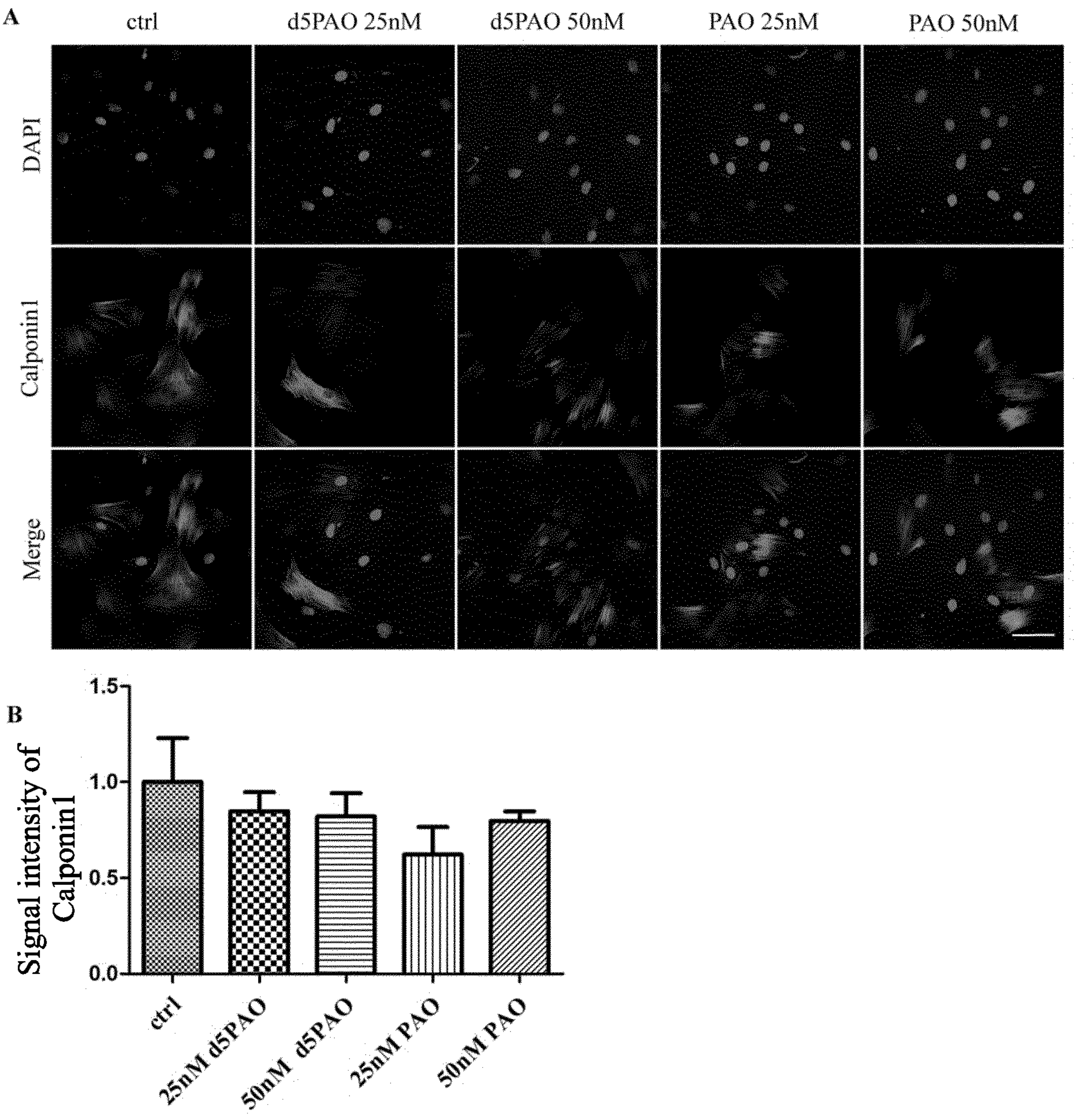
A
ctrl
d5PAO 25nM
d5PAO 50nM
PAO 25nM
PAO 50nM
DAPI
Calponin1
Merge
B
Signal intensity of Calponin1
1.5
1.0
0.5
0.0
ctrl
25nM d5PAO
50nM d5PAO
25nM PAO
50nM PAO

*FIG. 16*

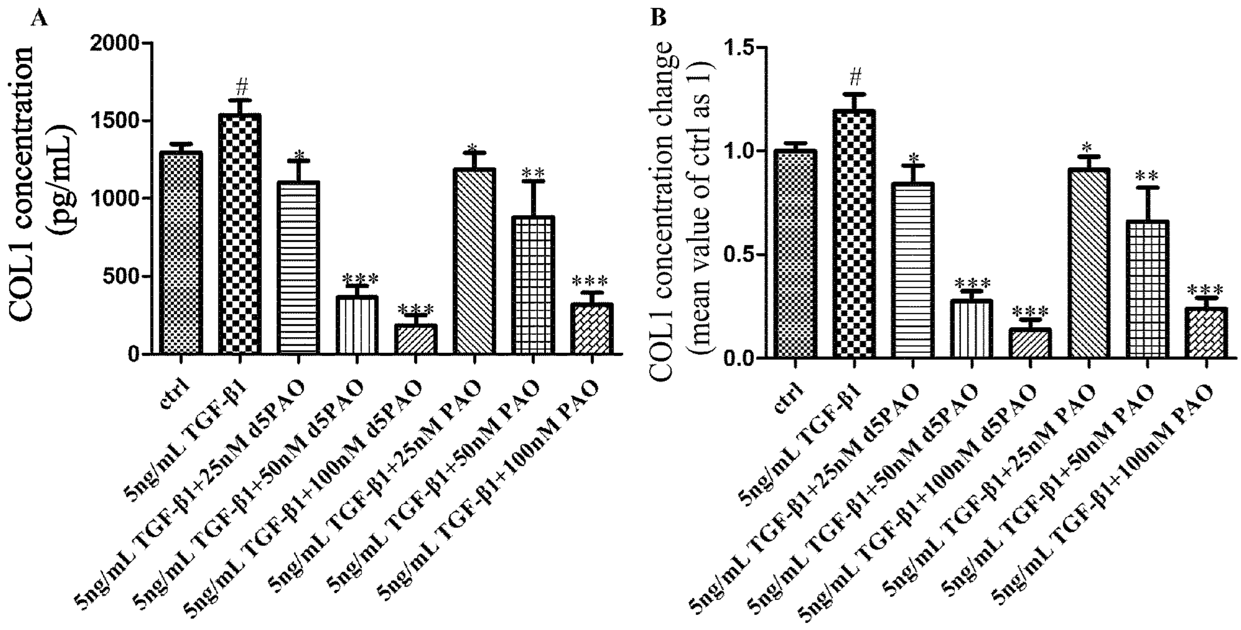
A
COL1 concentration (pg/mL)
2000
1500
1000
500
0
#
*
***
***
*
**
***
ctrl
5ng/mL TGF-β1
5ng/mL TGF-β1+25nM d5PAO
5ng/mL TGF-β1+50nM d5PAO
5ng/mL TGF-β1+100nM d5PAO
5ng/mL TGF-β1+25nM PAO
5ng/mL TGF-β1+50nM PAO
5ng/mL TGF-β1+100nM PAO
B
COL1 concentration change (mean value of ctrl as 1)
1.5
1.0
0.5
0.0

*FIG. 17*

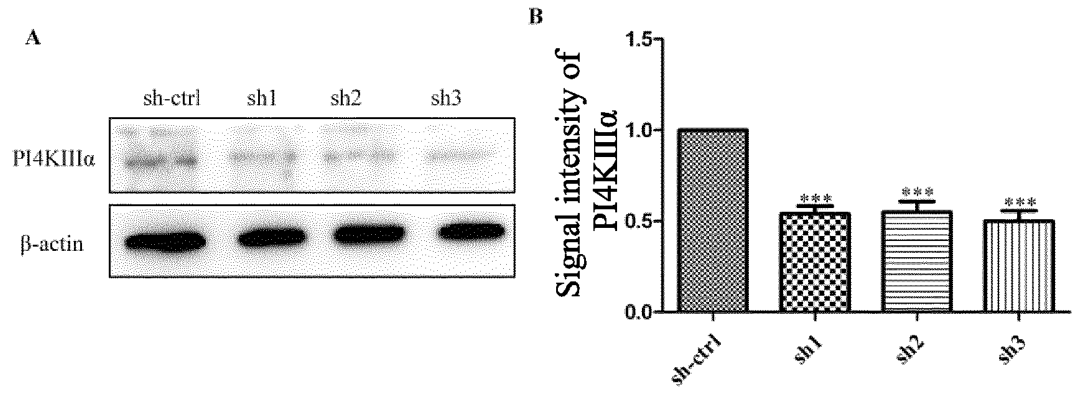
A
sh-ctrl
sh1
sh2
sh3
PI4KIIIα
β-actin
B
Signal intensity of PI4KIIIα
1.5
1.0
0.5
0.0
***
sh-ctrl
sh1
sh2
sh3

*FIG. 18*

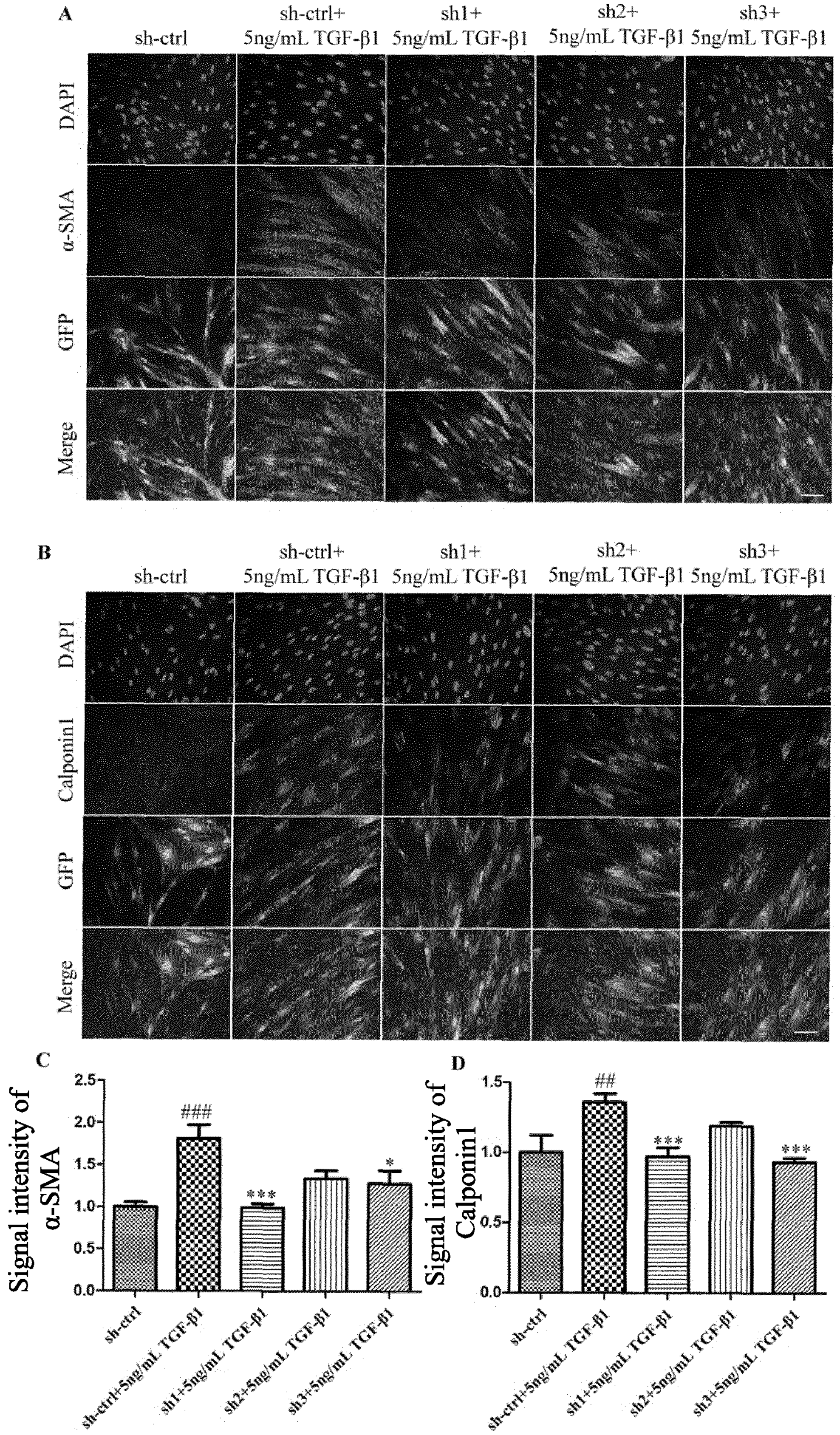
A
sh-ctrl
sh-ctrl+ 5ng/mL TGF-β1
sh1+ 5ng/mL TGF-β1
sh2+ 5ng/mL TGF-β1
sh3+ 5ng/mL TGF-β1
DAPI
α-SMA
GFP
Merge
B
sh-ctrl
sh-ctrl+ 5ng/mL TGF-β1
sh1+ 5ng/mL TGF-β1
sh2+ 5ng/mL TGF-β1
sh3+ 5ng/mL TGF-β1
DAPI
Calponin1
GFP
Merge
C
Signal intensity of α-SMA
2.5
2.0
1.5
1.0
0.5
0.0
###
***
*
sh-ctrl
sh-ctrl+5ng/mL TGF-β1
sh1+5ng/mL TGF-β1
sh2+5ng/mL TGF-β1
sh3+5ng/mL TGF-β1
D
Signal intensity of Calponin1
1.5
1.0
0.5
0.0
##
***
***
sh-ctrl
sh-ctrl+5ng/mL TGF-β1
sh1+5ng/mL TGF-β1
sh2+5ng/mL TGF-β1
sh3+5ng/mL TGF-β1

*FIG. 19*

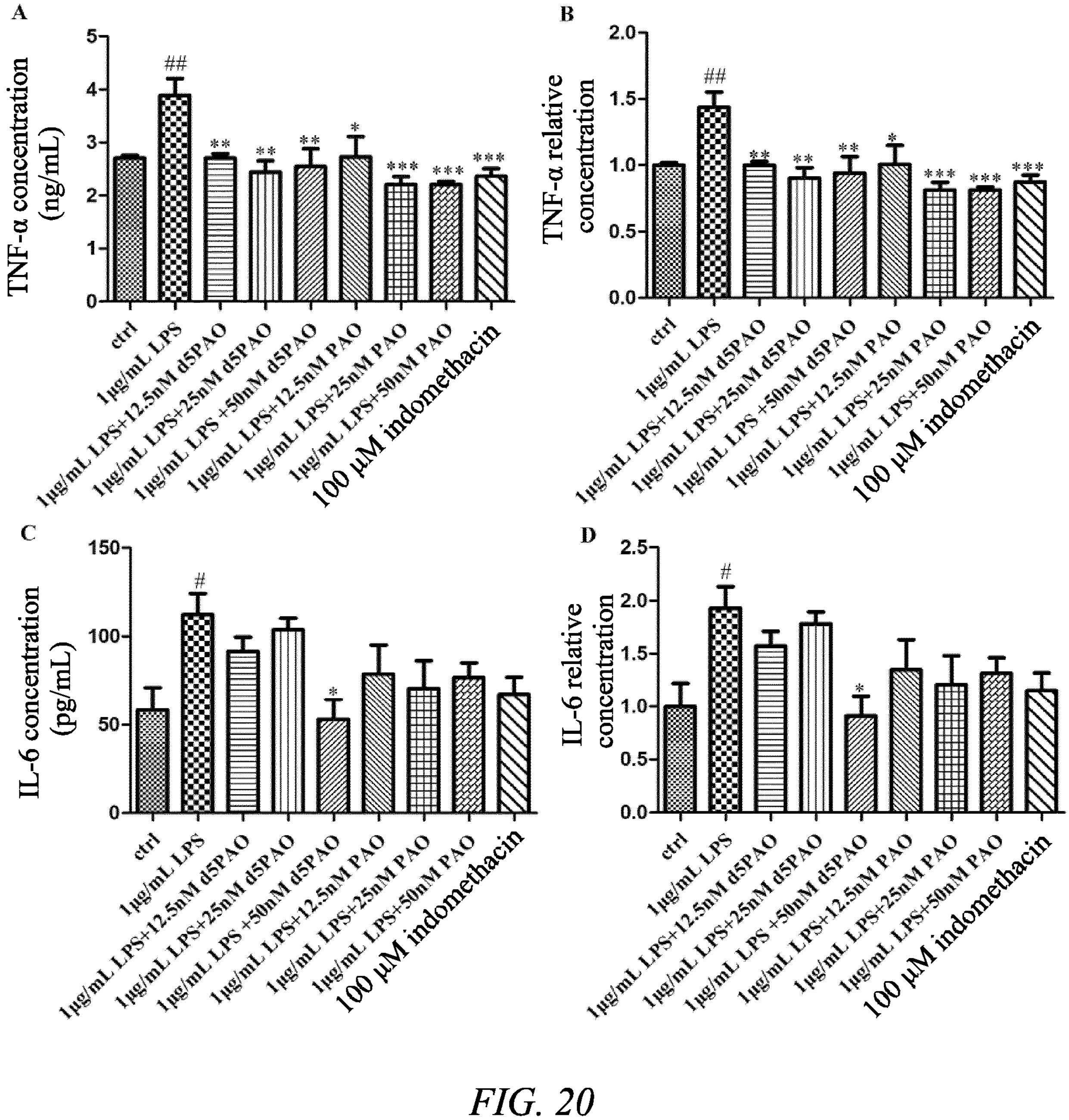
A
TNF-α concentration (ng/mL)
##
**
**
**
*
***
***
***
B
TNF-α relative concentration
##
**
**
**
*
***
***
***
C
IL-6 concentration (pg/mL)
#
*
D
IL-6 relative concentration
#
*
ctrl
1μg/mL LPS
1μg/mL LPS+12.5nM d5PAO
1μg/mL LPS+25nM d5PAO
1μg/mL LPS +50nM d5PAO
1μg/mL LPS+12.5nM PAO
1μg/mL LPS+25nM PAO
1μg/mL LPS+50nM PAO
100 μM indomethacin

*FIG. 20*

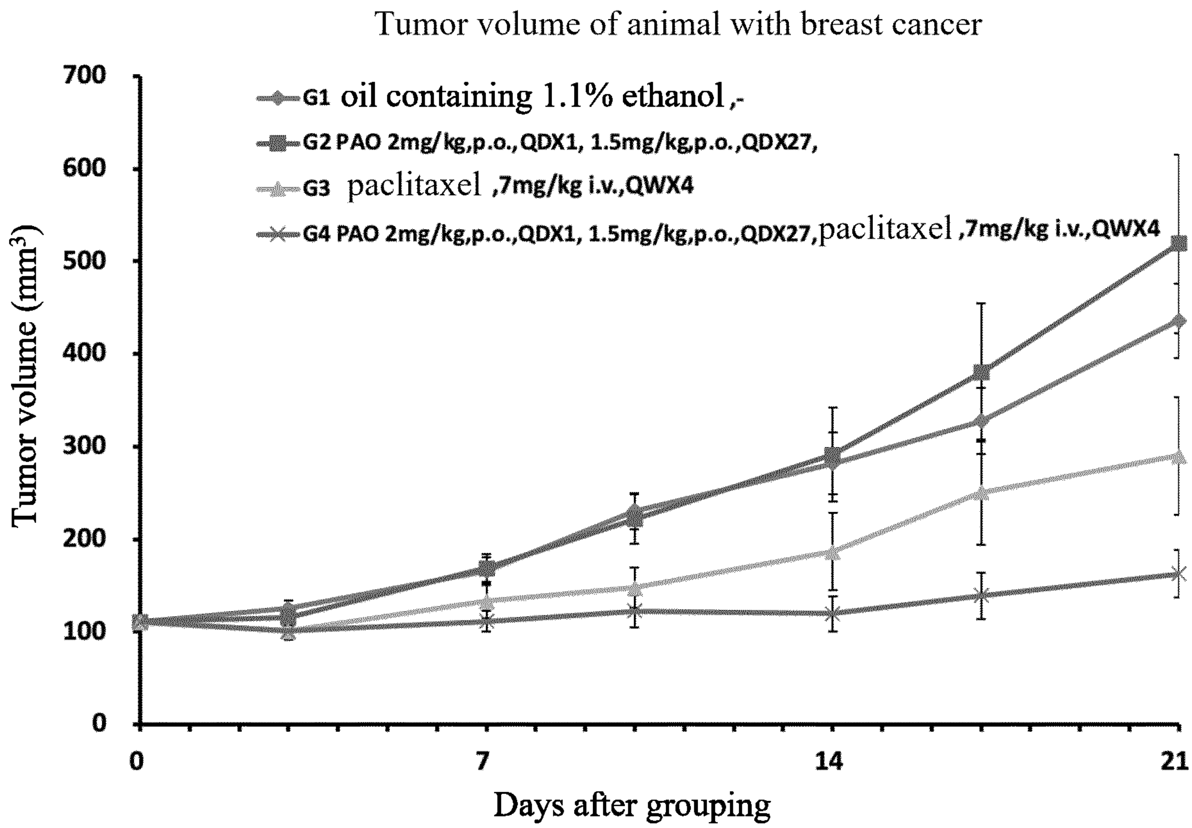
Tumor volume of animal with breast cancer
G1 oil containing 1.1% ethanol,-
G2 PAO 2mg/kg,p.o.,QDX1, 1.5mg/kg,p.o.,QDX27,
G3 paclitaxel ,7mg/kg i.v.,QWX4
G4 PAO 2mg/kg,p.o.,QDX1, 1.5mg/kg,p.o.,QDX27,paclitaxel ,7mg/kg i.v.,QWX4
Tumor volume ($mm^3$)
700
600
500
400
300
200
100
0
0
7
14
21
Days after grouping

*FIG. 21*

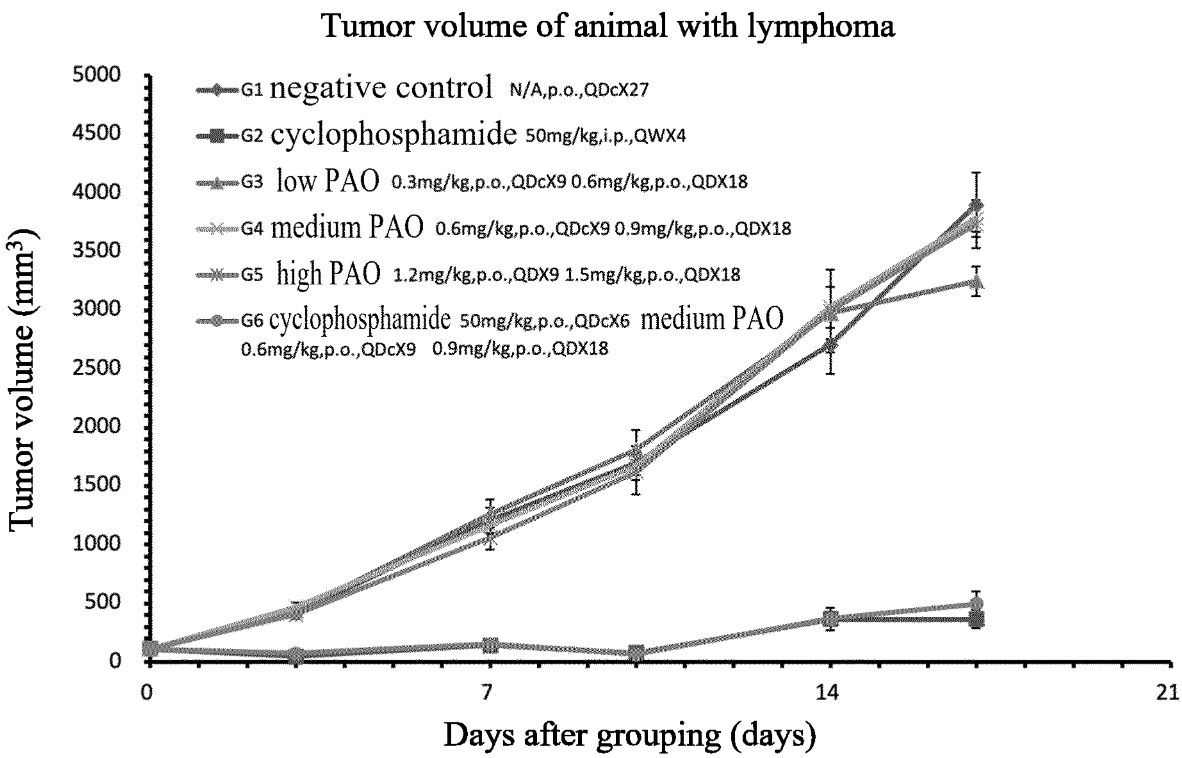
Tumor volume of animal with lymphoma
G1 negative control N/A,p.o.,QDcX27
G2 cyclophosphamide 50mg/kg,i.p.,QWX4
G3 low PAO 0.3mg/kg,p.o.,QDcX9 0.6mg/kg,p.o.,QDX18
G4 medium PAO 0.6mg/kg,p.o.,QDcX9 0.9mg/kg,p.o.,QDX18
G5 high PAO 1.2mg/kg,p.o.,QDX9 1.5mg/kg,p.o.,QDX18
G6 cyclophosphamide 50mg/kg,p.o.,QDcX6 medium PAO 0.6mg/kg,p.o.,QDcX9 0.9mg/kg,p.o.,QDX18
Tumor volume ($mm^3$)
5000
4500
4000
3500
3000
2500
2000
1500
1000
500
0
0
7
14
21
Days after grouping (days)

*FIG. 22*

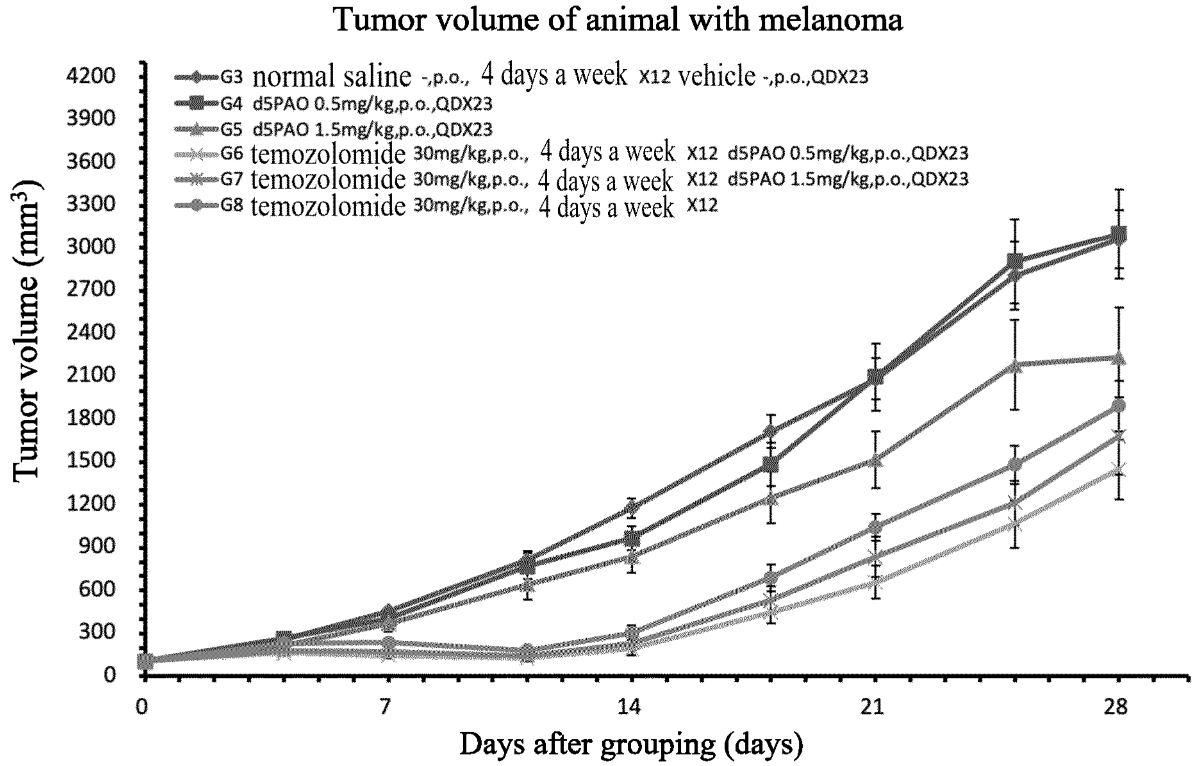
Tumor volume of animal with melanoma
G3 normal saline -,p.o., 4 days a week X12 vehicle -,p.o.,QDX23
G4 d5PAO 0.5mg/kg,p.o.,QDX23
G5 d5PAO 1.5mg/kg,p.o.,QDX23
G6 temozolomide 30mg/kg,p.o., 4 days a week X12 d5PAO 0.5mg/kg,p.o.,QDX23
G7 temozolomide 30mg/kg,p.o., 4 days a week X12 d5PAO 1.5mg/kg,p.o.,QDX23
G8 temozolomide 30mg/kg,p.o., 4 days a week X12
Tumor volume (mm3)
4200
3900
3600
3300
3000
2700
2400
2100
1800
1500
1200
900
600
300
0
0
7
14
21
28
Days after grouping (days)

*FIG. 23*

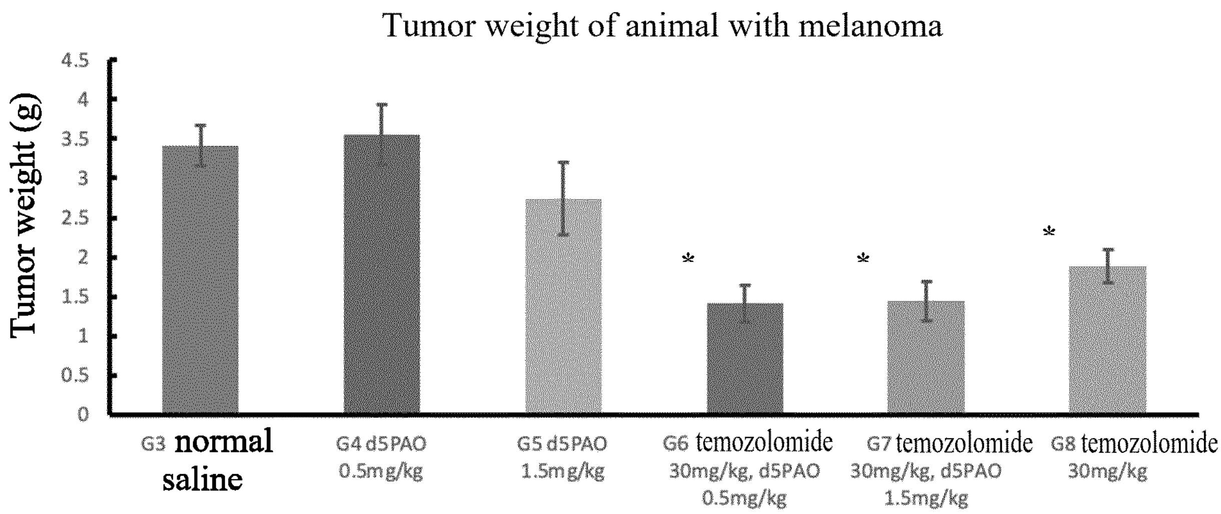
Tumor weight of animal with melanoma
Tumor weight (g)
4.5
4
3.5
3
2.5
2
1.5
1
0.5
0
*
*
*
G3 normal saline
G4 d5PAO 0.5mg/kg
G5 d5PAO 1.5mg/kg
G6 temozolomide 30mg/kg, d5PAO 0.5mg/kg
G7 temozolomide 30mg/kg, d5PAO 1.5mg/kg
G8 temozolomide 30mg/kg

*FIG. 24*

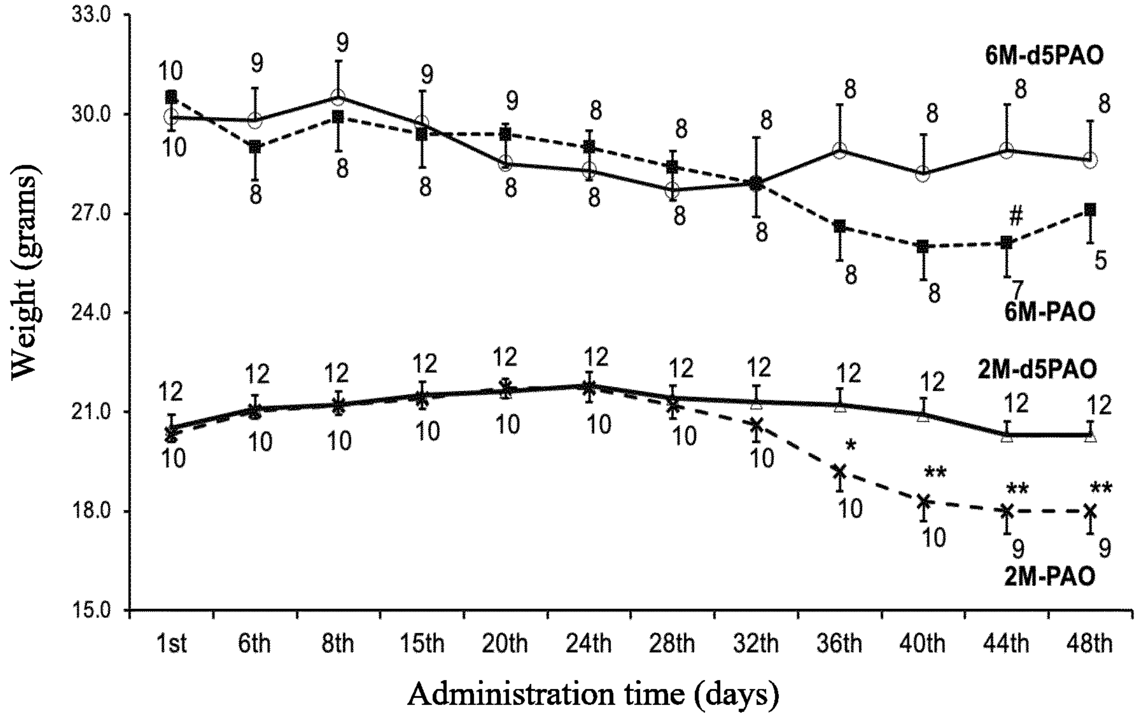
33.0
30.0
27.0
24.0
21.0
18.0
15.0
Weight (grams)
6M-d5PAO
6M-PAO
2M-d5PAO
2M-PAO
1st
6th
8th
15th
20th
24th
28th
32th
36th
40th
44th
48th
Administration time (days)

*FIG. 25*

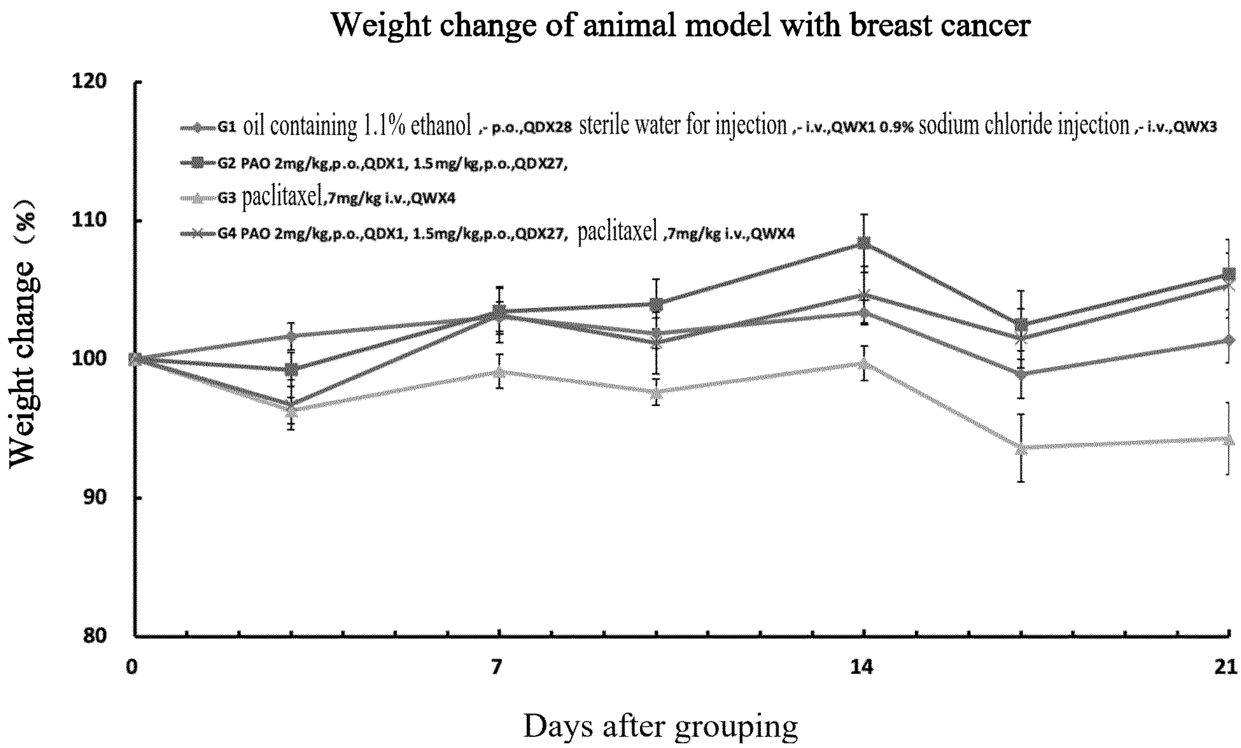
Weight change of animal model with breast cancer
G1 oil containing 1.1% ethanol ,- p.o.,QDX28 sterile water for injection ,- i.v.,QWX1 0.9% sodium chloride injection ,- i.v.,QWX3
G2 PAO 2mg/kg,p.o.,QDX1, 1.5mg/kg,p.o.,QDX27,
G3 paclitaxel,7mg/kg i.v.,QWX4
G4 PAO 2mg/kg,p.o.,QDX1, 1.5mg/kg,p.o.,QDX27, paclitaxel ,7mg/kg i.v.,QWX4
120
110
100
90
80
Weight change（%）
0
7
14
21
Days after grouping

*FIG. 26*

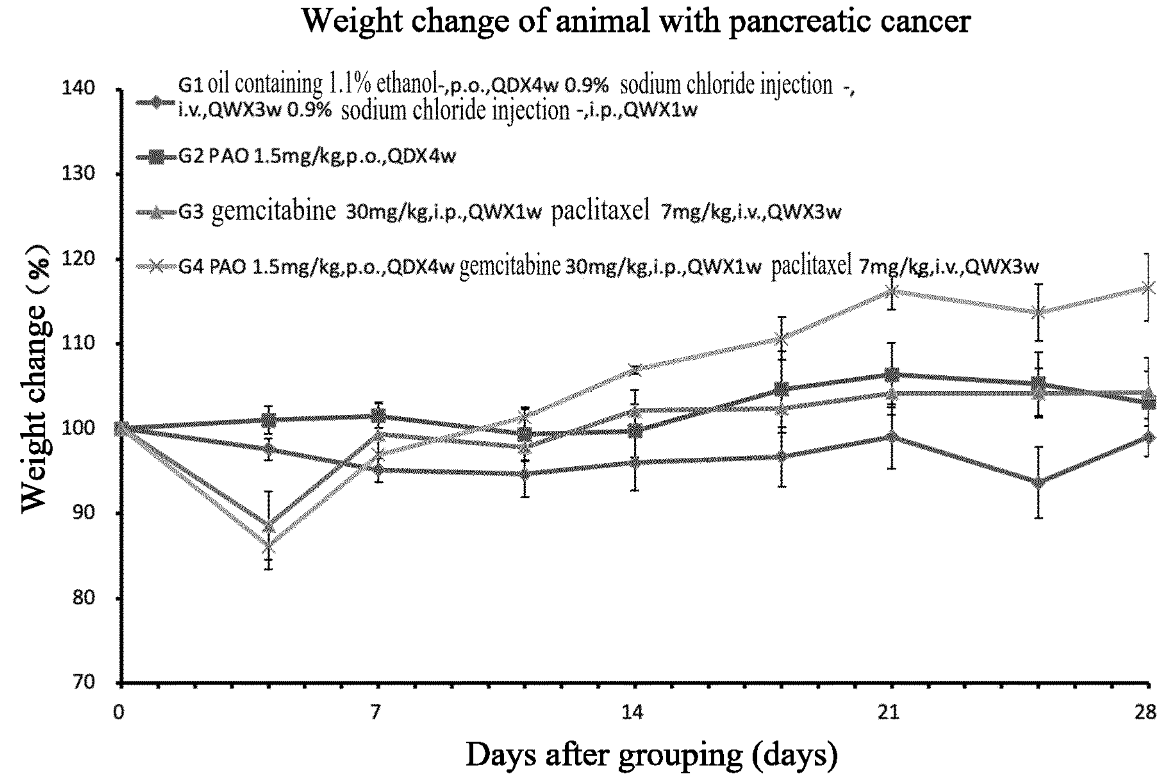
Weight change of animal with pancreatic cancer
G1 oil containing 1.1% ethanol-,p.o.,QDX4w 0.9% sodium chloride injection -, i.v.,QWX3w 0.9% sodium chloride injection -,i.p.,QWX1w
G2 PAO 1.5mg/kg,p.o.,QDX4w
G3 gemcitabine 30mg/kg,i.p.,QWX1w paclitaxel 7mg/kg,i.v.,QWX3w
G4 PAO 1.5mg/kg,p.o.,QDX4w gemcitabine 30mg/kg,i.p.,QWX1w paclitaxel 7mg/kg,i.v.,QWX3w
Weight change (%)
140
130
120
110
100
90
80
70
0
7
14
21
28
Days after grouping (days)

*FIG. 27*

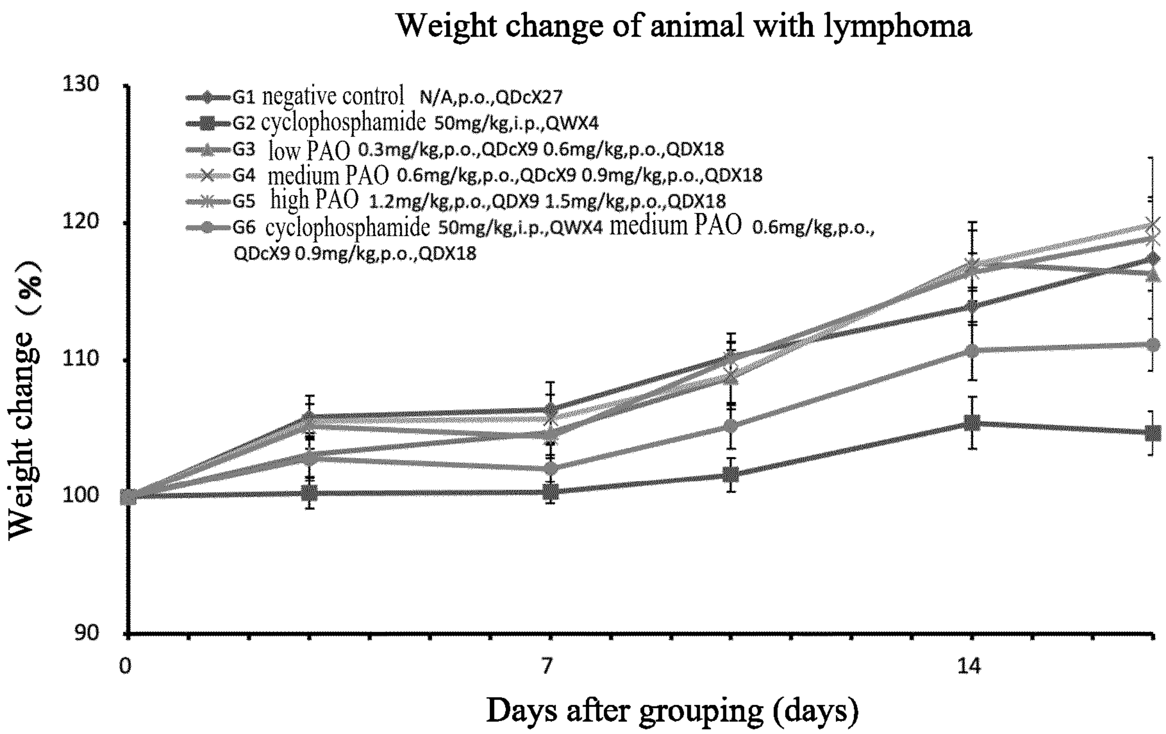
Weight change of animal with lymphoma
G1 negative control N/A,p.o.,QDcX27
G2 cyclophosphamide 50mg/kg,i.p.,QWX4
G3 low PAO 0.3mg/kg,p.o.,QDcX9 0.6mg/kg,p.o.,QDX18
G4 medium PAO 0.6mg/kg,p.o.,QDcX9 0.9mg/kg,p.o.,QDX18
G5 high PAO 1.2mg/kg,p.o.,QDX9 1.5mg/kg,p.o.,QDX18
G6 cyclophosphamide 50mg/kg,i.p.,QWX4 medium PAO 0.6mg/kg,p.o., QDcX9 0.9mg/kg,p.o.,QDX18
Weight change (%)
130
120
110
100
90
0
7
14
Days after grouping (days)

*FIG. 28*

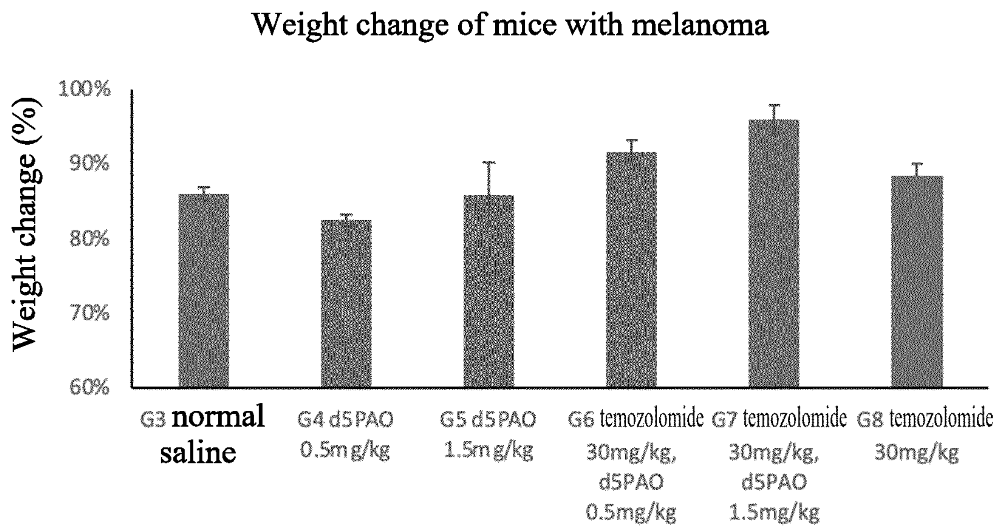


*FIG. 29*

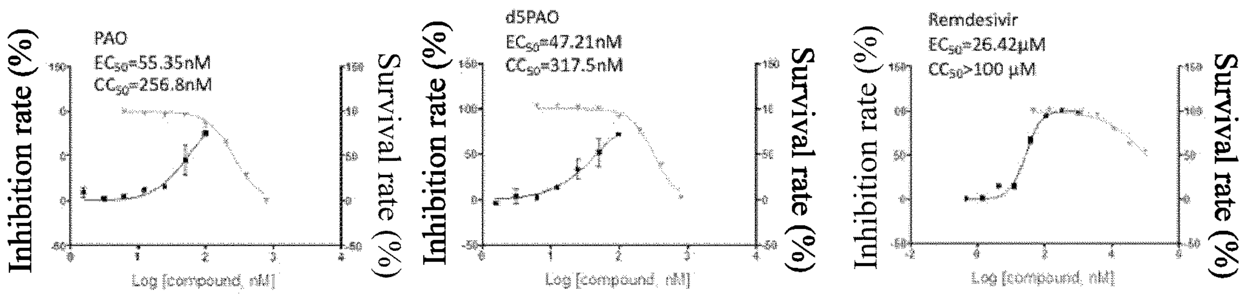


*FIG. 30*

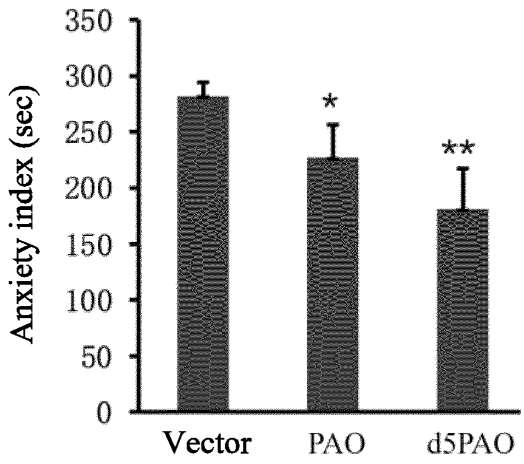


*FIG. 31*

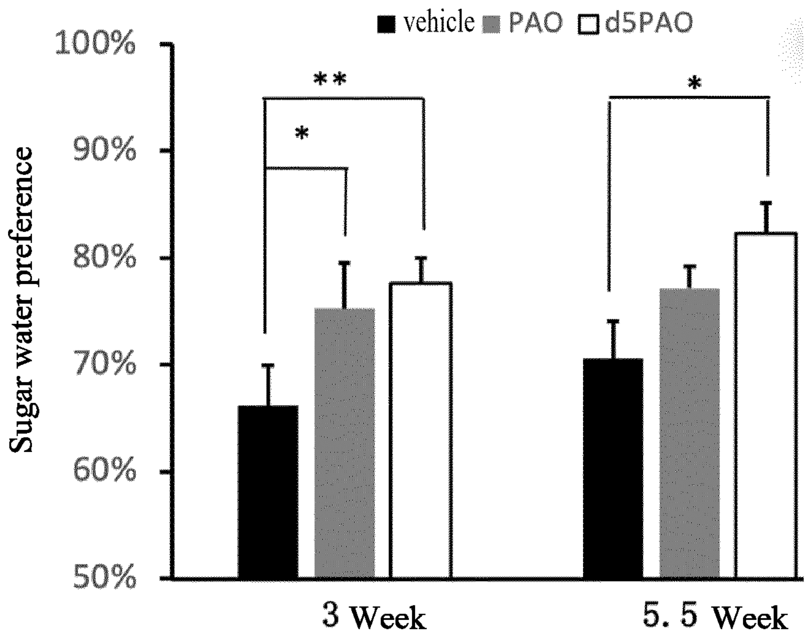
vehicle
PAO
d5PAO
100%
90%
80%
70%
60%
50%
Sugar water preference
**
*
*
3 Week
5. 5 Week

*FIG. 32*

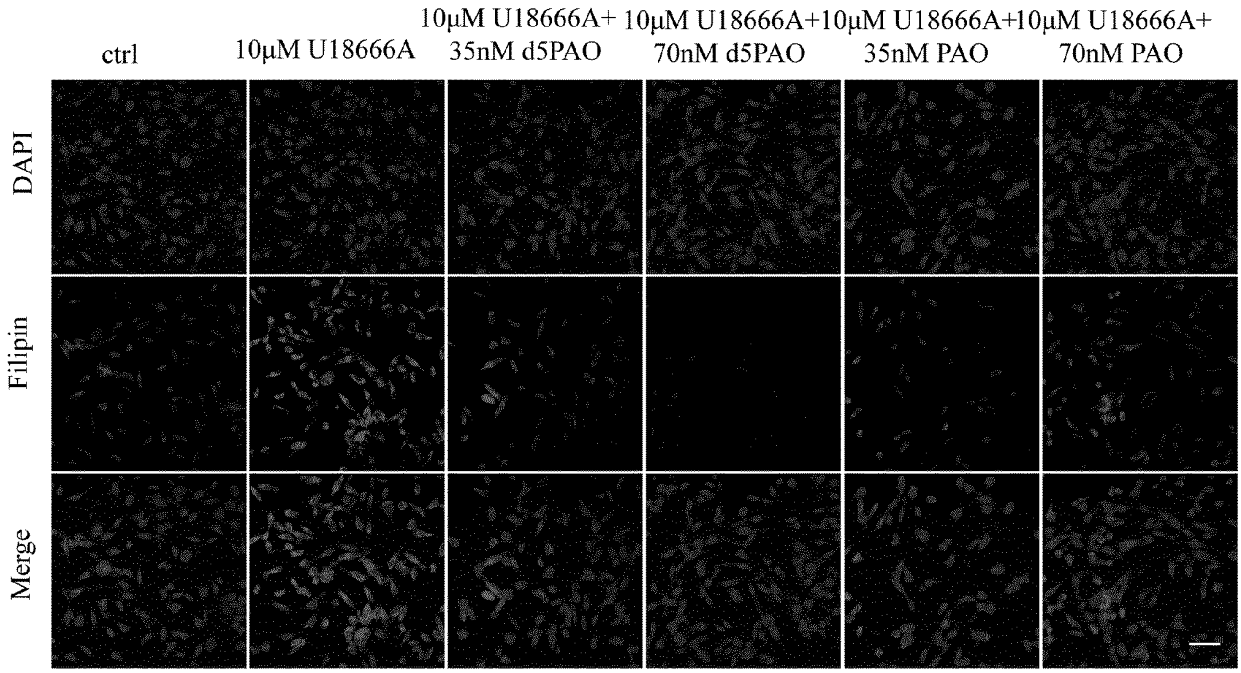
ctrl
10μM U18666A
10μM U18666A+ 35nM d5PAO
10μM U18666A+ 70nM d5PAO
10μM U18666A+ 35nM PAO
10μM U18666A+ 70nM PAO
DAPI
Filipin
Merge

*FIG. 33*

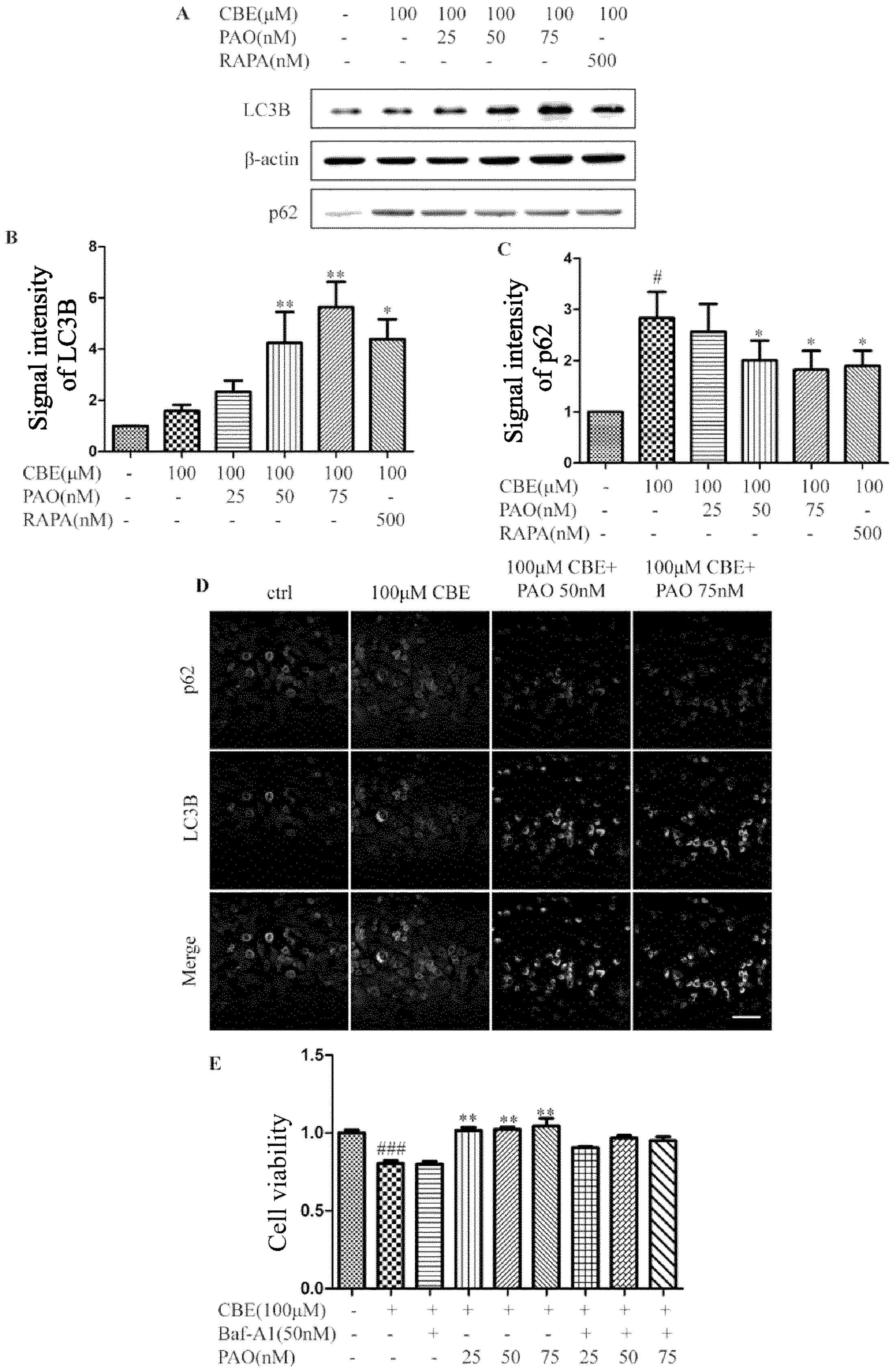
A
CBE(μM) - 100 100 100 100 100
PAO(nM) - - 25 50 75 -
RAPA(nM) - - - - - 500
LC3B
β-actin
p62
B
Signal intensity of LC3B
CBE(μM) - 100 100 100 100 100
PAO(nM) - - 25 50 75 -
RAPA(nM) - - - - - 500
C
Signal intensity of p62
CBE(μM) - 100 100 100 100 100
PAO(nM) - - 25 50 75 -
RAPA(nM) - - - - - 500
D
ctrl
100μM CBE
100μM CBE+ PAO 50nM
100μM CBE+ PAO 75nM
p62
LC3B
Merge
E
Cell viability
CBE(100μM) - + + + + + + + +
Baf-A1(50nM) - - + - - - + + +
PAO(nM) - - - 25 50 75 25 50 75

*FIG. 34*

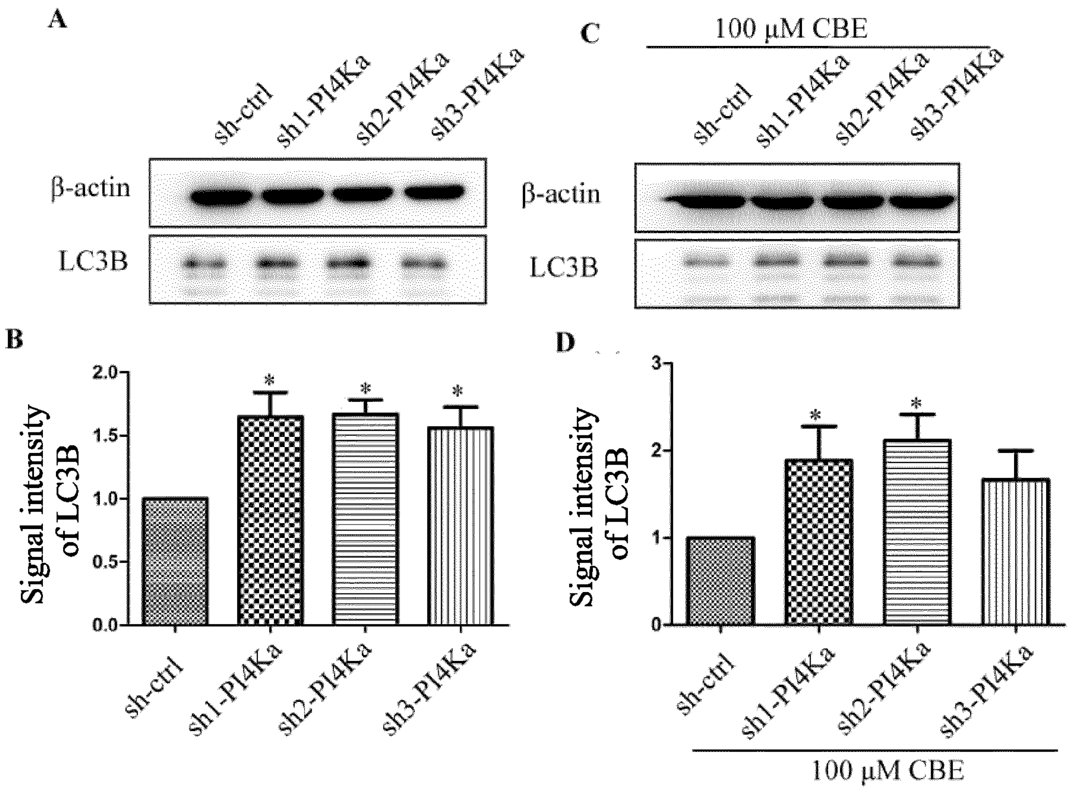
A
sh-ctrl
sh1-PI4Ka
sh2-PI4Ka
sh3-PI4Ka
β-actin
LC3B
C
100 μM CBE
B
Signal intensity of LC3B
2.0
1.5
1.0
0.5
0.0
*
D
3
2
1
0

*FIG. 35*

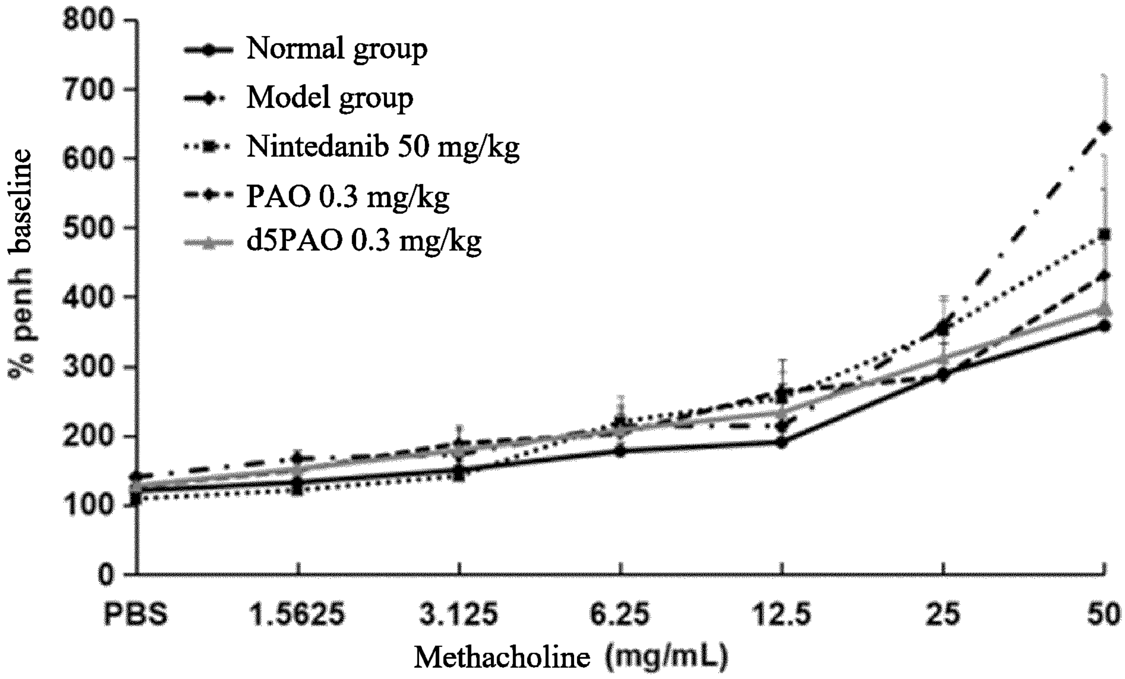
Normal group
Model group
Nintedanib 50 mg/kg
PAO 0.3 mg/kg
d5PAO 0.3 mg/kg
% penh baseline
800
700
600
500
400
300
200
100
0
PBS
1.5625
3.125
6.25
12.5
25
50
Methacholine (mg/mL)

*FIG. 36*

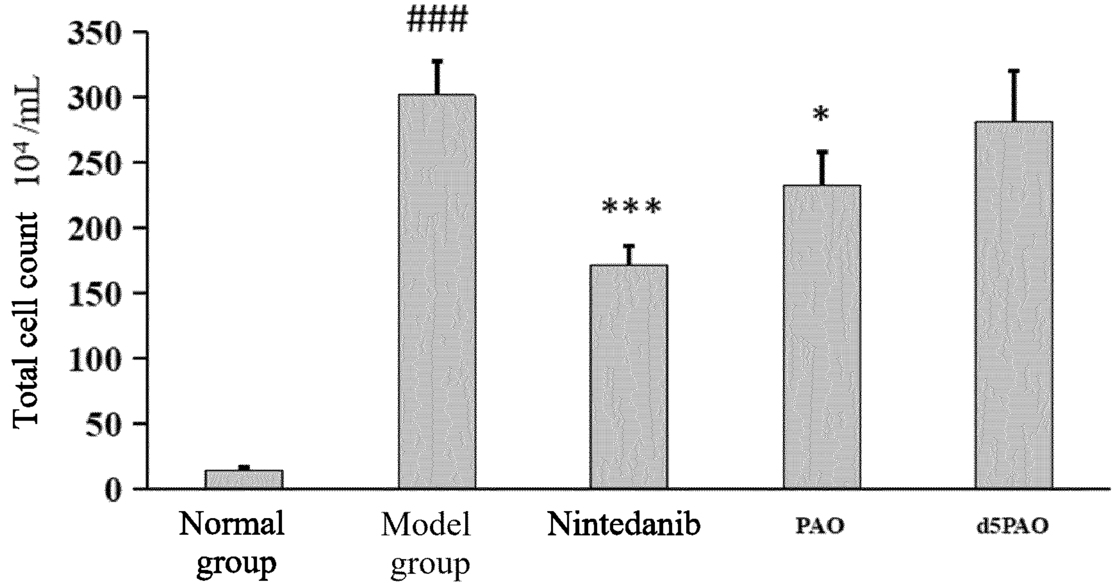
Total cell count 10⁴/mL
350
300
250
200
150
100
50
0
###
***
*
Normal group
Model group
Nintedanib
PAO
d5PAO

*FIG. 37*

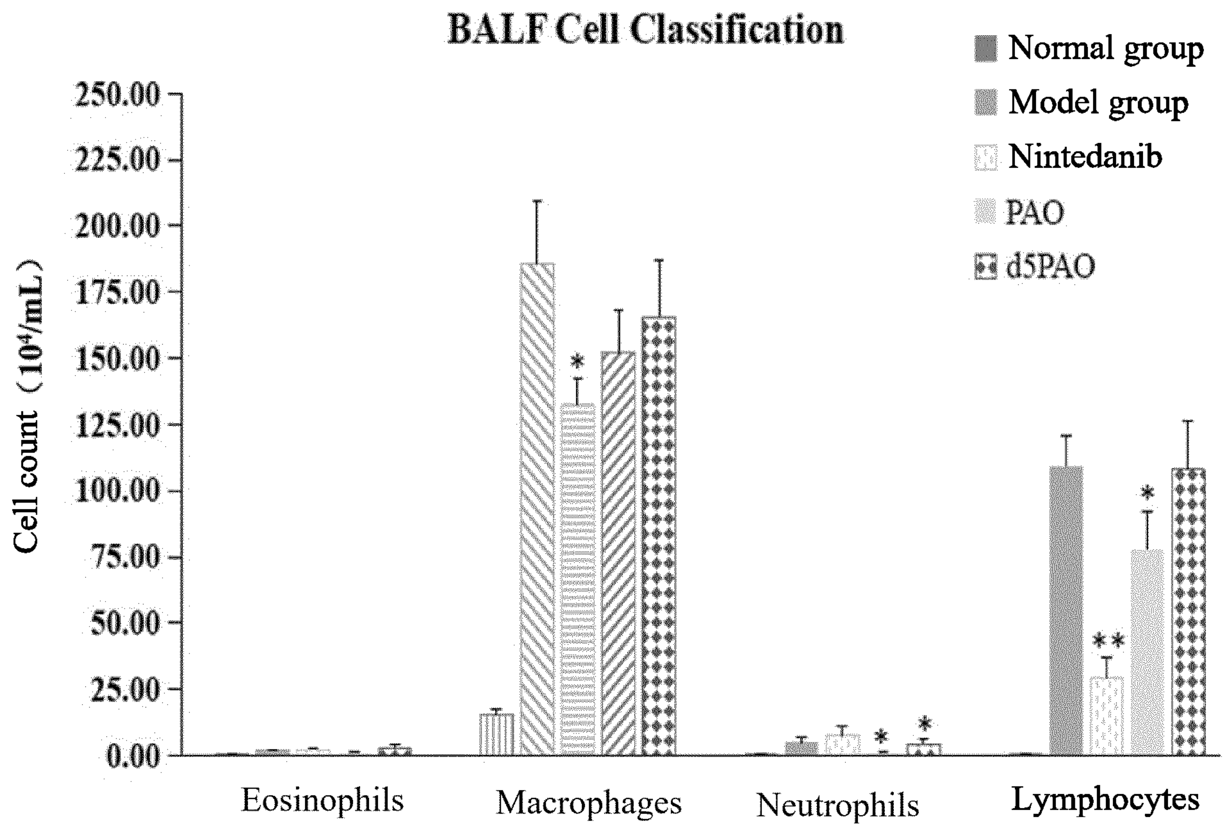
BALF Cell Classification
Normal group
Model group
Nintedanib
PAO
d5PAO
Cell count（10⁴/mL）
250.00
225.00
200.00
175.00
150.00
125.00
100.00
75.00
50.00
25.00
0.00
*
*
**
*
*
Eosinophils
Macrophages
Neutrophils
Lymphocytes

*FIG. 38*

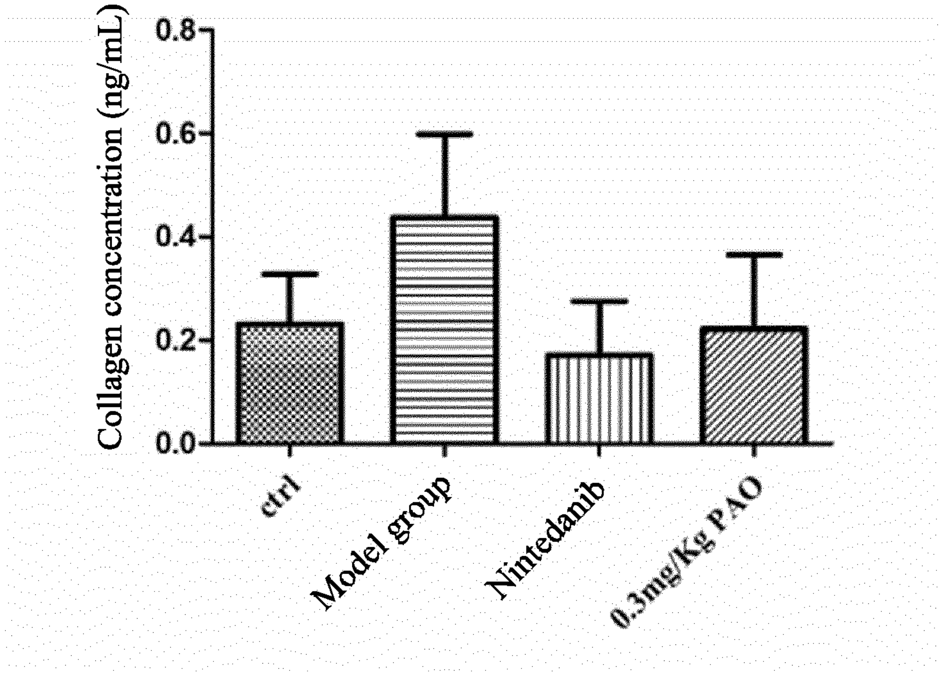
Collagen concentration (ng/mL)
0.8
0.6
0.4
0.2
0.0
ctrl
Model group
Nintedanib
0.3mg/Kg PAO

*FIG. 39*

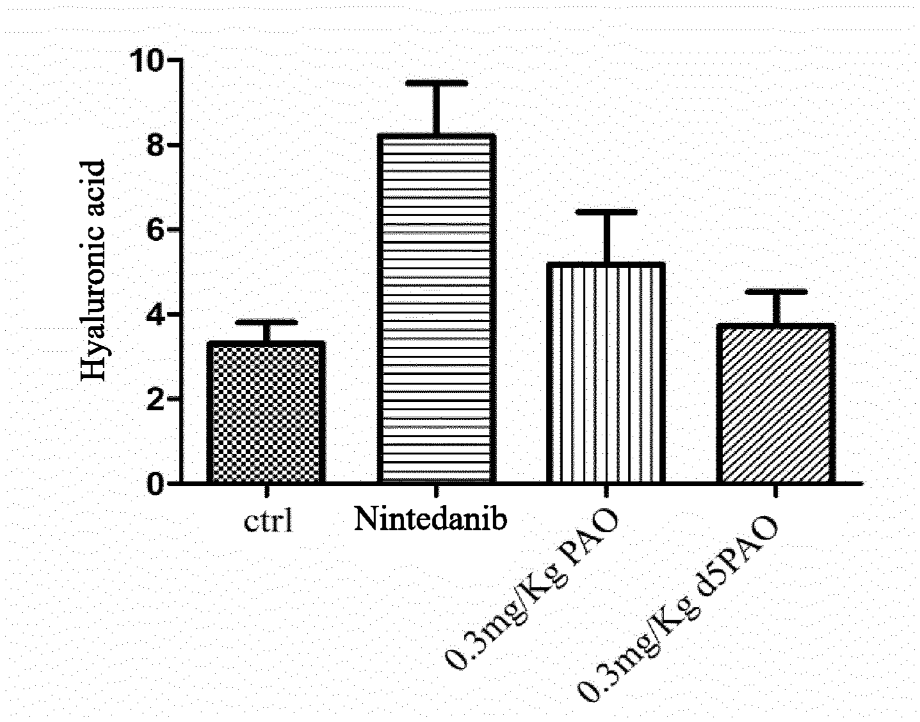
Hyaluronic acid
10
8
6
4
2
0
ctrl
Nintedanib
0.3mg/Kg PAO
0.3mg/Kg d5PAO

*FIG. 40*

**INTERNATIONAL SEARCH REPORT**

International application No.
**PCT/CN2021/084773**

**A. CLASSIFICATION OF SUBJECT MATTER**

C07F 9/74(2006.01)i; A61K 31/00(2006.01)i; A61P 1/16(2006.01)i; A61P 25/00(2006.01)i; A61P 29/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07F; A61K; A61P

Documentation searched other than minimum documentation to the extent that such documents are included in the fields searched

Electronic data base consulted during the international search (name of data base and, where practicable, search terms used)

CNPAT, CNTXT, CNKI, WPI, EPODOC, WOTXT, EPTXT, USTXT, ISI, STN: 挪贝肽, 黄福德, 王文安, 洪峰, 魏万国, 张建刚, 焦常平, 曹鲁乡, 氧化苯砷, 氧化苯胂, 氧苯砷, 氧苯胂, 苯砷氧, 苯胂氧, 苯砷, 苯胂, 胂, 砷, 氘, 重氢, 肿瘤, 癌, 恶病质, 阿尔茨海默, 阿尔兹海默, 老年痴呆, 炎, 肝, 肺, 纤维化, phenylarsine oxide, PAO, deuterium, D, tumor, cancer, cachexia, alzheimer+, AD, HSC, hepatic, liver, lung, fibrosis, PI4KIII

**C. DOCUMENTS CONSIDERED TO BE RELEVANT**

| Category* | Citation of document, with indication, where appropriate, of the relevant passages | Relevant to claim No. |
|---|---|---|
| X | CN 108721622 A (SHANGHAI ADVANCED RESEARCH INSTITUTE, CHINESE ACADEMY OF SCIENCES) 02 November 2018 (2018-11-02)<br>description, paragraphs [0004]-[0009] and [0103]-[0111] | 1-20, 22-38 |
| Y | CN 108721622 A (SHANGHAI ADVANCED RESEARCH INSTITUTE, CHINESE ACADEMY OF SCIENCES) 02 November 2018 (2018-11-02)<br>description, paragraphs [0004]-[0009] and [0103]-[0111] | 21 |
| X | WO 2016172955 A1 (JIANGSU NUO-BETA PHARMACEUTICAL TECHNOLOGY CO., LTD.) 03 November 2016 (2016-11-03)<br>description, page 3, paragraph 3 to page 4, paragraph 2, and page 33, paragraph 2 to page 42, paragraph 2 | 1-20, 22-38 |
| Y | WO 2016172955 A1 (JIANGSU NUO-BETA PHARMACEUTICAL TECHNOLOGY CO., LTD.) 03 November 2016 (2016-11-03)<br>description, page 3, paragraph 3 to page 4, paragraph 2, and page 33, paragraph 2 to page 42, paragraph 2 | 21 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 June 2021** | **24 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2021/084773

C. DOCUMENTS CONSIDERED TO BE RELEVANT

| Category* | Citation of document, with indication, where appropriate, of the relevant passages | Relevant to claim No. |
|---|---|---|
| X | WO 2016172952 A1 (JIANGSU NUO-BETA PHARMACEUTICAL TECHNOLOGY CO., LTD.) 03 November 2016 (2016-11-03) description, page 3, paragraph 3 to page 4, paragraph 2, and page 34, paragraph 2 to page 43, paragraph 2 | 1-20, 22-38 |
| Y | WO 2016172952 A1 (JIANGSU NUO-BETA PHARMACEUTICAL TECHNOLOGY CO., LTD.) 03 November 2016 (2016-11-03) description, page 3, paragraph 3 to page 4, paragraph 2, and page 34, paragraph 2 to page 43, paragraph 2 | 21 |
| X | WO 2016173562 A1 (JIANGSU NUO-BETA PHARMACEUTICAL TECHNOLOGY CO., LTD.) 03 November 2016 (2016-11-03) description, page 3, paragraph 3 to page 4, paragraph 2, and page 34, paragraph 2 to page 43, paragraph 2 | 1-20, 22-38 |
| Y | WO 2016173562 A1 (JIANGSU NUO-BETA PHARMACEUTICAL TECHNOLOGY CO., LTD.) 03 November 2016 (2016-11-03) description, page 3, paragraph 3 to page 4, paragraph 2, and page 34, paragraph 2 to page 43, paragraph 2 | 21 |
| X | 仝艳艳 等 (TONG, Yanyan et al.),. "氧化苯砷体外对大鼠原代肝星状细胞活化的阻抑作用研究 (Inhibition of Primary Cultured Hepatic Stellate Cell Activation by Phenylarsine Oxide in Vitro)" *实用肝脏病杂志 (Journal of Practical Hepatology),* Vol. 19, No. 4, 31 July 2016 (2016-07-31), pp. 413-417 | 1-20, 22-38 |
| Y | 仝艳艳 等 (TONG, Yanyan et al.). "氧化苯砷体外对大鼠原代肝星状细胞活化的阻抑作用研究 (Inhibition of Primary Cultured Hepatic Stellate Cell Activation by Phenylarsine Oxide in Vitro)" *实用肝脏病杂志 (Journal of Practical Hepatology),* Vol. 19, No. 4, 31 July 2016 (2016-07-31), pp. 413-417 | 21 |
| X | 萧水银 等 (XIAO, Shuiyin et al.). "氧化苯砷抑制内皮细胞移行及血管新生之研究 (Non-official translation: Inhibition of Endothelial Cell Migration and Angiogenesis by Phenylarsine Oxide)" *第六届海峡两岸心血管科学研讨会论文摘要集 (Abstracts of the 6th Scientific Conference on Cardiovascular Sciences Across the Strait),* 31 July 2007 (2007-07-31), pp. 64 and 65 | 1-20, 22-38 |
| Y | 萧水银 等 (XIAO, Shuiyin et al.). "氧化苯砷抑制内皮细胞移行及血管新生之研究 (Non-official translation: Inhibition of Endothelial Cell Migration and Angiogenesis by Phenylarsine Oxide)" *第六届海峡两岸心血管科学研讨会论文摘要集 (Abstracts of the 6th Scientific Conference on Cardiovascular Sciences Across the Strait),* 31 July 2007 (2007-07-31), pp. 64 and 65 | 21 |
| Y | LLOYD, Nicholas C. et al. "Substituted Phenylarsonic Acids; Structures and Spectroscopy" *Journal of Organometallic Chemistry,* Vol. 693, No. 14, 30 April 2008 (2008-04-30), pp. 2443-2450 | 21 |
| Y | CN 109369724 A (LANZHOU UNIVERSITY) 22 February 2019 (2019-02-22) description, embodiment 4 | 21 |

Form PCT/ISA/210 (second sheet) (January 2015)

**INTERNATIONAL SEARCH REPORT**
**Information on patent family members**

International application No.
**PCT/CN2021/084773**

| Patent document cited in search report | | | Publication date (day/month/year) | Patent family member(s) | | | Publication date (day/month/year) |
|---|---|---|---|---|---|---|---|
| CN | 108721622 | A | 02 November 2018 | None | | | |
| WO | 2016172955 | A1 | 03 November 2016 | KR | 20180012274 | A | 05 February 2018 |
| | | | | EP | 3290525 | A4 | 19 December 2018 |
| | | | | US | 2019211371 | A1 | 11 July 2019 |
| | | | | AU | 2015392754 | B2 | 14 March 2019 |
| | | | | CN | 107849608 | A | 27 March 2018 |
| | | | | JP | 2018520367 | A | 26 July 2018 |
| | | | | AU | 2015392754 | A1 | 21 December 2017 |
| | | | | JP | 6636614 | B2 | 29 January 2020 |
| | | | | KR | 20200011567 | A | 03 February 2020 |
| | | | | EP | 3290525 | A1 | 07 March 2018 |
| WO | 2016172952 | A1 | 03 November 2016 | JP | 2020203907 | A | 24 December 2020 |
| | | | | JP | 2018521112 | A | 02 August 2018 |
| | | | | KR | 102048137 | B1 | 25 November 2019 |
| | | | | KR | 20190131142 | A | 25 November 2019 |
| | | | | RU | 2017141805 | A | 31 May 2019 |
| | | | | JP | 6755938 | B2 | 16 September 2020 |
| | | | | EP | 3290514 | A1 | 07 March 2018 |
| | | | | RU | 2017141805 | A3 | 31 May 2019 |
| | | | | RU | 2724493 | C2 | 23 June 2020 |
| | | | | KR | 20180006396 | A | 17 January 2018 |
| | | | | EP | 3290514 | A4 | 22 May 2019 |
| | | | | AU | 2016255474 | A1 | 21 December 2017 |
| | | | | CN | 107849545 | A | 27 March 2018 |
| | | | | WO | 2016173562 | A1 | 03 November 2016 |
| | | | | US | 2019314321 | A1 | 17 October 2019 |
| | | | | AU | 2016255474 | B2 | 30 January 2020 |
| | | | | AU | 2020202847 | A1 | 21 May 2020 |
| | | | | AU | 2016255474 | A8 | 01 March 2018 |
| WO | 2016173562 | A1 | 03 November 2016 | JP | 2020203907 | A | 24 December 2020 |
| | | | | JP | 2018521112 | A | 02 August 2018 |
| | | | | KR | 102048137 | B1 | 25 November 2019 |
| | | | | KR | 20190131142 | A | 25 November 2019 |
| | | | | RU | 2017141805 | A | 31 May 2019 |
| | | | | JP | 6755938 | B2 | 16 September 2020 |
| | | | | EP | 3290514 | A1 | 07 March 2018 |
| | | | | RU | 2017141805 | A3 | 31 May 2019 |
| | | | | RU | 2724493 | C2 | 23 June 2020 |
| | | | | KR | 20180006396 | A | 17 January 2018 |
| | | | | EP | 3290514 | A4 | 22 May 2019 |
| | | | | AU | 2016255474 | A1 | 21 December 2017 |
| | | | | CN | 107849545 | A | 27 March 2018 |
| | | | | US | 2019314321 | A1 | 17 October 2019 |
| | | | | WO | 2016172952 | A1 | 03 November 2016 |
| | | | | AU | 2016255474 | B2 | 30 January 2020 |
| | | | | AU | 2020202847 | A1 | 21 May 2020 |
| | | | | AU | 2016255474 | A8 | 01 March 2018 |
| CN | 109369724 | A | 22 February 2019 | CN | 109369724 | B | 09 March 2021 |

Form PCT/ISA/210 (patent family annex) (January 2015)